(19) Europäisches Patentamt
European Patent Office
Office européen des brevets

(11) Publication number: 0 259 014 A2

(12) **EUROPEAN PATENT APPLICATION**

(21) Application number: 87306904.1

(51) Int. Cl.⁴: C07H 15/236 , A61K 31/70

(22) Date of filing: 04.08.87

(30) Priority: 04.08.86 JP 181850/86

(43) Date of publication of application: 09.03.88 Bulletin 88/10

(84) Designated Contracting States: AT BE CH DE ES FR GB IT LI LU NL SE

(71) Applicant: ZAIDAN HOJIN BISEIBUTSU KAGAKU KENKYU KAI
14-23, Kami Osaki 3-chome
Shinagawa-ku Tokyo(JP)

(72) Inventor: Umezawa, Hamao
23, Toyotama Kita 4-chome Nerima-ku
Tokyo(JP)
Inventor: Umezawa, Sumio
1, Banshu-cho Shinjuku-ku
Tokyo(JP)
Inventor: Tsuchiya, Tsutomu
1-17-201, Eda Higashi 3-chome Midori-ku
Yokohama-shi Kangawa-ken(JP)
Inventor: Shitara, Tetsuo
16-18 Nakahara 2-chome Jujoh
Kita-ku Tokyo(JP)
Inventor: Kobayashi, Yoshihiko
11-3, Shimodacho 1-chome Kohhoku-ku
Yokohama-shi Kangawa-ken(JP)

(74) Representative: Baverstock, Michael George Douglas et al
BOULT, WADE & TENNANT 27 Furnival Street
London, EC4A 1PQ(GB)

# (54) 5-Deoxy-5-fluorokanamycin B derivatives and processes for the production thereof.

(57) As new compounds are now provided 5-deoxy-5-fluorokanamycin B, 5,3'-dideoxy-5-fluorokanamycin B, 5,4'-dideoxy-5-fluorokanamycin B and 5,3',4'-trideoxy-5-fluorokanamycin B as well as 1-N-(3-amino-2-hydroxypropionyl)-or 1-N-(4-amino-2-hydroxybutyryl)-derivatives thereof which are all useful as antibacterial agents and exhibit improved antibacterial activities. These new compounds may be produced by synthetic processes starting from an appropriately N,O-protected 5-epi-kanamycin B, 3'-deoxy-5-epi-kanamycin B, 4'-deoxy-5-epi-kanamycin B or 3',4'-dideoxy-5-epi-kanamycin B derivative which is now prepared by a combination of reaction steps.

EP 0 259 014 A2

# 5-DEOXY-5-FLUOROKANAMYCIN B DERIVATIVES AND PROCESSES FOR THE PRODUCTION THEREOF

This invention relates to 5-deoxy-5-fluorokanamycin B and its derivates which are all new semi-synthetic aminoglycosidic antibiotics and which are new compounds exhibiting high antibacterial activity against a variety of kanamycin-sensitive bacteria and kanamycin-resistant bacteria and are useful as antibacterial agents. This invention also relates to a pharmaceutical composition containing 5-deoxy-5-fluorokanamycin B or a derivative thereof as the active ingredient. This invention further relates to processes for the production of the new compounds of this invention.

Various deoxy derivatives of kanamycin A, B and C are already known. These known deoxy-derivatives of the kanamycins have useful antibacterial activities, but the antibacterial spectra of these known deoxy-derivatives of the kanamycins are of different ranges. These known deoxykanamycin derivatives may become inactive against such new resistant strains of bacteria which will occasionally occur in future. Accordingly, it is always desirable that new, antibacterial compounds having better properties than the known antibacterial kanamycin derivatives should be produced and provided for use in therapeutic treatment of bacterial infections.

The Applicants suspected that if they could succeed in synthetizing such a new kanamycin A derivative having the 3'-hydroxyl group replaced by a fluoro group, namely 3'-fluro-3'-deoxykanamycin A, this compound might be active against some kanamycin-resistant strains of bacteria which are already known and also against some other resistant strains which will possibly occur in future. Already, the Applicants have succeeded in synthetizing this 3'-fluoro-3'-deoxykanamycin A as a semi-synthetic aminoglycosidic antibiotic (see Japanese patent application No. 161615/84; Japanese patent application first publication "Kokai" No. 40297/86; EP-A-0173614; and US-A-4,634,688).

Further, the Applicants succeeded in synthetizing 3'-fluoro-3'-deoxykanamycin B, and they have found that this new compound, 3'-fluoro-3'-deoxykanamycin B has remarkable antibacterial activities against various gram-positive and gram-negative bacteria, including resistant bacteria (Japanese patent application No. 262700/84; Japanese patent application first publication "Kokai" No. 140597/86).

However, it has been found that the new compound, 3'-fluoro-3'-deoxykanamycin B having a hydroxyl group at the 4'-position exhibits a low antibacterial activity against some resistant strains of bacteria capable of producing a phosphorylating enzyme and/or an adenylating enzyme which can react with the 4'-hydroxyl group, for example, Staphylococcus aureus Ap 01 and Staphylococcus epidermidis 109. As a result of further researches, the Applicants succeeded in synthetizing 3', 4'-dideoxy-3'-fluoro-kanamycin B (see Japanese patnet application No. 188525/85; Japanese patent application first publication "Kokai" No. 51694/87; U.S. patent application SN. 899,100; and European patent application No. 86 401892.4).

Furthermore, the Applicants succeeded in synthetizing new compounds, 1-N-[RS)-or (S)-3-amino-2-hydroxy-propionyl]-3'-fluoro-3'-deoxykanamycins A and B, as well as 1-N-[(S)-4-amino-2-hydroxybutyryl]-3'-fluoro-3'-deoxykanamycins A and B by acylating the 1-amino group of the 3'-fluoro-3'-deoxykanamycin A or 3'-fluoro-3'-deoxykanamycin B with (RS)-or (S)-3-amino-2-hydroxypropionic acid or (S)-4-amino-2-hydroxybutyric acid. It was found that these new derivatives of 3'-fluoro-3'-deoxykanamycins A and B exhibit remarkable antibacterial activities against various gram-positive and gram-negative bacterial, including the resistant bacteria (Japanese patent application No. 76706/85; Japanese patent application first publication "Kokai' No. 236791/86).

Moreover, the Applicants succeeded in synthetizing a new compound, 2'3'-dideoxy-2'-fluoro-kanamycin A firstly, and it was found that this new compound also exhibits an antibacterial activity against various gram-positive and gram-negative bacteria, including various resistant bacteria (see the specification of Japanese patent application No. 263759/84; Japanese patent application first publication "Kokai" No. 143393/86; U.S. patent application SN. 807,485; and European patent application No. 85 115901.2). The Applicants also succeeded in synthetizing new compounds, 1-N-[(RS)-or (S)-3-amino-2-hydroxypropionyl]-2',3'-dideoxy-2'-fluorokanamycin A and 1-N-[(S)-4-amino-2-hydroxybutyryl]-2',3'-dideoxy-2'-fluorokanamycin A by acylating the 1-amino group of the 2',3'-dideoxy-2'-fluorokanamycin A with (RS)-or (S)-3-amino-2-hydroxypropionic acid or (S)-4-amino-2-hydroxybutyric acid. It was also found that these new compounds have remarkable antibacterial activities against a variety of gram-positive bacteria and gram-negative bacteria, including various resistant bacteria (see. Japanese patent application No. 231027/85; Japanese patent application first publication "Kokai" No. 93296/87; U.S. patent application No. 807,485; and European patent application No. 85 115901.2).

On the other hand, the publication "Aminocyclitol Antibiotics" pages 371-392, edited by K.L. Rinehart and T.Suami (published by the American Chemical Society in 1980) discloses the production of 5-deoxy-5-fluoro-5-epi-sisomicin according to a method comprising reacting diethylaminosulfur trifluoride (hereinafter abbreviated as DAST) with a protected derivative of sisomicin in which all the amino groups and all the hydroxyl groups except the 5-hydroxyl group have been protected, to effect the replacement of the 5-hydroxy group by a fluoro substituent with concomitant steric inversion of the substitutent at the 5-position and thereby produce a corresponding protected derivative of 5-deoxy-5-fluoro-5-epi-sisomicin, and then removing the protective groups from the latter protected derivative. This Publication also refers to 5-deoxy-5-fluorosisomicin, though a method of producing 5-deoxy-5-fluorosisomicin is not disclosed specifically. This Publication further describes a comparison between the minimum growth inhibitory concentrations of 5-epi-sisomicin, 5-deoxy-5-fluoro-5-epi-sisomicin and 5-deoxy-5-fluorosisomicin against various kinds of sisomicin-sensitive bacteria and sisomicin-resistant bacteria, revealing that 5-deoxy-5-fluoro-5-epi-sisomicin and 5-deoxy-5-fluorosisomicin exhibit lower antibacterial activities than that of 5-epi-sisomicin or sisomicin itself against resistant strains of <u>Escherichia</u> <u>coli</u>, resistant strains of <u>Klebsiella</u> <u>pneumoniae</u> and <u>Pseudomonas</u> <u>aeruginosa</u>. This reference also contains some descriptions indicating that 5-deoxy-5-fluoro-5-epi-sisomicin exhibits antibacterial activity as high as or lower than that of sisomicin. Accordingly, the disclosures of this Publication do not teach that the antibacterial activity of sisomicin could be enhanced by replacement of the 5-hydroxyl group of sisomicin by the fluoro group.

Further, the "Journal of Carbohydrate Chemistry" Vol. 1, page 289 (1982) discloses the production of 4″-deoxy-4″-fluoro-4″-epi-kanamycin A starting from kanamycin A, although this literature shows thât the antibacterial activity of 4″-deoxy-4″-fluoro-4″-epi-kanamycin A is lower than that of the parent kanamycin A. Furthermore, "Tetrahedron Letters" Vol. 24, No. 17, pages 1763-1766(1983) discloses that 6″-deoxy-6″-fluorokanamycin A is produced by reacting a 2′,3′,4′,2″,4″-penta-O-acetyl-tetra-N-tert.-butoxycarbonyl-kanamycin A derivative with tetrabutylammonium fluoride to yield 2′,3′,4′,2″,4″-penta-O-acetyl-tetra-N-tert.-butoxycarbonyl-6″-deoxy-6″-fluorokanamycin A, followed by removing from this product the O-acetyl groups and the N-tert.-butoxycarbonyl groups according to the conventional deprotection techniques; also that 5,6″-dideoxy-5,6″-difluoro-5-epi-kanamycin A is produced by reacting the above-mentioned 2′,3′,4′,2″,4″-penta-O-acetyl-tetra-N-tert.-butoxycarbonylkanamycin A with DAST to yield 2′,3′,4′,2″,4″-penta-O-acetyl-tetra-N-tert.-butoxycarbonyl-5,6″-dideoxy-5,6″-difluoro-5-epi-kanamycin A, followed by removing from this product the residual protective groups in a conventional manner; and further that 5-deoxy-5-fluoro-5,4″-di-epi-kanamycin A is produced by reacting the 2′,3′,4′,2″,6″-penta-O-acetyl-tetra-N-tert.-butoxycarbonylkanamycin A with DAST to give 2′,3′,4′,2″,6″-penta-O-acetyl-1,3,6′-tri-N-tert.-butoxycarbonyl-3″-N,4″-O-carbonyl-5-deoxy-5-fluoro-5,4″-di-epi-kanamycin A along with 2′,3′,4′,2″,6″-penta-O-acetyl-tetra-N-tert.-butoxycarbonyl-5-deoxy-5-fluoro-5,4″-di-epi-kanamycin A, followed by removing from the latter product the residual protective groups in a conventional manner.

In the reference "Tetrahedron Letters" Vol. 24, No. 17, pages 1763-1766 (1983), there is disclosed that the 6″-deoxy-6″-fluorokanamycin A, 5,6″-dideoxy-5,6″-difluoro-5-epi-kanamycin A and 5-deoxy-5-fluoro-5,4″-di-epi-kanamycin A show antibacterial activities substantially as high as that of the parent kanamycin A. Accordingly, this reference does not have any disclosure teaching that the antibacterial activity of kanamycin A can be enhanced by replacement of the 6″-hydroxyl group and/or the 5-hydroxyl group of kanamycin A by a fluorine atom.

In another publication, "Chemical Abstracts" <u>90</u>, 104, 301 (1979), there is disclosed a method of producing 5-deoxy-5-fluoro-5-epi-kanamycin A, which comprises reacting DAST with a protected derivative of kanamycin A whose the amino groups and the hydroxyl groups other than the 5-hydroxyl group have been protected with conventional protective groups, to yield a corresponding protected derivative of 5-deoxy-5-fluoro-5-epi-kanamycin A, and then removing the protective groups from the latter protected reaction product. But this literature does not disclose that the antibacterial activity of 5-deoxy-5-fluoro-5-epi-kanamycin A so obtained is improved over that of the parent kanamycin A.

As will be clear from the above, it has not been found that the antibacterial activity of sisomicin can advantageously be improved with the cases of 5-deoxy-5-fluoro-5-epi-sisomicin and 5-deoxy-5-fluorosisomicin which are obtained by reacting the 5-hydroxyl group with DAST and thereby replacing the 5-hydroxyl group by a fluorine atom, with or without possible concomitant inversion of the steric arrangement of the 5-substituent. Besides, it has not yet been recognized that the antibacterial activity of kanamycin A can advantageously be enhanced with the case of the 5-deoxy-5-fluoro-5-epi-kanamycin A which is obtained by reacting DAST with the 5-hydroxyl group of kanamycin A and thereby substituting a fluoro group for the 5-hydroxyl group, accompanied by the inversion of the steric disposition of the 5-substituent.

Meanwhile, the Applicants have already found that 3'-deoxy-3-fluorokanamycins A and B, 3',4'-dideoxy-3'-fluorokanamycin B and 2',3'-dideoxy-2'-fluorokanamycin A can each exhibit their improved antibacterial activities, as compared to those of their corresponding parent compounds not having the fluoro substituent. On the other hand, the Applicants have recognized that the replacement of the 5-hydroxyl group of sisomicin or kanamycin A by the fluoro substituent cannot bring about any advantageous improvement or enhancement in the antibacterial activity of the parent sisomicin or kanamycin A. Despite this discovery, the Applicants suspected that if 5-deoxy-5-fluorokanamycin B, as a new compound, can be synthetized firstly from kanamycin B, this 5-deoxy-5-fluorokanamycin B might have antibacterial activity which is advantageously enhanced or improved as compared to the antibacterial activity of kanamycin B itself. Therefore, the Applicants made researches in an attempt to synthetize 5-deoxy-5-fluorokanamycin B.

The above-mentioned suspicion of the present Applicants that said 5-deoxy-5-fluorokanamycin B might exhibit improved antibacterial activity over that of kanamycin B arose as described below. Thus, the Applicants considered that the molecule of Kanamycin B normally takes a steric structure represented by the following formula (A)

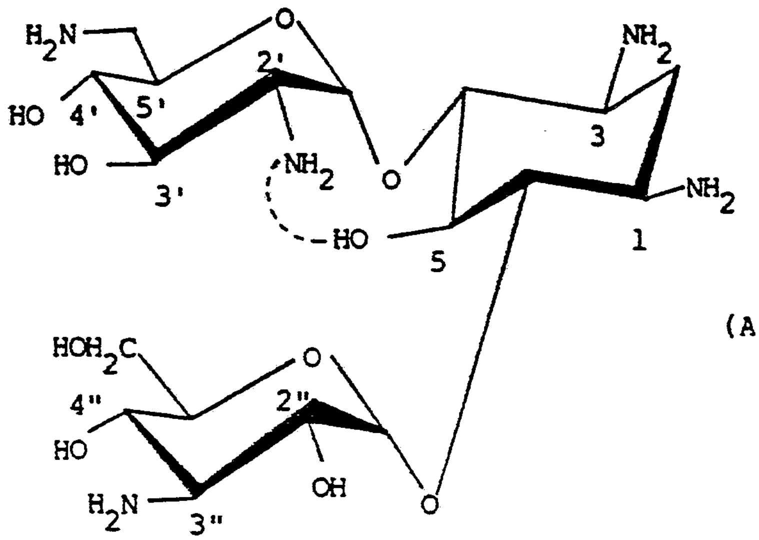

(A)

and that any interaction (which probably occurs due to the sucking action exhibited by the hydrogen bound or due to the repulsion between the nitrogen atom and oxygen atom) is exerted between the equatorial (non-axial) 5-hydroxy group of the deoxystreptamine moiety and the equatorial 2'-amino group of the 2,6-diamino-sugar moiety in the kanamycin B molecule. The Applicants further considered that the above-mentioned interaction might put restrictions not only on the angle of the chemical linkage between the deoxystreptamine moiety and the 2,6-diamino-sugar moiety as bonded to the 4-hydroxyl group of said deoxystreptamine moiety, but also on the degree of freedom of the rotations of the deoxystreptamine moiety and the 2,6-diamino-sugar moiety, and so on, whereby the relative and steric dispositions of the three cyclic moieties of the kanamycin B molecule can be decided to a great extent. Then, the Applicants considered that if they could obtain the new compound, 5-deoxy-5-fluorokanamycin B, in the molecule of which the 5-hydroxyl group has been replaced by the fluorine atom having a higher electro-negativity, but with the equatorial disposition of the original 5-hydroxyl group being retained for the 5-fluoro group in the molecule of the new kanamycin B compound, the interaction as exerted between the equatorial 5-fluoro group and the equatorial 2'-amino group of the 5-deoxy-5-fluorokanamycin B would be different from the interaction which is originally exerted between the 5-hydroxyl group and the 2'-amino group of the molecule of kanamycin B, with the consequence that the relative and steric dispositions of the three cyclic moieties of the kanamycin B molecule would be changed, affecting the antibacterial activity of the kanamycin B compound so that the new compound 5-deoxy-5-fluorokanamycin B would be able to exhibit antibacterial activities which would be considerably different from the antibacterial activity of kanamycin B itself. As a result of their researches, the Applicants have now succeeded in synthetizing 5-deoxy-5-fluorokanamycin B, and we have determined the antibacterial activity of this new compound, discovering that the antibacterial activity of 5-deoxy-5-fluorokanamycin B is improved over that of the parent kanamycin B. That is to say, it has been discovered that 5-deoxy-5-fluorokanamycin B is significantly active against Escherichia coli K-12 LA290 R55 (a resistant strain having the enzyme for adenylating the 2"-hydroxyl group of kanamycin B) and

against Escherichia coli JR 225 (a resistant strain having the enzyme for acetylating the 3-hydroxyl group of kanamycin B) and also against Providensia Pv 16 (a resistant strain having the enzyme for acetylating the 2′-amino group of kanamycin B).

In the course of studying the process for the synthetic production of 5-deoxy-5-fluorokanamycin B, and as an outcome of the Applicants' study, it has been found that, firstly, such a special type of an N,O-protected derivative of kanamycin B all of whose amino groups and hydroxyl groups except the 5-hydroxyl group have been blocked with suitable protective groups is needed as an intermediate raw material to be substituted by the fluoro group at the 5-position thereof. Accordingly, the Applicants continued their study to provide a method for preparing such special type of the N,O-protected kanamycin B derivative, and as a result, succeeded in synthetizing a 3′,4′,2″,4″,6″-penta-O-protected-1,3,2′,6′,3″-penta-N-protected-(sulfonylated)-kanamycin B having the free 5-hydroxyl group by a method which comprises selecting an amino-protecting group of the sulfonyl type from amongst many and various kinds of known amino-protecting groups, introducing the amino-protecting group of the sulfonyl type into all the amino groups of kanamycin B for the protection of the amino groups and then reacting the resulting N-protected (sulfonylated)-kanamycin B derivative with an acyl chloride, preferably acetyl chloride in anhydrous pyridine at a low temperature of 0°C or less to acylate selectively all the hydroxyl groups other than the 5-hydroxyl group of the kanamycin B molecule; or alternatively by a method which comprises blocking both the 4″-and 6″-hydroxyl groups of kanamycin B simultaneously with a di-valent hydroxyl-protecting group such as an alkylidene group and then reacting the resulting 4″,6″-di-O-protected kanamycin B derivative with an acyl halogenide, acyl anhydride or N-acyl-imidazole under selected reaction conditions for the protection of all the hydroxyl groups other than the 5-hydroxyl group, so that the 5-hydroxyl group is not acylated while the 3′-, 4′-and 2″-hydroxyl groups are selectively acylated.

Furthermore, the Applicants discovered that the replacement by the fluoro group of the 5-hydroxyl group of the above-mentioned special type of the N,O-protected kanamycin B derivative may successfully be achieved by reacting with DAST or the other dialkylaminosulfur trifluoride or a bis-(dialkylamino)-sulfur difluoride which can give a good efficiency of fluorination under mild reaction conditions (see "J. Org. Chem." 40 , No. 5, pages 574-578 (1975)). While the Applicants have noticed also that when the dialkylaminosulfur trifluoride or bis-(dialkylamino)-sulfur difluoride is reacted with the above-mentioned 3′,4′,2″, 4″,6″-penta-O-protected-1,3,2′,6′3″-penta-N-protected (sulfonylated)-kanamycin B, disadvantageously, the steric arrangement of the 5-substituent can inevitably be inverted from the original disposition of the 5-hydroxyl group, simultaneously to the replacement thereof by the fluoro substitutent, thereby giving a 5-deoxy-5-fluoro-5-epi-kanamycin B derivative. As a result of the Applicants' continued study, it was suspected that if they could prepare at first a suitable N,O-protected 5-epi-kanamycin B derivative by effecting exclusively the steric inversion of the 5-hydroxyl group of a suitable N,O-protected kanamycin B before this N,O-protected kanamycin B was reacted with a fluorination agent such as DAST to replace its 5-hydroxyl group by the fluoro group; and if the Applicants could react the above suitable N,O-protected 5-epi-kanamycin B derivative with a fluorination agent such as DAST to effect the second inversion of the 5-epi-substituent and the simultaneous substitution by the fluoro group at the 5-position, whereby the intended suitable N,O-protected 5-deoxy-5-fluorokanamycin B derivative which was not in the 5-epimer form would successfully be produced in an ingenious way. The Applicants thus continued their research to discover that the steric inversion of the 5-hydroxyl group of the above-mentioned 3′,4′,2″,4″,6″-penta-O-protected-1,3,2′,6′,3″-penta-N-protected(sulfonylated)-kanamycin B could be achieved at good efficiency by reacting the latter N,O-protected kanamycin B derivative with triphenylphosphine [$P(C_6H_5)_3$], diethyl azodicarboxylate ($C_2H_5$-$O_2C$-N = N-$CO_2$-$C_2H_5$) and an organic acid such as benzoic acid together according to Mitsunobu's reaction (see O. Mitsunobu & Y. Yamada "Bull. Chem. Soc. Jpn." 40, 2380 (1967)). Thus, was prepared from the above-mentioned N,O-protected kanamycin B derivative a 5-O-benzoyl-5-epi-3′,4′,2″,4″,6″-penta-O-protected-1,3,2′,6′3″-penta-N-protected(sulfonylated)-kanamycin B according to the Mitsunobu's reaction, and the latter compound being then treated with a methanolic solution of sodium methoxide to remove the benzoyl group from the 5-epi-hydroxyl group and remove the acyl groups from the 3′-, 4′-, 2″-, 4″-and 6″-hydroxyl groups thereof or alternatively removing the acyl groups from the 3′-, 4′-and 2″-hydroxyl group thereof (in cases where the 4″-and 6″-hydroxyl groups have been protected by a di-valent hydroxyl-protecting group), and thereby to afford a corresponding 1,3,2′,6′,3″-penta-N-protected-5-epi-kanamycin B or alternatively a corresponding 4,6″-di-O-protected-1,3,2′,6′,3″-penta-N-protected-5-epi-kanamycin B. The Applicants subsequently treated the latter protected 5-epi-kanamycin B derivative so obtained by reacting with an N-acyl-imidazole or other acylating agent in the same manner as described above, to acylate selectively all the hydroxyl groups other than the 5-hydroxyl group of the 5-epi-kanamycin B compound, without involving the acylation of the 5-hydroxyl group. In this way, the Applicants succeeded in preparing a 3′,4′,2″,4″,6″-penta-O-protected-1,3,2′,6′,3″-penta-N-protected(sulfonylated)-5-epi-kanamycin B. When the latter N,O-protected 5-epi-kanamycin B derivative was reacted with DAST or another dialkylamino-sulfur trifluoride or a bis-(dialkylamino)-sulfur difluoride or an equivalent fluorination agent thereof to produce a 3',4',2",4",6"-penta-O-protected-1,3,2',6'-3"-penta-N-protected(sulfonylated)-5-deoxy-5-fluorokanamycin B, from which were then removed all the amino-protecting groups and the hydroxyl-protecting groups according to the conventional deprotection techniques, there was successfully produced the desired 5-deoxy-5-fluorokanamycin B starting from Kanamycin B. It has also been found that 5-deoxy-5-fluorokanamycin B so synthetized exhibits remarkably improved antibacterial activity as compared with the parent kanamycin B.

Moreover, the Applicants have found that the amino-protecting groups which are present in the above-mentioned 3',4',2", 4",6"-penta-O-protected-1,3,2',6',3"-penta-N-protected (sulfonylated)-kanamycin B, whose 5-hydroxyl group is to be sterically inverted according to Mitsunobu's reaction as made in the above process of synthetizing 5-deoxy-5-fluorokanamycin B from kanamycin B, should be selected from an alkylsulfonyl group such as mesyl; an aralkylsulfonyl group such as benzylsulfonyl; and an arylsulfonyl group such as tosyl, as otherwise Mitsunobu's reaction could not proceed smoothly, and also that the tosyl group is then a most preferred amino-protecting group. On the other hand, it has been found that the reaction for the steric inversion of the 5-epi-hydroxyl group and the replacement of the hydroxyl group by the fluoro group through the treatment with the fluorination agent such as DAST can proceed to a sufficient extent, even when the amino-protecting groups present in the N,O-protected 5-epi-kanamycin B derivative to be treated with said fluorination agent do not belong to the aforesaid amino-protecting group of the sulfonyl type. Therefore, the Applicants have now found that 5-deoxy-5-fluorokanamycin B can be synthesized also according to such a process which comprises treating the above 3',4',2",4",6"-penta-O-protected-1,3,2',6',3"-penta-N-protected(sulfonylated)-5-epi-kanamycin B with an alkali metal in liquefied ammonia, thus removing all the amino-protecting groups of the sulfonyl type therefrom to produce a 3',4',2",4",6"-penta-O-protected and N-unprotected-5-epi-kanamycin B, then protecting all the amino groups of the latter O-protected 5-epi-kanamycin B derivative by introducing thereinto optional, known amino-protecting groups, inclusive of an acyl group, for example, an alkanoyl group such as acetyl; an aroyl group such as benzoyl; an alkoxycarbonyl group such as ethoxycarbonyl and tert.-butoxycarbonyl; an aralkyloxycarbonyl group such as benzyloxycarbonyl; an aryloxycarbonyl group such as phenoxycarbonyl; and an amino-protecting group of the Schiff's base type such as a benzylidene group or a p-nitrobenzylidene group, to yield a 3',4',2",4",6"-penta-O-protected-1,3,2',6',3"-penta-N-protected (i.e., as protected by the N-protecting method other than sulfonylation)-5-epi-kanamycin B, and then reacting the 5-hydroxyl group of the latter compound with a fluorination agent such as DAST to produce a corresponding N,O-protected-5-deoxy-5-fluorokanamycin B, followed by removing all the protective groups therefrom. Accordingly, for instance, 3',4',2",4",6"-penta-O-benzoyl-1,3,2',6',3"-pentakis(N-ethoxycarbonyl)-5-epi-kanamycin B which was employed as an intermediate product in the method of Suami et al for the synthesis of 5-epi-kanamycin B (see "Bulletin of the Chemical Society of Japan" Vol. 52. No. 3, 955-956 (1979)) may also be converted into a corresponding N,O-protected 5-deoxy-5-fluorokanamycin B of formula (IV) by reacting with the fluorination agent such as DAST in accordance with the process of the third aspect of this invention.

Moreover, the Applicants have found that the above-described processes for the protection of the amino groups and all the hydroxyl groups except the 5-hydroxyl group of kanamycin B; and the Mitsunobu's reaction for effecting the inversion of the 5-hydroxyl group which is applied to kanamycin B; as well as the process for the fluorination and inversion of the epi-disposed 5-hydroxyl group which is effected by reacting the epi-5-hydroxyl group with a dialkylaminosulfur trifluoride such as DAST or a bis-(dialkylamino)-sulfur difluoride or an equivalent fluorination agent thereof are effectively applicable to 3'-deoxykanamycin B, 4'-deoxykanamycin B and 3',4'-dideoxykanamycin B which are all the deoxy-derivatives of kanamycin B, and thus the Applicants have succeeded in synthesizing 5,3'-dideoxy-5-fluorokanamycin B from 3'-deoxykanamycin B (namely, tobramycin); 5,4'-dideoxy-5-fluorokanamycin B from 4'-deoxykanamycin B (see "Bull. Chem. Soc. Jpn." 50, page 2362 (1977)); and 5,3',4'-trideoxy-5-fluorokanamycin B from 3',4'-dideoxykanamycin B (namely, dibekacin). Then, the Applicants have further found that these three new compounds, 5,3'-dideoxy-5-fluorokanamycin B, 5,4'-dideoxy-5-fluorokanamycin B and 5,3',4'-trideoxy-5-fluorokanamycin B each exhibit enhanced antibacterial activities.

Furthermore, the Applicants have succeeded in acylating the 1-amino group of these new compounds, 5-deoxy-5-fluorokanamycin B, 5,3'-dideoxy-5-fluorokanamycin B, 5,4'-dideoxy-5-fluorokanamycin B and 5,3',4'-trideoxy-5-fluorokanamycin B with 3-amino-2-(RS)-or (S)-hydroxypropionyl group or 4-amino-2-(S)-hydroxybutyryl group to prepare the corresponding 1-N-(α-hydroxy-ω-aminoalkanoyl) derivatives of the above 5-fluorokanamycin B compounds, respectively, as further new compounds. The Applicants have also found that these new 1-N-(α-hydroxy-ω-aminoalkanoyl) derivatives of the above 5-fluorokanamycin B compounds each exhibit further enhanced antibacterial activities.

According to a first aspect of this invention, therefore, there is provided a 5-deoxy-5-fluorokanamycin B or a derivative thereof represented by the general formula

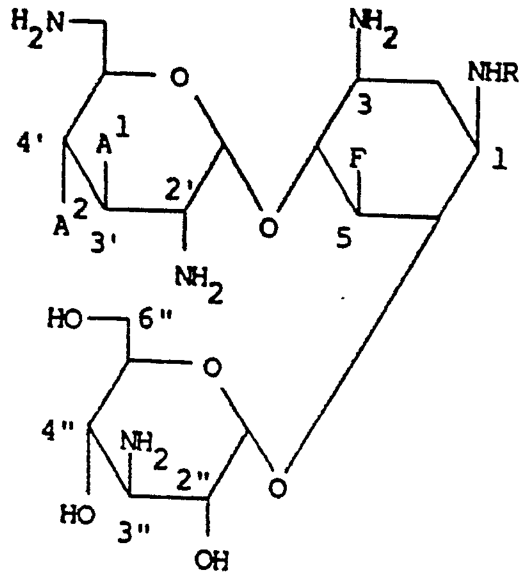

(I)

wherein R is a hydrogen atom or an α-hydroxy-ω-aminoalkanoyl group of the formula

$$-CO-\underset{\displaystyle OH}{\underset{|}{CH}}-(CH_2)_n-NH_2$$

where n is an integer of 1 or 2, and $A^1$ and $A^2$ are independently a hydroxyl group or a hydrogen atom, or a pharmaceutically acceptable acid addition salt thereof.

Examples of 5-deoxy-5-fluorokanamycin B or a derivative thereof having the general formula (I) which are provided according to the first aspect of this invention include thirteen compounds as specified below, and all these specific compounds are in the form of colorless and powdery substances of basic nature which exhibit no definite melting point. The names of these specific compounds are listed below, and their specific optical rotations are also indicated.

(1) 5-Deoxy-5-fluorokanamycin B, namely the compound of the general formula (I) where R is a hydrogen atom and $A^1$ and $A^2$ are each a hydroxyl group (hereinafter nominated as Compound No. 1 of this invention).

$[\alpha]_D^{23}$ + 117° (c 0.6, water).

(2) 5,3'-Dideoxy-5-fluorokanamycin B or 5-deoxy-5-fluorotobramycin, namely the compound of the general formula (I) where R and $A^1$ are each a hydrogen atom and $A^2$ is a hydroxyl group (hereinafter nominated as Compound No. 2 of this invention).

$[\alpha]_D^{23}$ + 124° (c 1.0, water).

(3) 5,4'-Dideoxy-5-fluorokanamycin B, namely the compound of the general formula (I) where R and $A^2$ are each a hydrogen atom and $A^1$ is a hydroxyl group (Compound No. 3 of this invention).

$[\alpha]_D^{23}$ + 120° (c 1.0, water).

(4) 5,3',4'-Trideoxy-5-fluorokanamycin B, namely the compound of the general formula (I) where R, $A^1$ and $A^2$ are each a hydrogen atom (Compound No. 4 of this invention).

$[\alpha]_D^{24}$ + 122° (c 1, water).

(5) 1-N-(3-Amino-2-(S)-hydroxypropionyl)-5-deoxy-5-fluorokanamycin B, namely the compound of the general formula (I) where R is a 3-amino-2-(S)-hydroxypropionyl group, and $A^1$ and $A^2$ are each a hydroxyl group (Compound No. 5 of this invention).

$[\alpha]_D^{23}$ + 83° (c 1, water).

(6) 1-N-(4-Amino-2-(S)-hydroxybutyryl)-5-deoxy-5-fluorokanamycin B, namely the compound of the general formula (I) where R is a 4-amino-2-(S)-hydroxybutyryl group, and $A^1$ and $A^2$ are each a hydroxyl group (Compound No. 6 of this invention).

$[\alpha]_D^{23}$ + 85° (c 1, water).

(7) 1-N-(3-Amino-2-(RS)-hydroxypropionyl)-5,3'-dideoxy-5-fluorokanamycin B, namely the compound of the general formula (I) where R is a 3-amino-2-(RS)-hydroxypropionyl group, $A^1$ is a hydrogen atom and $A^2$ is a hydroxyl group (Compound No. 7 of this invention).

$[\alpha]_D^{23}$ + 75° (c 1, water).

(8) 1-N-(3-Amino-2-(S)-hydroxypropionyl)-5,3'-dideoxy-5-fluorokanamycin B, namely the compound of the general formula (I) where R is a 3-amino-2-(S)-hydroxypropionyl group, $A^1$ is a hydrogen atom and $A^2$ is a hydroxyl group (Compound No. 8 of this invention).

$[\alpha]_D^{23}$ + 79° (c 1.0, water).

(9) 1-N-(4-Amino-2-(S)-hydroxybutyryl)-5,3'-dideoxy-5-fluorokanamycin B, namely the compound of the general formula (I) where R is a 4-amino-2-(S)-hydroxybutyryl group, $A^1$ is a hydrogen atom and $A^2$ is a hydroxyl group (Compound No. 9 of this invention).

$[\alpha]_D^{23}$ + 77° (c 3.0, water).

(10) 1-N-(4-Amino-2-(S)-hydroxybutyryl)-5,4'-dideoxy-5-fluorokanamycin B, namely the compound of the general formula (I) where R is a 4-amino-2-(S)-hydroxybutyryl group, $A^1$ is a hydroxyl group and $A^2$ is a hydrogen atom (Compound No. 10 of this invention).

$[\alpha]_D^{25}$ + 82° (c 1, water).

(11) 1-N-(3-Amino-2-(RS)-hydroxypropionyl)-5,3',4'-trideoxy-5-fluorokanamycin B, namely the compound of the general formula (I) where R is a 3-amino-2-(RS)-hydroxypropionyl group, and $A^1$ and $A^2$ are each a hydrogen atom (Compound No. 11 of this invention).

$[\alpha]_D^{25}$ + 82° (c 1, water).

(12) 1-N-(3-Amino-2-(S)-hydroxypropionyl)-5,3',4'-trideoxy-5-fluorokanamycin B, namely the compound of the general formula (I) where R is a 3-amino-2-(S)-hydroxypropionyl group, and $A^1$ and $A^2$ are each a hydrogen atom (Compound No. 12 of this invention).

$[\alpha]_D^{25}$ + 82° (c 1, water).

(13) 1-N-(4-Amino-2-(S)-hydroxybutyryl)-5,3',4'-trideoxy-5-fluorokanamycin B, namely the compound of the general formula (I) where R is a 4-amino-2-(S)-hydroxybutyryl group, and $A^1$ and $A^2$ are each a hydrogen atom (Compound No. 13 of this invention).

$[\alpha]_D^{25}$ + 83° (c 1, water).

The antibacterial activity of the compounds of this invention according to the general formula (I) as above was estimated by determining their minimum inhibitory concentrations (MIC., mcg/ml) against the growth of various kinds of bacteria by a standard serial dilution method on Mueller Hinton agar medium as incubated at 37°C for 18 hours (but Mycobacterium 607 incubated for 40 hours). The antibacterial spectra so determined of the new compounds No. 1 to No. 13 (each in the form of the free base) of this invention are as shown in Table 1 below. For comparison, the antibacterial spectra of kanamycin B (abbreviated as KMB), 3'-deoxykanamycin B, namely tobramycin (abbreviated as TMB), 3',4'-dideoxykanamycin B, namely dibekacin (abbreviated as DKB) and 1-N-(4-amino-2-(S)-hydroxybutyryl)-kanamycin A, namely amikacin (abbreviated as AMK) were determined in the same manner as above and are as shown in Table 1, too.

Table I-(1)

| Tested Microorganisms | Resistance mechanism | MIC. (mcg/ml) Compound No. 1 of this invention | Compound No. 2 of this invention | Compound No. 3 of this invention | Compound No. 4 of this invention | Compound No. 5 of this invention | Compound No. 6 of this invention |
|---|---|---|---|---|---|---|---|
| Staphylococcus aureus 209P | | 0.39 | 0.39 | 0.39 | 0.78 | 0.39 | 0.39 |
| Staphylococcus aureus Ap01 | ANT(4') | 25 | 0.39 | 0.78 | 3.12 | 6.25 | 6.25 |
| Bacillus subtilis PCI219 | | 0.39 | 0.78 | 0.39 | 0.78 | 0.78 | 0.39 |
| Corynebacterium bovis 1810 | | 0.78 | 1.56 | 0.78 | 6.25 | <0.2 | <0.2 |
| Escherichia coli K-12 | | 0.78 | <0.2 | 1.56 | 3.12 | <0.2 | <0.2 |
| Escherichia coli K-12 R5 | AAC(6') | 100 | 25 | 100 | 100 | 12.5 | 12.5 |
| Escherichia coli K-12 ML 1629 | APH(3')-I | >100 | 0.78 | >100 | 3.12 | 0.78 | 0.78 |
| Escherichia coli K-12 LA 290 R55 | ANT(2") | 3.12 | 3.12 | 6.25 | 6.25 | 1.56 | 0.78 |
| Escherichia coli JR225 | AAC(3) | 0.78 | 0.78 | 6.25 | 3.12 | 0.2 | <0.2 |
| Escherichia coli JR66/W677 | {APH(3')-II ANT(2") | >100 | 0.78 | >100 | 3.12 | 1.56 | 0.78 |
| Mycobacterium 607 | | 1.56 | 0.78 | 3.12 | 6.25 | 6.25 | 12.5 |
| Klebsiella pneumoniae 22#3038 | {APH(3')-II ANT(2") | >100 | 3.12 | >100 | 6.25 | 3.12 | 3.12 |
| Proteus rettgeri GN 311 | | <0.2 | <0.2 | 0.2 | 0.78 | <0.2 | <0.2 |
| Serratia marcescens | | 6.25 | 6.25 | 12.5 | 12.5 | 1.56 | 1.56 |
| Providencia sp. Pv 16 | AAC(2') | 3.12 | 3.12 | 6.25 | 12.5 | 0.78 | 0.78 |
| Pseudomonas aeruginosa A3 | | 1.56 | <0.2 | 3.12 | 0.39 | 0.2 | <0.2 |
| Pseudomonas aeruginosa GN 315 | AAC(6') | 100 | 50 | 100 | 100 | 6.25 | 6.25 |

Table I-(2)

| Tested Microorganisms | Resistance mechanism | MIC. (mcg/ml) Compound No. 7 of this invention | Compound No. 8 of this invention | Compound No. 9 of this invention | Compound No. 10 of this invention | Compound No. 12 of this invention | Compound No.13 of this invention |
|---|---|---|---|---|---|---|---|
| Staphylococcus aureus 209P | | 0.39 | 0.39 | 0.39 | 0.39 | 0.39 | 0.39 |
| Staphylococcus aureus Ap01 | ANT(4') | 1.56 | 0.78 | 0.78 | 0.39 | 0.78 | 0.78 |
| Bacillus subtilis PCI219 | | 0.2 | 0.2 | <0.2 | 0.39 | 0.39 | 0.39 |
| Corynebacterium bovis 1810 | | <0.2 | <0.2 | <0.2 | <0.2 | 0.2 | <0.2 |
| Escherichia coli K-12 | | 0.2 | 0.2 | <0.2 | 0.2 | 0.2 | 0.2 |
| Escherichia coli K-12 R5 | AAC(6') | 6.25 | 6.25 | 3.12 | 12.5 | 50 | 50 |
| Escherichia coli K-12 ML 1629 | APH(3')-I | 0.78 | 0.78 | 0.78 | 0.78 | 0.78 | 0.39 |
| Escherichia coli K-12 LA 290 R55 | ANT(2") | 0.78 | 1.56 | 0.78 | 1.56 | 1.56 | 0.78 |
| Escherichia coli JR225 | AAC(3) | 0.78 | 0.78 | 0.39 | 0.78 | 0.39 | 0.2 |
| Escherichia coli JR66/W677 | {APH(3')-II ANT(2") | 1.56 | 0.78 | 0.78 | 1.56 | 1.56 | 0.78 |
| Mycobacterium 607 | | 0.78 | 0.78 | 0.78 | 6.25 | 0.78 | 0.78 |
| Klebsiella pneumoniae 22#3038 | {APH(3')-II ANT(2") | 3.12 | 1.56 | 1.56 | 3.12 | 1.56 | 0.78 |
| Proteus rettgeri GN 311 | | 0.78 | 0.39 | <0.2 | 0.2 | <0.2 | <0.2 |
| Serratia marcescens | | 3.12 | 3.12 | 3.12 | 3.12 | 1.56 | 1.56 |
| Providencia sp. Pv 16 | AAC(2') | 0.78 | 0.78 | 0.78 | 0.78 | 1.56 | 0.78 |
| Pseudomonas aeruginosa A3 | | 0.39 | 0.39 | 0.39 | 0.39 | 0.39 | 0.39 |
| Pseudomonas aeruginosa GN 315 | AAC(6') | 6.25 | 6.25 | 6.25 | 6.25 | 6.25 | 6.25 |

Table I-(3)

| Tested Microorganisms | Resistance mechanism | MIC. (mcg/ml) KMB (Comparative) | TMB (Comparative) | DKB (Comparative) | AMK (Comparative) |
|---|---|---|---|---|---|
| Staphylococcus aureus 209P | | 0.39 | <0.2 | 0.39 | 1.56 |
| Staphylococcus aureus Ap01 | ANT(4') | 25 | 1.56 | 0.78 | 3.12 |
| Bacillus subtilis PCI219 | | <0.2 | <0.2 | 0.39 | 1.56 |
| Corynebacterium bovis 1810 | | 0.78 | 6.25 | 6.25 | 1.56 |
| Escherichia coli K-12 | | 0.39 | 0.39 | 0.2 | 0.78 |
| Escherichia coli K-12 R5 | AAC(6') | 50 | 100 | 50 | 25 |
| Escherichia coli K-12 ML 1629 | APH(3')-I | >100 | 0.78 | 0.78 | 1.56 |
| Escherichia coli K-12 LA 290 R55 | ANT(2") | 12.5 | 25 | 50 | 1.56 |
| Escherichia coli JR225 | AAC(3) | 12.5 | 25 | 100 | 1.56 |
| Escherichia coli JR66/W677 | {APH(3')-II ANT(2") | >100 | 12.5 | 25 | 3.12 |
| Mycobacterium 607 | | 1.56 | 0.78 | 1.56 | 0.78 |
| Klebsiella pneumoniae 22#3038 | {APH(3')-II ANT(2") | >100 | 12.5 | 50 | 3.12 |
| Proteus rettgeri GN 311 | | 0.2 | 0.2 | 0.2 | 0.78 |
| Serratia marcescens | | 3.12 | 12.5 | 25 | 6.25 |
| Providencia sp. Pv 16 | AAC(2') | 12.5 | 6.25 | 25 | 0.78 |
| Pseudomonas aeruginosa A3 | | 1.56 | <0.2 | 0.2 | 1.56 |
| Pseudomonas aeruginosa GN 315 | AAC(6') | 50 | 50 | 100 | 25 |

The new compound of the general formula (I) according to this invention is usually obtained in the form of a free base, a hydrate or a carbonate thereof. The new compound of the formula (I) may, if desired, be converted into a pharmaceutically acceptable, non-toxic acid addition salt thereof in a known manner by reacting the free base form of said compound with a pharmaceutically acceptable inorganic acid such as hydrochloric acid, sulfuric acid, phosphoric acid, nitric acid and the like; or with a pharmaceutically acceptable organic acid such as acetic acid, malic acid, citric acid, ascorbic acid, methanesulfonic acid and the like.

A compound of the general formula (I) according to this invention or an acid addition salt thereof may be mixed with a pharmaceutically acceptable liquid or solid carrier or vehicle to prepare a pharmaceutical composition, especially an antibacterial composition which may be administered for therapeutic treatment of various bacterial infections in animals, including humans.

According to another aspect of this invention, therefore, there is provided a pharmaceutical composition comprising a compound of the general formula (I) as described above or a pharmaceutically acceptable acid addition salt thereof, as the active ingredient, in association with a pharmaceutically acceptable solid or liquid carrier for the active ingredient.

As will be clear from the antibacterial data of Table 1 above, the compound of the general formula (I) according to this invention can exhibit high antibacterial activity against a variety of bacteria and show a wide and excellent antibacterial spectrum. It has also been found that, unexpectedly, the compounds of this invention show improvement in exhibiting very much lower acute toxicity upon intravenous administration into mice, as demonstrated by the fact that Compounds No. 2, No. 4, No. 9 and No. 13 of this invention, for instance, showed $LD_{50}$ values of 200, 140, 150 and 150 mg/kg, respectively, upon intravenous injection to mice, whereas kanamycin B, 3'-deoxykanamycin B (tobramycin), 3',4'-dideoxykanamycin B and 1-N-(4-amino-2-(S)-hydroxybutyryl)-3',4'-dideoxykanamycin B (namely, habekacin) show $LD_{50}$ values of 110, 80, 80 and 90 mg/kg, respectively, in the same test of acute toxicity.

For the production of the compound of the general formula (I) where R is a hydrogen atom, which is covered by the new compounds having the general formula (I), there is provided according to the third aspect of this invention a process for the production of 5-deoxy-5-fluorokanamycin B or a derivative thereof represented by the general formula

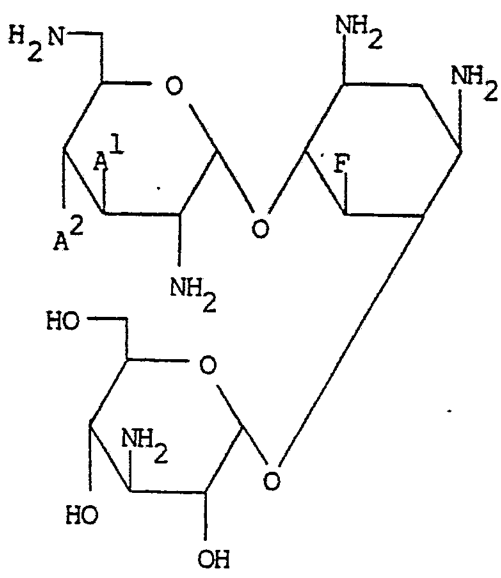

(Ia)

wherein $A^1$ and $A^2$ are independently a hydroxyl group or a hydrogen atom, which comprises (i) reacting an N,O-protected 5-epi-kanamycin B derivative represented by the general formula

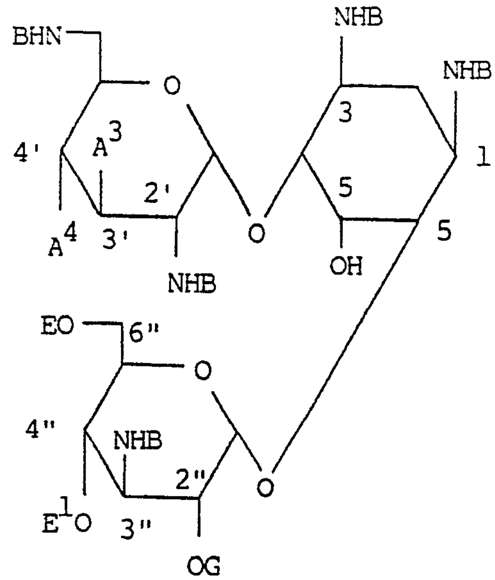

(II)

wherein $A^3$ and $A^4$ are independently a protected hydroxyl group -OG or a hydrogen atom, where G denotes an acyl group as the hydroxyl-protecting group, B is an amino-protecting group, and E and $E^1$ are each an acyl group of the same nature as the aforesaid group G and are the hydroxyl-protecting group, or E and $E^1$ as taken together form a di-valent hydroxyl-protecting group; and the group G present at the 2"-hydroxyl group is an acyl group of the same nature as the aforesaid group G and is the hydroxyl-protecting group, with a dialkylaminosulfur trifluoride of the formula

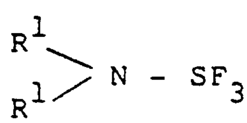

(III)

wherein $R^1$ is an alkyl group of 1 to 4 carbon atoms, or with a bis(dialkylamino)-sulfur difluoride of the formula

$R^1N-SF_2-NR^1$ (III')

wherein $R^1$ is as defined above, or with a fluorination agent equivalent to the compound of formula (III) or (III') in a non-polar organic solvent, to effect the steric inversion of the epi-hydroxyl group at the 5-position of the compound of formula (II) and also the replacement of the 5-hydroxyl group by a fluoro substituent, thereby producing an N,O-protected 5-deoxy-5-fluorokanamycin B derivative represented by the formula

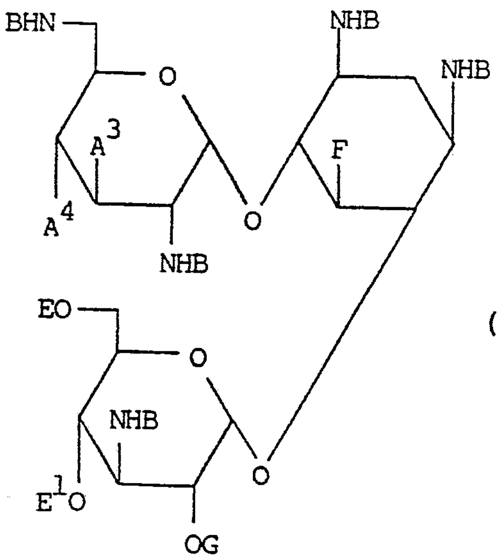

(IV)

wherein $A^3$, $A^4$, B, E, $E^1$ and G are as defined above, and then (ii) removing the remaining amino-protecting group (B) and the remaining hydroxyl-protecting groups (E, $E^1$ and G) from the compound of formula (IV) in a known manner.

The preparation of the N,O-protected 5-epi-kanamycin B derivatives of formula (II) which may be used as the starting compound in the process according to the third aspect of this invention will be illustrated hereinafter in detail.

Amino-protecting group B for each of the five amino groups at 1-, 3-, 2'-, 6'-and 3"-positions of the starting compounds of formula (II) may be any of the known amino-protecting groups which are insusceptible to the reaction intended, and it may be, for example, an acyl group, including an alkanoyl group such as acetyl and trifluoroacetyl; and an aroyl group such as benzoyl; an alkoxycarbonyl group such as methoxycarbonyl, ethoxycarbonyl and butoxycarbonyl; an aralkyloxycarbonyl group such as benzyloxycarbonyl and phenetyloxycarbonyl; an aryloxycarbonyl group such as phenoxycarbonyl and methoxyphenoxycarbonyl; and a sulfonyl type group, including an alkylsulfonyl group, an aralkylsulfonyl group such as benzylsulfonyl and an arylsulfonyl group such as tosyl.

In cases where the starting compound of formula (II) has the hydroxyl group(s) at the 3'-and/or 4'-position thereof, it is necessary that the hydroxyl groups at the 3'-and/or 4'-position and the 2"-position should be protected by a known hydroxyl-protecting group (G) which may be selected from an acyl group, preferably an alkanoyl or an aroyl group. Similarly, the 4"-and 6"-hydroxyl groups need to have been protected by known hydroxyl-protecting groups (E, $E^1$) which may be selected from an acyl group, preferably an alkanoyl or an aroyl group. The acyl group for the protection of an hydroxyl group may be an alkanoyl group, typically an alkanoyl group of 2-5 carbon atoms such as acetyl, propionyl and butyryl, or an aroyl group, typically phenylcarbonyl which may have alkyl substituent(s) on the phenyl ring, preferably benzoyl. Alternatively, the 4"-hydroxyl-protecting group ($E^1$) and 6"-hydroxyl-protecting group (E) as taken together may form a single known di-valent hydroxyl-protecting group, for example, an alkylidene group of 2-8 carbon atoms such as ethylidene and isopropylidene, a cycloalkylidene group such as cyclohexylidene, or a tetrahydropyranylidene group. It is convenient that the protecting groups $E^1$ and E as taken together form an isopropylidene or a cyclohexylidene group.

Examples of the dialkylaminosulfur trifluoride of formula (III), which may be used as the fluorination agent, include typically dimethylaminosulfur trifluoride, diethylaminosulfur trifluoride and dipropylaminosulfur trifluoride. Exemplary of the bis(dialkylamino)-sulfur difluorides of formula (III') which may also be used as the fluorination agent are bis(dimethylamino)-sulfur difluoride and bis(diethylamino)-sulfur difluoride. All of these compounds are known fluorinating agents [see, for example, "J.Org.Chem.", 40, No. 5, 574-578 (1975)].

The reaction between a compound of formula (II) and a fluorination agent of formula (III) or (III') may be carried out in a non-polar organic solvent, for example, an aromatic hydrocarbon such as benzene, toluene and xylene or a halogenated hydrocarbon, preferably chlorinated hydrocarbon such as chloromethane, dichloromethane and chloroform, at a temperature in the range of -70°C - +50°C, preferably at room temperature in anhydrous conditions. In general, the fluorination agent of formula (III) or (III') may be used in an amount of 1-10 moles per mole of the compound of formula (II). An amine such as tertiary alkyl amine or pyridine may be present as the acid binding agent in the reaction mixture.

The step for the simultaneous fluorination and inversion of the 5-hydroxyl group in the process above may alternatively be effected by sulfonylating the 5-hydroxyl group (for example, by alkylsulfonylating such as mesylating or trifluoromethylsulfonylating or by aralkylsulfonylating such as benzylsulfonylating), followed by subjecting the resulting 5-sulfonyloxy derivative to the action of a conventional fluorinating reagent, for example, a tetraalkylammonium fluoride (such as tetrabutylammonium fluoride), potassium fluoride and cesium fluoride, to remove the 5-sulfonyloxy group and introduce a fluoro-substituent simultaneously. Arylsulfonylation such as tosylation is not effective to achieve the intended sulfonylation of the 5-hydroxyl group. The sulfonylation reaction may be carried out by dissolving the starting compound of formula (II) in a solvent such as pyridine, adding to the solution an alkylsulfonyl or aralkylsulfonyl halogenide such as methylsulfonyl chloride or benzylsulfonyl chloride or a corresponding anhydride in an amount of 1-10 molar equivalent and conducting the reaction at a temperature in the range of -20°C - +100°C, preferably at room temperature under anhydrous conditions. The fluorination of the 5-O-sulfonyl derivative may also be effected in such solvents as ethylether, tetrahydrofuran, acetonitrile, dimethylformamide, sulfolane, hexamethyl phosphortriamide with the addition of an excess amount of a fluorinating agent such as tetraethyl-or tetrabutylammonium fluoride, cesium fluoride,or potassium fluoride containing a crown ether,at a temperature in the range of 0° - 150°C.

After the completion of the fluorination of the 5-hydroxyl group, the reaction solution is added to an aqueous solution of an alkali metal carbonate or an alkali metal hydrogen carbonate, preferably an aqueous sodium hydrogen carbonate, to neutralize the acidic matter. The resulting mixture so neutralized is extracted with chloroform and the extract is washed with water, dried and concentrated under reduced pressure to remove the chloroform and to leave the N,O-protected 5-deoxy-5-fluorokanamycin B derivative of formula (IV) as a solid.

The amino-protecting groups (B) and the hydroxyl-protecting groups (E, $E^1$ and G) in the compound of formula (IV) may be removed by a known deprotecting method, separately. Thus, an amino-protecting group of the alkoxycarbonyl or aryloxycarbonyl type may be removed by an alkaline hydrolysis, whereas an amino-protecting group of the aralkyloxycarbonyl type may be removed by an alkaline hydrolysis or by reduction. An amino-protecting group of the sulfonyl type may also be removed in a known manner by treating the compound of formula (IV) with metallic sodium in liquefied ammonia (see, for example, U.K. Patent No. 1555661 and Japanese Patent Publication No. 29720/85). A hydroxyl-protecting group of the acyl type (E, $E^1$, G) may be removed by hydrolysis in an aqueous alkali metal hydroxide solution, e.g. an aqueous sodium hydroxide solution. In cases where $E^1$ and E taken together form a single di-valent hydroxyl-protecting group of the alkylidene, cycloalkylidene or tetrahydropyranylidene type, such a group may be removed by hydrolysis in the presence of an inorganic acid, an organic acid or a cation exchange resin of strongly acidic nature, e.g. a cation exchange resin of sulfonic acid type (see, for example, U.K. Patent No. 2, 043,634).

The removal of all the protective groups in the compound of formula (IV) results in the formation of the desired compound of formula (Ia). The isolation and purification of the compound of formula (Ia) may suitably be effected by chromatography on a molecular sieve agent such as CM-Sephadex® C-25 (a product of Pharmacia Co.) as eluted with aqueous ammonia in a gradient elution technique.

Further, the production of the compound of the general formula (I) where R is an α-hydroxy-ω-aminoalkanoyl group may be achieved according to this invention. Thus, according to the fourth aspect of this invention, there is provided a process for the production of a 1-N-(α-hydroxy-ω-aminoalkanoyl)-5-deoxy-5-fluorokanamycin B derivative represented by the general formula

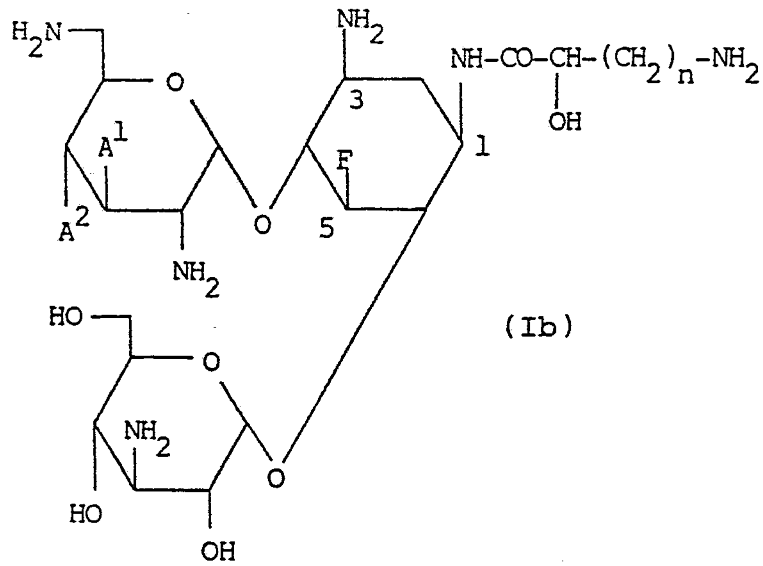

(Ib)

wherein $A^1$ and $A^2$ are independently a hydroxyl group or a hydrogen atom and n is an integer of 1 or 2, which comprises (i) reacting or acylating the 1-amino group of 5-deoxy-5-fluorokanamycin B or a derivative thereof represented by the general formula

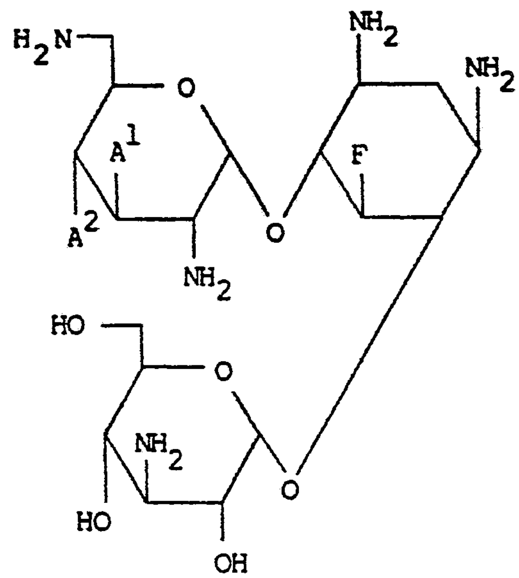

(Ia)

wherein $A^1$ and $A^2$ are each as defined above, or the 1-amino group of such protected derivative of the compound of formula (Ia) some or all of whose amino groups other than the 1-amino group has or have been protected by amino-protecting groups, with an α-hydroxy-ω-aminoalkanoic acid represented by the general formula

$$HOOC-CH(OH)-(CH_2)_n-NH_2 \quad (V)$$

wherein n is an integer of 1 or 2, or such protected derivative of said aminoalkanoic acid of formula (V) whose amino group has been protected by an amino-protecting group, or an equivalent reactive derivative thereof, and then (ii) removing from the resulting 1-N-acylated product compound the remaining amino-protecting group when the latter groups exist, to produce the compound of formula (Ib).

In the process according to the fourth aspect of this invention, in general, the amino-protecting groups which may be used to protect all or some of the amino groups other than the 1-amino group of the starting 5-deoxy-5-fluorokanamycin B derivative of formula (Ia) may be any of those ordinarily used as the amino-protecting groups. In most cases, the amino-protecting groups are selected from monovalent amino-protecting groups, including typically an alkoxycarbonyl group such as tert-butoxycarbonyl and tert-amyloxycarbonyl; a cycloalkyloxycarbonyl group such as cyclohexyloxycarbonyl; an aralkyloxycarbonyl group such as benzyloxycarbonyl; a hydrolytically cleavable, substituted lower alkanoyl group such as trifluoroacetyl and o-nitrophenoxyacetyl; a phosphinothioyl group such as diphenylphosphinothioyl and dimethylphosphinothioyl; a phosphinyl group such as diphenylphosphinyl, and the like. The amino-protecting groups may also be in a divalent form such as phthaloyl or in the form of a Schiff base with the amino group to be protected. The introduction of amino-protecting groups of these kinds may be conducted by reacting the compound of formula (Ia) with an appropriate known reagent for introduction of the amino-protecting group which may be in the form of an acylating agent such as an acid halide, acid azide, active ester, acid anhydride and the like, in the manner known per se in the conventional synthesis of peptides. By choosing the quantity of the reagent for introduction of the amino-protecting group employed in a proportion of 0.5 to 6 moles per mole of the compound of formula (Ia), it is possible to prepare a mixture of different, partially amino-protected derivatives at any ratio, due to the difference in the reactivity of the respective amino groups of the compound of formula (Ia).

In the process for the production of compounds of formula (Ib) according to the fourth aspect of this invention, it is possible to use, as the starting compound, such amino-protected derivative of the compounds of formula (Ia) all or some of whose amino groups other than the 1-amino group have been protected, for example, a 3,2',6',3"-tetra-N-protected derivative, a 3,2',6'-tri-N-protected derivative, a 6',3"-di-N-protected derivative or a 6'-mono-N-protected derivative. Of course, a mixture of two or more of those partially N-protected derivatives may also be used, without being purified or isolated, for the 1-N-acylation step of this process.

In order to ensure that the desired compound of general formula (Ib) can be produced in a high yield by the process according to the fourth aspect of this invention, only the 1-amino group of the starting compound of formula (Ia) needs to be selectively acylated with the α-hydroxy-ω-amino-alkanoic acid of formula (V). Accordingly, it will be evident that most preferably, such a protected derivative of the compound of formula (Ia) in which all the amino groups other than the 1-amino group have been protected, namely a 3,2',6',3"-tetra-N-protected-5-deoxy-5-fluorokanamycin B derivative is employed as the starting substance to be 1-N-acylated in this process.

To prepare such a 3,2',6',3"-tetra-N-protected-5-deoxy-5-fluorokanamycin B derivative from the compound of formula (Ia), the following procedure may conveniently be used, for instance. Thus, a 3,2',6'-tri-N-protected derivative of 5-deoxy-5-fluorokanamycin B is first prepared from 5-deoxy-5-fluorokanamycin B of formula (Ia) according to the method of U.S. Patent No. 4,136,254 (corresponding to Japanese patent application first publication "Kokai" No. 153944/77) comprising reacting 5-deoxy-5-fluorokanamycin B with a cation of a di-valent transition metal such as copper (II), nickel (II), cobalt (II) and others for the formation of a metal complex, reacting the metal complex with an acylation reagent known as the amino-protective group-introducing agent for the protective N-acylation of all the amino groups other than the two 1-and 3"-amino groups of the kanamycin B moiety in the metal complex [the 1-and 3"-amino groups having been blocked by complexing with the di-valent transition metal cation], and removing the di-valent metal cation from the N-acylated metal complex, e.g. by treatment with a cation-exchange resin or by treatment with hydrogen sulfide or aqueous ammonia to afford a 3,2',6'-tri-N-acylated derivative of 5-deoxy-5-fluorokanamycin B, or according to the method of Claim 1 of U.S. Patent No. 4,297,485 (corresponding to Japanese patent application first publication "Kokai" No. 64598/80; Japanese patent application No. 138402/78) comprising reacting 5-deoxy-5-fluorokanamycin B with zinc cation instead of the above-mentioned di-valent transition metal cation and subsequently processing the resultant zinc complex in a similar way to the above-mentioned method of U.S. patent No. 4,136,254. In this way, a 3,2',6'-tri-N-protected 5-deoxy-5-fluorokanamycin B derivative can be prepared from the compound of formula (Ia) in a high yield. The 3"-amino group of the 3,2',6'-tri-N-protected-5-deoxy-5-fluorokanamycin B derivative so prepared can further be protected according to the selective 3"-N-acylation method of claim 15 of U.S. patent No. 4,297,485 (corresponding to Japanese patent application first publication "Kokai" No. 164696/80; Japanese patent application No. 73064/79) for the production of an amino-protected derivative of an aminoglycoside antibiotic of which all the amino groups other than the 1-amino group have been protected selectively, so that a 3,2',6',3"-tetra-N-protected derivative of the compound of formula (Ia) can be prepared in a high yield. In accordance with the selective 3"-N-acylation method of claim 15 of U.S. patent No.

4,297,485, there may be used as a selective 3"-N-acylating reagent an ester, typically an alkyl ester of formic acid an ester, typically an alkyl ester of a di-halo-or tri-halo-alkanoic acid or N-formylimidazole, whereby the 3"-amino group can be protected selectively with the formyl or di-or tri-haloalkanoyl group in a high yield, without involving the acylation of the 1-amino group of said 3,2',6'-tri-N-protected derivative.

In the process according to the fourth aspect of this invention, the 1-amino group of the compound of formula (Ia) or the 1-amino group of a partially amino-protected derivative or derivatives of the compound of formula (Ia) may be acylated with the α-hydroxy-ω-amino-alkanoic acid of formula (V) in which the amino group has been protected or not. The 1-N-acylation reaction intended may be conducted according to any conventional method for the synthesis of peptides, for instance, according to so-called dicyclohexylcarbodiimide method, mixed acid anhydride method, azide method, active ester method and the like, using the α-hydroxy-ω-amino-alkanoic acid of formula (V) as such or in the form of a reactive derivative thereof (as a functional equivalent thereof). The amino-protecting group to be used for the protection of the amino group of the α-hydroxy-ω-amino-alkanoic acid of formula (V) may be the same as or different from those used for the protection of amino groups of the starting compound of formula (Ia). Particularly preferred examples of the amino-protecting group for this purpose are tert-butoxycarbonyl group and p-methoxybenzyloxycarbonyl group which are easily cleavable by treatment with aqueous trifluoroacetic or acetic acid or with dilute aqueous hydrochloric acid. A benzyloxycarbonyl group which is removable by a conventional hydrogenolysis in the presence of a catalyst of a platinum group metal, such as palladium or platinum oxide is also a convenient N-protecting group.

The 1-N-acylation of the starting compound of formula (Ia) or of a partially N-protected derivative or derivatives thereof intended may desirably be carried out in an aqueous organic solvent according to the active ester method using the α-hydroxy-ω-amino-alkanoic acid compound of formula (V) in the form of its active ester. For example, N-hydroxysuccinimide ester of (S)-4-tert-butoxycarbonylamino-2-hydroxybutyric acid may preferably be used as the active ester which may be prepared by a conventional method of preparing such active esters. This active ester may usually be used in a proportion of from 1 to 3 molar equivalents and preferably of from 1 to 1.5 molar equivalents per mole of the starting compound of formula (Ia) or a partially amino-protected derivative thereof to be 1-N-acylated. The aqueous organic solvent used as the reaction medium may be a water-miscible organic solvent such as dioxane, 1,2-dimethoxyethane, dimethylformamide (DMF), tetrahydrofuran (THF), triethylamine and the like. The 1-N-acylation may be effected at ambient temperature but generally at a temperature of 0°C-90°C, preferably of 0°C-30°C and for a reaction time of 10 minutes to 18 hours and preferably of 30 minutes to 60 minutes.

When the 1-N-acylation reaction is conducted using as the starting compound a partially amino-protected derivative of compound (Ia) of which some, but not all, of the amino groups other than the 1-amino group has or have been protected, for example, the 6'-N-protected derivative of the starting compound of formula (Ia), the N-acylation products as formed may partially be purified by column chromatography, for example, on silica gel so that the unreacted starting material is removed, giving a mixture of the desired 1-N-mono-acylated product with the otherwise N-acylated products. These mixed acylation products may, without being purified and/or isolated, be subjected directly to the subsequent deprotecting step, followed by the step or steps of purification and isolation so that the desired 1-N-mono-acylated prdouct of formula (Ib) is obtained.

In the second step of the process according to the fourth aspect of this invention, the 1-N-acylated product (including the mixed acylation products) as obtained from the 1-N-acylation step is subjected to the removal of the amino-protecting groups, if they still remain in the 1-N-acylated product. The removal of the protecting groups is effected by a conventional deprotecting technique. Thus, the amino-protecting group of alkoxycarbonyl type is removed by acid hydrolysis with an aqueous solution of trifluoroacetic acid or acetic acid and the like or with a dilute aqueous solution of an inorganic acid such as hydrochloric acid. The aralkyloxycarbonyl group such as benzyloxycarbonyl may easily be removed by an ordinary catalytic reduction (hydrogenolysis). Whan a phthaloyl group remains as the amino-protecting group, it can be removed by heating in a solution of hydrazine hydrate in a lower alkanol such as methanol and ethanol.

It is convenient to conduct the synthesis of the new compounds of formula (Ib) according to this invention by using as the initial compound a non-protected compound of formula (Ia), for example,5-deoxy-5-fluorokanamycin B, and using a multi-step procedure comprising the preparation of an amino-protected derivative from the initial compound according to the zinc-complex method of Japanese patent application "Kokai" No. 64598/80 and the selective 3"-N-protecting method of Japanese patent application "Kokai" No. 164696/80 and subsequent synthesis route according to the fourth aspect of this invention.

Thus, according to this particular multi-step procedure, the starting 5-deoxy-5-fluorokanamycin B of formula (Ia) and zinc acetate are either suspended in dimethylsulfoxide (DMSO) or dissolved in a mixture of water and dimethylformamide (DMF), and the resulting suspension or solution of the 5-deoxy-5-fluorokanamycin B-zinc complex as formed is reacted with N-benzyloxycarbonyloxy-succinimide

( 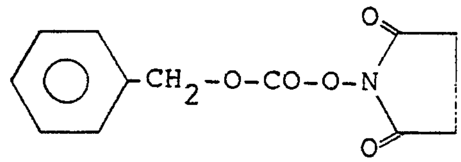

as an amino-protecting benzyloxycarbonyl group-introducing reagent) to protect the 3-, 2′-and 6′-amino groups of the kanamycin B moiety of the zinc complex with the benzyloxycarbonyl groups, followed by removing the zinc cation from the resultant 3,2′,6′-tris-N-benzyloxycarbonylated 5-deoxy-5-fluorokanamycin B-zinc complex by treating with a cation exchange resin such as Amberlite CG-50, to give 3,2′,6′-tris-N-benzyloxycarbonyl-5-deoxy-5-fluorokanamycin B (Compound a) (... Stage 1). Compound a is then reacted with ethyl trifluoroacetate in DMSO or DMF to protect the 3″-amino group of Compound a with the trifluoroacetyl group, affording 3,2′,6′-tris-N-benzyloxycarbonyl-3″-N-trifluoroacetyl-5-deoxy-5-fluorokanamycin B (Compound b) (... Stage 2). Further, Compound b is reacted with N-benzyloxycarbonyl-(S)-4-amino-2-hydroxybutyryloxysuccinimide or with N-benzyloxycarbonyl-(S)-or (RS)-3-amino-2-hydroxypropionyloxysuccinimide in an aqueous tetrahydrofuran (THF) in the presence of sodium carbonate, so that the 1-amino group of Compound b is selectively acylated with the (S)-4-benzyloxycarbonylamino-2-hydroxybutyryl group or with the (S)-or (RS)-3-benzyloxycarbonylamino-2-hydroxypropionyl group (... Stage 3), whereby there is formed 1-N-[(S)-4-(benzyloxycarbonyl)amino-2-hydroxybutyryl]-or 1-N-[(S)-or (RS)-3-(benzyloxycarbonyl)amino-2-hydroxypropionyl)-3,2′,6′-tris-N-(benzyloxycarbonyl)-3″-N-trifluoroacetyl-5-deoxy-5-fluorokanamycin B (Compound c) as the 1-N-acylation product.

Compound c is then subjected to a deprotecting treatment comprising acidic or alkaline hydrolysis for removal of the amino-protecting trifluoroacetyl group therefrom and to subsequent catalytic hydrogenolysis in the presence of a platinum group metal catalyst such as palladium or Raney nickel for removal of the amino-protecting benzyloxycarbonyl groups therefrom (... Stage 4), so that the desired compound of formula (Ib) is afforded.

The preparation of the aforesaid N,O-protected 5-epi-kanamycin B derivatives of formula (II) to be used as the starting compound in the process according to the third aspect of this invention will now be described. This is achievable by a process consisting of a series of reaction steps given below.

The first step for this preparation relates to the introduction of amino-protecting group (B′), which may be selected from alkylsulfonyl, aralkylsulfonyl and arylsulfonyl groups, into the amino groups of kanamycin B, 3′-deoxykanamycin B, 4′-deoxykanamycin B or 3′,4′-dideoxykanamycin B. This may be carried out in a known manner (see, for example, U.K. Patent No. 1,555,661 and Japanese Patent Publication No. 29720/85), that is, by reacting kanamycin B, 3′-deoxykanamycin B, 4′-deoxykanamycin B or 3′,4′-dideoxykanamycin B with a sulfonic acid halide of formula (VI)

$R^3SO_2X$ (VI)

wherein $R^3$ is a lower alkyl group, an aralkyl group, particularly benzyl or an aryl group, particularly phenyl and X is a chlorine or bromine atom, preferably tosyl chloride in an aqueous dioxane in the presence of sodium carbonate at a temperature of 0°-50°C, resulting in the formation of a 1,3,2′,6′,3″-penta-N-protected (i.e. N-sulfonylated)-kanamycin B derivative having formula (VII)

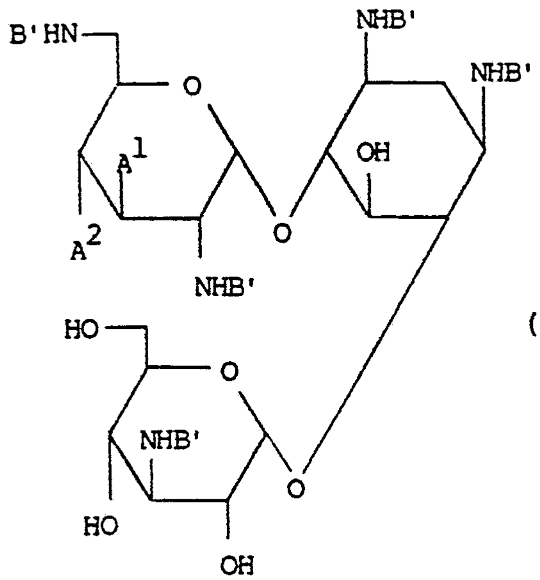

(VII)

wherein A¹ and A² each are a hydroxyl group or hydrogen atom and B′ is an amino-protecting group of sulfonyl type having the formula $R^3SO_2$-where $R^3$ has the same meaning as defined above.

In the second step, all the hydroxyl groups other than the 5-hydroxyl group of the resulting compound of formula (VII) are protected. A first procedure available to this end is to react the compound of formula (VII) with acetyl chloride in an amount of 5 moles or somewhat higher moles per mole of the compound (VII) in dry pyridine at a temperature of 50°C or lower, whereby all the hydroxyl groups except the 5-hydroxyl group of the compound (VII), that is, the 3′,4′,2″,4″ and 6″-hydroxyl groups (provided that the 3′-position and/or the 4′-position has then the hydroxyl group), can be acetylated for the protection thereof. In general, acetyl chloride may be substituted by other acyl chlorides, including other lower alkanoyl chlorides and aroyl chlorides such as benzoyl chloride, with the result that all the hydroxyl groups other than the 5-hydroxyl group of the compound (VII) are protected by acylation without acylating the 5-hydroxyl group, similarly to the case of use of acetyl chloride. Thus, there is afforded a 1,3,2′,6′,3″-penta-N-protected (sulfonylated)-3′,4′,2″,4″,6″-penta-O-protected (acylated)-or -4′,2″,4″,6″-tetra-O-protected(acylated)-or -3′,2″,4″,6″-tetra-O-protected(acylated)-or -2″,4″,6″-tri-O-protected (acylated)-kanamycin B derivative having formula (VIII)

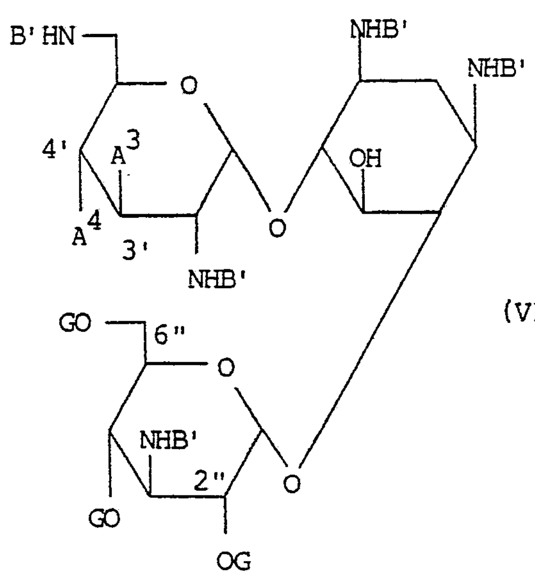

(VIII)

wherein A³ and A⁴ each are a hydrogen or a group -OG, G is an acyl radical derived from the acyl chloride used and B′ has the same meaning as defined above.

An alternative, second procedure to effect the second step as described above comprises protecting both the 4″-and 6″-hydroxyl groups of the compound of formula (VII) with a divalent hydroxyl-protecting group and then protecting the remaining free hydroxyl group or groups other than the 5-hydroxyl group, that is, the 2″-hydroxyl group and, if any, the 3′-and/or 4′-hydroxyl group, with an acyl group, a known hydroxyl-protecting group. In the protection of both the 4″-and 6″-hydroxyl groups by a divalent hydroxyl-protecting group, the reaction for this purpose may be effected by treating the compound of formula (VII) in dry dimethylformamide with a dimethoxyalkane such as 2,2-dimethoxypropane, benzaldehyde, dimethylacetal, a dimethoxycycloalkane such as 1,1-dimethoxycyclohexane (cyclohexanone•dimethylacetal) or 5,6-dihydro-4-methoxy-2H-pyrane in the presence of a catalytic amount of p-toluenesulfonic acid in a known manner as, for example, described in U.K. Patent No. 2,043,643 B, to yield a 4″,6″-O-protected-1,3,2′,6′,3″-penta-N-protected(sulfonylated)-kanamycin B derivative having formula (VIII′)

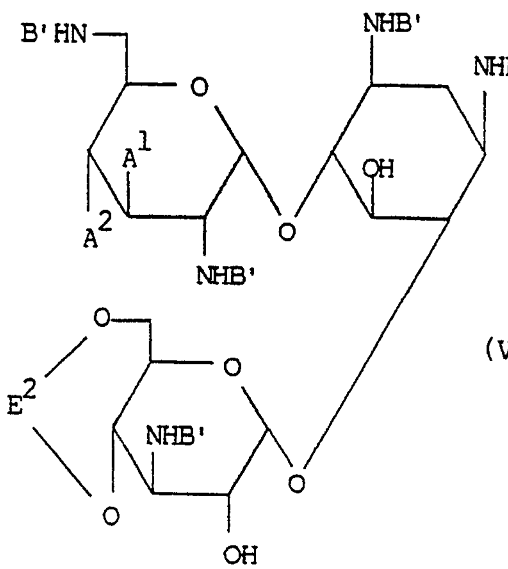

(VIII′)

wherein $A^1$, $A^2$ and B′ have the same meanings as defined above, $-E^2-$is a divalent hydroxyl-protecting group, including an alkylidene group, an aralkylidene group such as benzylidene, a cycloalkylidene group and tetrahydropyranylidene group. The subsequent protection of the remaining hydroxyl group or groups other than the 5-hydroxyl group, that is, the 2″-hydroxy group and, if any, the 3′-hydroxyl group and/or the 4′-hydroxyl group, may be effected by acylation which is known and conventionally used for the protection of hydroxyl group with an acyl group. Examples of the acyl groups available as the hydroxyl-protecting groups may include an alkanoyl group, preferably acetyl and an aroyl group, preferably benzoyl. This acylation reaction may conveniently be carried out by reacting the compound of formula (VIII′) in dry dimethylformamide with such an acyl chloride as referred to above or with an N-acylimidazole, including an N-alkanoylimidazole such as N-acetylimidazole and an N-aroylimidazole such as N-benzoylimidazole, in the presence of pyridine at a temperature of 0°C-50°C, to give the O-protection by an acyl group at the 2″-hydroxyl group and, if any, at the 3′-and/or 4′-hydroxyl-group, but leaving the 5-hydroxyl group free.

Therefore, the above-mentioned alternative second procedure involving the two selective acylation reactions leads to the formation of a 3′,4′,2″,4″,6″-penta-O-protected-or a 3′,2″,4″,6″-tetra-O-protected-or a 4′,2″,4″,6″-tetra-O-protected-or a 2″,4″,6″-tri-O-protected-1,3,2′,6′,3″-penta-N-protected(sulfonylate)-kanamycin B derivative having formula (VIII″)

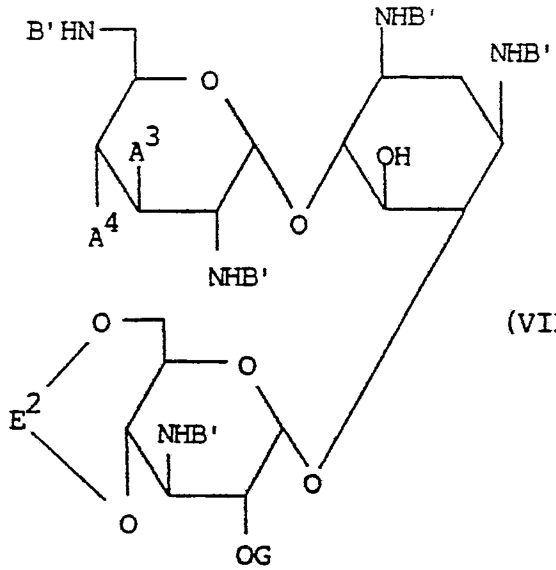

(VIII")

wherein B′ and $E^2$ have the same meanings as defined above, $A^3$ and $A^4$ each are a hydrogen or a group -OG and G is an acyl.

The third step for the preparation of the starting compound of formula (II) relates to the inversion of the 5-hydroxyl group of the compound of either formula (VIII) or formula (VIII") to give the corresponding 5-epimer. This inversion may be effected by utilizing Mitsunobu's reaction in a known manner, that is, by treating the compound of either formula (VIII) or formula (VIII") with triphenylphosphine, diethyl azodicarboxylate ($C_2H_5OC-N=N-COC_2H_5$) and e.g. benzoic acid in an anhydrous polar organic solvent such as dry tetrahydrofuran usually at room temperature. Thus, there is formed a 5-O-benzoyl-5-epi-kanamycin B derivative of formula (IX)

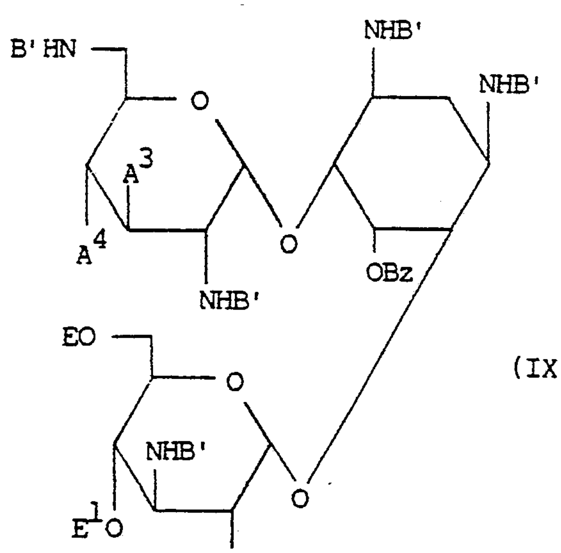

(IX)

wherein $A^3$, $A^4$, B′ and G have the same meanings as defined above, E and $E^1$ each are the same as that of G (an acyl), or E and $E^1$ together form a divalent group as defined for $-E^2-$, and Bz is benzoyl. The benzoic acid mentioned above may be replaced by another organic acid, giving a 5-O-acyl-5-epi-kanamycin B derivative corresponding to the compound of formula (IX). Alternatively, the inversion of the 5-hydroxyl group may be carried out according to a known method by reacting the 5-O-sulfonylated compound of formula (VIII") with sodium acetate or sodium benzoate to give the compound of formula (IX) or a corresponding 5-O-acetyl derivative.

The fourth step is for the purpose of removing the 5-O-benzoyl group from the compound of formula (IX) as obtained in the third step above. This is effected by treating the compound of formula (IX) with a methanolic sodium methylate solution at a temperature from room temperature to the reflux temperature of methanol, with the result that both the 5-O-benzoyl group and the hydroxyl-protecting groups of acyl type on the other hydroxyl groups of the compound of formula (IX) are removed simultaneously. Thus, the fourth step leads to the formation of 5-epi-kanamycin B derivative of formula (X)

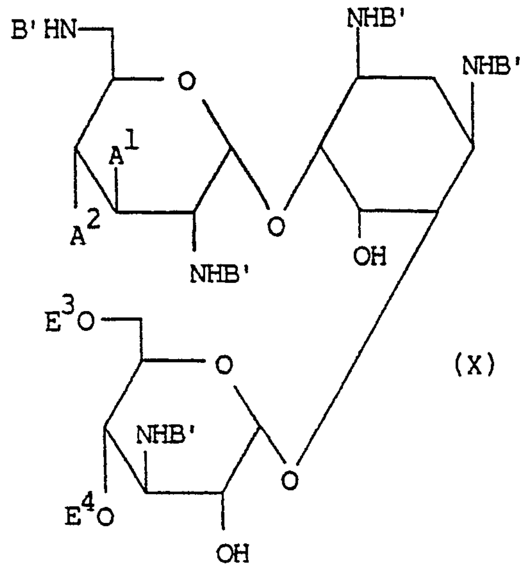

(X)

wherein $A^1$, $A^2$ and B′ have the same meanings as defined above, and $E^3$ and $E^4$ each are hydrogen or $E^3$ and $E^4$ together form a divalent hydroxyl-protecting group $-E^2-$as above-mentioned.

If necessary, the compound of formula (X) is then hydrolyzed in the presence of an inorganic or organic acid, for example, methanesulfonic acid, or a strongly acidic cation exchange resin, to remove the divalent hydroxyl-protecting group $-E^2-$from the 4″-and 6″-hydroxyl groups which are being so protected, and to afford a penta-N-protected(sulfonylated)-5-epi-kanamycin B derivative of formula (X′)

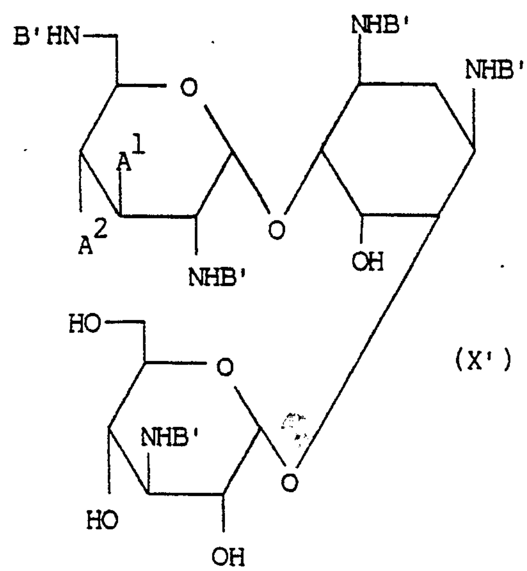

(X′)

wherein $A^1$, $A^2$ and B′ have the same meanings as defined above.

The fifth step relates to a selective O-protection reaction, which is to protect all the hydroxyl groups, but not the 5-hydroxyl groups, of the compound of formula (X) or (X'). To this end, the compound of formula (X) or (X') is reacted with an acyl chloride or an N-acylimidazole, for example, an N-alkanoylimidazole or N-aroylimidazole in dry dimethylsulfoxide or dry pyridine at a temperature of 0°-50°C under an anhydrous condition in the same manner as in the second step above. Under these conditions, only the 5-hydroxyl group remains unprotected with an acyl group. Thus, there is formed an N,O-protected-5-epi-kanamycin B derivative of formula (XI)

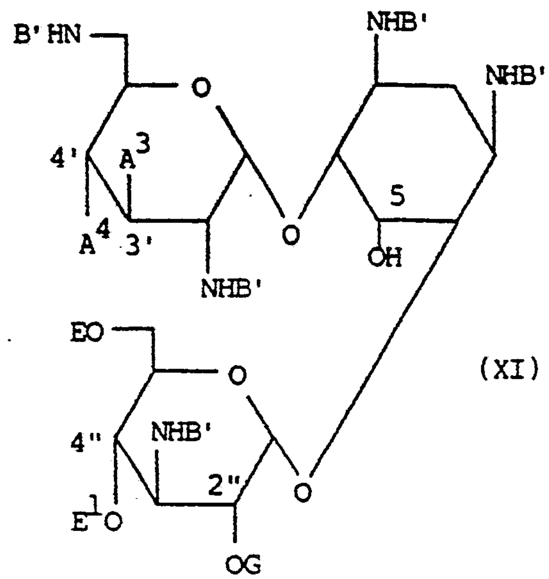

(XI)

wherein B' is an amino-protecting group of sulfonyl type as defined above, $A^3$ and $A^4$ each are hydrogen or a protected hydroxyl group -OG where G is a hydroxyl-protecting group of acyl type as derived from the acyl chloride or N-acylimidazole used above, G in the 2"-OG is also the same acyl group as above, and E and $E^1$ each are an acyl group G as defined above, or E and $E^2$ together form a divalent hydroxyl-protecting group which is the same as $-E^2-$in the compound of formula (X).

If desired, the sixth step may be conducted as a subsequent step for substituting the amino-protecting group of sulfonyl type (B') of the compound of formula (XI), by an amino-protecting group of another type. For this purpose, the compound of formula (XI) is subjected to a known deprotecting reaction, typically by treating with metallic sodium in liquefied ammonia, to give the corresponding O-protected-5-epi-kanamycin B derivative of formula (XII)

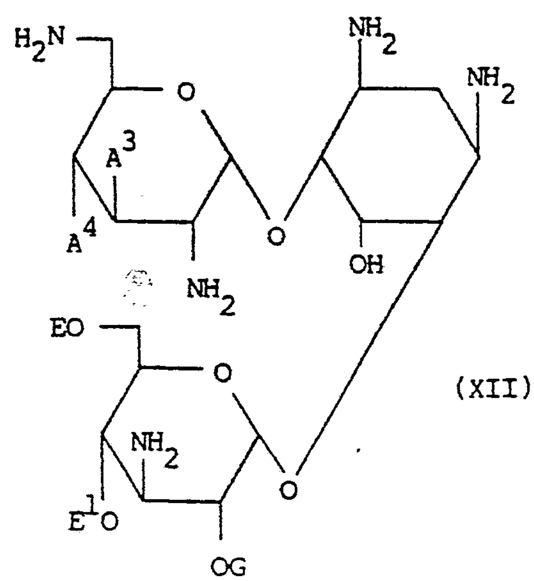

(XII)

wherein $A^3$, $A^4$, E, $E^1$ and G have the same meanings as defined above. Then, the compound of formula (XII) having the five free amino groups is again protected with the desired amino-protecting group B. This may be done by a known or conventional method for N-protection. Typical desired amino-protecting groups known to be used are those of acyl type, including an alkanoyl group such as acetyl, an aroyl group such as benzoyl, an alkoxycarbonyl group such as ethoxycarbonyl and tert-butoxycarbonyl and an aralkyloxycarbonyl group such as benzyloxycarbonyl, and those of sulfonyl type other than B′ group above as desired. Thus, there is finally formed the desired, N,O-protected 5-epi-kanamycin B derivative of formula (II)

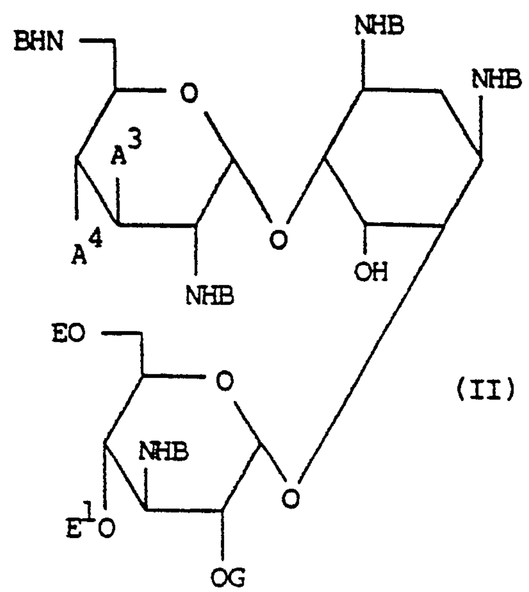

(II)

wherein $A^3$, $A^4$, E, $E^1$ and G have the same meanings as defined above and each B is an amino-protecting group as introduced in the sixth step.

The compound of formula (XI) or (II) which is prepared by the process comprising the first to the fifth steps or the first to the sixth steps as described above, respectively, is then used as the starting compound in the process according to the third aspect of this invention for the fluorine-substitution on the 5-epi-hydroxyl group.

The pharmaceutical composition according to the aforesaid second aspect of this invention may be formulated into suitable forms for oral, parenteral or intrarectal administration. A composition in the form of an injectable solution may contain 0.1% to 20.0% by weight of the compound (I) as active ingredient, and also one or more of a pH-adjuster, buffer, stabilizer, excipient, local anesthetic and an additive for rendering the solution isotonic. The injectable solution may be prepared to be adapted for subcutaneous, intramuscular or intravenous injection by any conventional pharmaceutical technique. Solid compositions for oral administration which may be in the form of tablets, coated tablets, granules, powder and capsules, may contain excipients for the active ingredient, and if required, other additives, including disintegrators, lubricants, colorants, flavors and the like. The proportion of the active compound to the carrier may be at in ratio of 1:1 to 1:100 by weight and may usually be chosen appropriately depending on the form of the orally administrable formulation prepared. Suppository formulations may contain excipients and, if necessary, surfactant and lubricants additionally to the active compound.

The optimum dosage of the new compound (I) administered will, of course, depend on the mode of administration and the treatment desired. For men, the unit dosage for injections generally contains from 50 mg to 500 mg of the compound (I), which may be administered intravenously or intramuscularly in divided doses one or more times per day. The new compound of the formula (I) used in the composition of this invention may be administered orally to an adult person at a dosage of 50 mg to 500 mg once a day.

This invention is now illustrated with reference to the following Examples. Referential Example 1 illustrates the preparation of a starting compound of the general formula (II) to be used in the process according to the third aspect of this invention. Examples 1 to 14 and 14 to 16 illustrate the process according to the third aspect of this invention, while Examples 5 to 13 illustrate the process according to the fourth aspect of this invention.

The respective compounds which were obtained as the final product in these Examples 1 to 16 are particular examples of the compound of the general formula (I) according to the first aspect of this invention.

Referential Example 1

(A) Preparation of 3',4',2",4",6"-penta-O-acetyl-1,3,2',6',3"-penta-N-tosylkanamycin B [Compound (1)]

1,3,2',6',3"-Penta-N-tosylkanamycin B (110 mg) was dissolved in dry pyridine (2.2 ml) to which acetyl chloride (0.062 ml) was added at 0°C, followed by effecting the reaction at 0°C. Two hours later, acetyl chloride (0.029 ml) was added further, followed by effecting further reaction for 3 hours (for the acetylation). Water (0.12 ml) was thereafter added to the reaction solution as obtained. The resultant mixture was concentrated and the residue was extracted with chloroform. The extract was washed with saturated aqueous solution of sodium bi-carbonate, washed with water, and then dried over anhydrous sodium sulfate. The resulting solution was concentrated to dryness under reduced pressure, thereby affording the titled Compound (1) (147 mg).

Specific rotation $[\alpha]_D^{23}$ : +25° (c 1.0, chloroform).

(B) Preparation of 3',4',2",4",6"-penta-O-acetyl-5-O-benzoyl-1,3,2',6',3"-penta-N-tosyl-5-epi-kanamycin B [Compound (2)]

Compound (1) (4.23 g) as obtained in step (A) above was dissolved in dry tetrahydrofuran (64 ml), followed by successive addition of triphenylphosphine (2,27g), diethyl azodicarboxylate (1.34 ml) and benzoic acid (1.06 g). The reaction was then conducted at room temperature. Two hours later, triphenylphosphine (2.27 g), diethyl azodicarboxylate (1.34 ml) and benzoic acid (1.06 g) were successively added again, and the reaction was further conducted overnight at room temperature (for effecting Mitsunobu's reaction). The reaction mixture was concentrated to dryness and the residue was taken up in chloroform. The chloroform solution was washed successively with a saturated aqueous solution of sodium bi-carbonate and water and then dried over anhydrous sodium sulfate. The resultant solution was concentrated under reduced pressure. The residue was purified by column chromatography on silica gel (325 g; the column was developed with chloroform, followed by gradient elution with a mixed eluent of chloroform and ethanol while changing their ratio from 20:0 to 20:1), thereby affording the titled Compound (2) (2.38 g).

Specific rotation $[\alpha]_D^{23}$ : +20° (c 1.0, chloroform).

$^1$H-NMR spectrum (in deuterochloroform in the presence of a drop of $D_2O$):

δ1.48 (1H, q, H-2ax),
δ1.54 (3H, s, Ac),
δ1.64 (3H, s, Ac),
δ1.84 (3H, s, Ac),
δ1.94 (3H, s, Ac),
δ1.97 (3H, s, Ac),
δ2.17 (1H, dt, H-2eq),
δ2.34 (3H, s, $CH_3$ of Ts),
δ2.38 (3H, s, $CH_3$ of Ts),
δ2.40 (3H, s, $CH_3$ of Ts),
δ2.47 (3H, s, $CH_3$ of Ts),
δ2.51 (3H, s, $CH_3$ of Ts),
δ3.78 (1H, dd, H-6),
δ6.01 (1H, bt, H-5).

(C) Preparation of 5-epi-1,3,2',6',3"-penta-N-tosyl-kanamycin B [Compound (3)]

Compound (2) (1.20 g) as obtained in the step (B) above was dissolved in a methanolic solution (24 ml) of sodium methoxide (0.2 mol/l), followed by effecting the reaction at 58°C for 37 hours (for the removal of the acetyl and benzoyl groups). After adding dilute hydrochloric acid to the reaction mixture and neutralizing same, the resultant mixture was concentrated to dryness. The residue was washed with a large volume of water and then dried to afford the titled Compound (3) (0.930 g).

(D) Preparation of 3',4',2",4",6"-penta-O-acetyl-1,3,2',6',3"-penta-N-tosyl-5-epi-kanamycin B [Compound (4)]

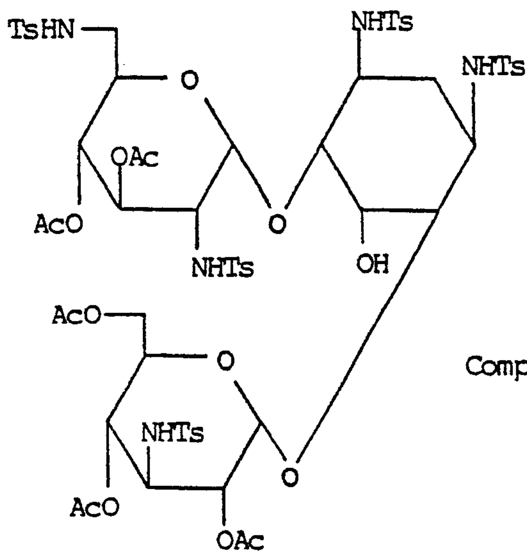

Compound (4)

wherein Ac means an acetyl group and Ts denotes a tosyl group, and Ac and Ts will hereinafter have the same meanings as above.

Compound (3) (898 mg) as obtained in step (C) above was dissolved in dry pyridine (18 ml), followed by addition of acetyl chloride (0.77 ml) at 0°C. The reaction was effected at 0°C for 1 hour (for the protection of the hydroxyl groups by acetylation). The reaction mixture was added with water (0.97 ml) and concentrated to dryness. The residue was taken up in chloroform. The resulting solution was washed successively with a 10% aqueous solution of potassium hydrogen sulfate, a saturated aqueous solution of sodium bi-carbonate and water, and was then dried over anhydrous sodium sulfate. The solution was concentrated to dryness, thereby affording the titled Compound (4) as a colorless solid (1086 mg).

Specific rotation $[\alpha]_D^{23}$ : +23° (c 1.0, chloroform).

$^1$H-NMR spectrum (in deuterochloroform):

δ1.60 (3H, s, Ac),
δ1.71 (3H, s, Ac),
δ1.93 (3H, s, Ac),
δ1.97 (3H, s, Ac),
δ2.02 (3H, s, Ac),
δ2.38 (6H, s, $CH_3$ × 2 of Ts),
δ2.40 (3H, s, $CH_3$ of Ts),
δ2.48 (3H, s, $CH_3$ of Ts),
δ2.49 (3H, s, $CH_3$ of Ts),

Example 1

(A) Preparation of 3',4',2",4",6"-penta-O-acetyl-1,3,2',6',3"-penta-N-tosyl-5-deoxy-5-fluorokanamycin B [Compound (5)]

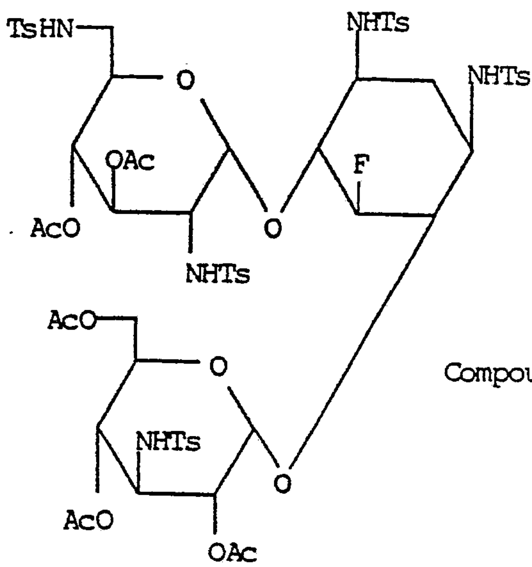

Compound (5)

Diethylaminosulfur trifluoride (DAST) (0.452 ml) was diluted with dry dichloromethane (17.4 ml) and dry pyridine (0.9 ml), to which was added at 0°C a solution in dry dichloromethane (26 ml) of Compound (4) (1085 mg), which had been obtained in the step (D) of Referential Example 1. The reaction was effected at room temperature for 1.5 hours (for the fluorination and inversion of the epi-5-OH group). The reaction mixture was poured into an ice-cooled saturated aqueous solution of sodium bi-carbonate (45 ml). After thoroughly stirring the resultant mixture, the dichloromethane layer was taken out, washed successively with a saturated aqueous solution of sodium bi-carbonate and water, and dried over anhydrous sodium sulfate. The resultant dried solution was concentrated to dryness, thereby affording the titled Compound (5) as a solid (1.10 g).

$^{19}$F-NMR spectrum (in deuterochloroform in the presence of a drop of $D_2O$; internal standard: trichlorofluoromethane):

-188.87 ppm (dt).

(B) Preparation of 5-deoxy-5-fluorokanamycin B [Compound No. 1 of this invention]

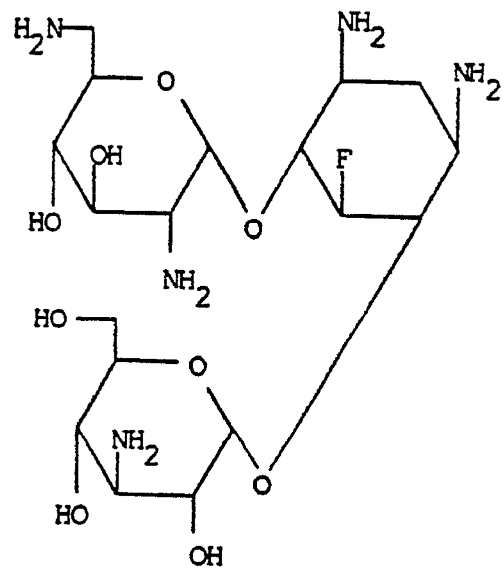

Compound (5) (1.1 g) as obtained in step (A) above was dissolved in liquefied ammonia (300 ml) at -60°C, to which metal sodium (about 1.1 g) was then added. The reaction was conducted at -50°C for 5 minutes (for the removal of the tosyl groups). Methanol (30 ml) was added to the reaction mixture, followed by evaporation of the ammonia. After concentration of the reaction mixture under reduced pressure, the residue was dissolved in water (110 ml) and then heated at 50°C for 30 minutes (for the removal of the acetyl groups). The resulting reaction mixture was cooled to room temperature, to which "Dowex 50W × 2 Resin" (trade name; 80 ml) was added to adsorb the resultant deprotected reaction product thereon. The deprotected reaction product which was adsorbed by the resin was placed together with the resin on the top of a column of 40 ml of the same resin. After washing the column with water, the column was eluted with 1N aqueous ammonia, and the ninhydrin-positive fractions of the eluate were collected. In a column of "CM-Sephadex C-25" (trade name; 85 ml), the reaction product thus obtained was further developed gradiently with aqueous ammonia while changing the concentration of ammonia from 0 N to 0.15 N. Fractions of the eluate containing the intended compound were collected and concentrated. The titled Compound was obtained as a colorless solid (98 mg).

Specific rotation $[\alpha]_D^{23}$ : +117° (c 0.6, water). $^1$H-NMR spectrum (in deutero-hydrochloric acid; internal standard: sodium 3-(trimethylsilyl)propionate-$d_4$):

δ2.08 (1H, q, H-2ax),
δ2.60 (1H, bdt, H-2eq),
δ3.31 (1H, dd, H-6'a),
δ4.24 (1H, q, H-6 or H-4),
δ4.49 (1H, q, H-4 or H-6),
δ4.99 (1H, dt, H-5),
δ5.16 (1H, d, H-1"),
δ5.78 (1H, d, H-1').

<u>Referential Example 2</u>

(A) Preparation of 1,3,2',6',3"-penta-N-tosyl-tobramycin [Compound (6)]

Tobramycin, namely, 3'-deoxykanamycin B (600 mg) was dissolved in water (40 ml), into which sodium carbonate (900 mg) was added and dissolved. Dioxane (40 ml) was added further, followed by still further addition of tosyl chloride (1469 mg) under ice-cooling and stirring. 1.5 Hours later, sodium carbonate (450 mg) and tosyl chloride (735 mg) were again added to the reaction mixture obtained. Three hours later, sodium carbonate (450 mg) and tosyl chloride (735 mg) were added further. The reaction was effected

overnight at room temperature (for the N-tosylation). The reaction mixture was concentrated to dryness, and the residue was washed with a large volume of water and then dried. The residue was thereafter dissolved in chloroform-ethanol, followed by reprecipitation with ethyl ether. A precipitate as deposited was washed with ethyl ether to afford the titled Compound (6) (1721 mg).

Specific rotation $[\alpha]_D^{23}$ : +24° (c 1.0, pyridine).

(B) Preparation of 4",6"-O-cyclohexylidene-1,3,2',6',3"-penta-N-tosyltobramycin [Compound (7)]

Compound (6) (1.72 g) as obtained in step (A) above was dissolved in dry dimethylformamide (35 ml), followed by addition of p-toluenesulfonic acid (60 mg) and 1,1-dimethoxycyclohexane (1.25 ml). Under reduced pressure of 30 mmHg, the reaction was conducted at 50°C under stirring to achieve the 4",6"-O-cyclohexylidenation. The reaction mixture was cooled and poured into a saturated aqueous solution of sodium bi-carbonate (25 ml). The resulting mixture was concentrated to dryness. The residue was washed with a large volume of water and then dried. The residue was washed with ethyl ether to afford the titled Compound (7) (1.76 g).

Specific rotation $[\alpha]_D^{23}$ : +3° (c 1, pyridine).

(C) Preparation of 4',2"-di-O-acetyl-4",6"-0-cyclohexylidene-1,3,2',6',3"-penta-N-tosyltobramycin [Compound (8)]

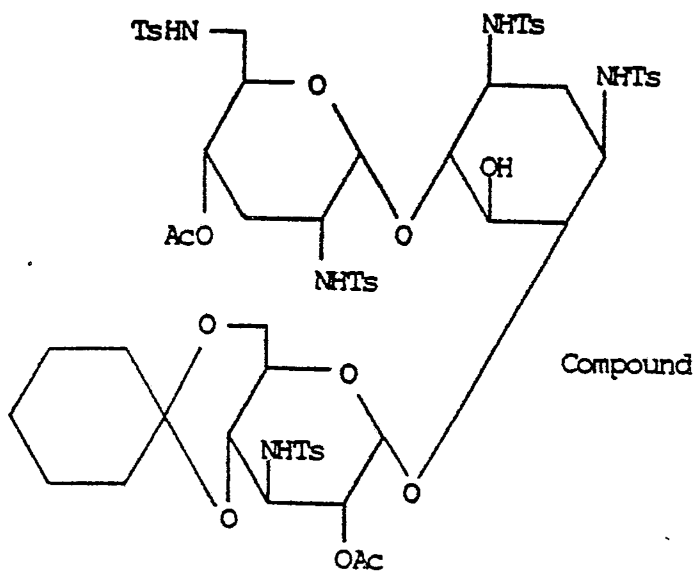

Compound (8)

Compound (7) (1059 mg) as obtained in the step (B) above was dissolved in dry dimethylsulfoxide (4.8 ml), followed by addition of dry pyridine (0.53 ml) and N-acetylimidazole (356 mg). The reaction was made at 37°C. upon elapsed time of 12 hours, 24 hours, 36 hours, 48 hours, 60 hours and 72 hours, 360 mg portions of N-acetylimidazole were added (for the protection of the hydroxyl groups of acetylation). 84 Hours later, water (2 ml) was added to the reaction mixture, and the resultant mixture was poured into a saturated aqueous solution of sodium bi-carbonate (106 ml.). A precipitate as deposited was collected by filtration, washed with water, dried and then washed with ethyl ether, thereby to afford the titled Compound (8) (1084 mg).

Specific rotation $[\alpha]_D^{23}$ : +18° (c 1.0, chloroform).

(D) Preparation of 4′,2″-di-O-acetyl-5-O-benzoyl-4″,6″-O-cyclohexylidene-1,3,2′,6′,3″-penta-N-tosyl-5-epi-tobramycin [Compound (9)]

Compound (8) (448 mg) as obtained in the step (C) above was dissolved in dry tetrahydrofuran (6.8 ml), followed by successive addition of triphenylphosphine (253 mg), diethyl azodicarboxylate (0.15 ml) and benzoic acid (119 mg). The reaction was effected at room temperature Two hours later, triphenylphosphine (253 mg), diethyl azodicarboxylate (0.15 ml) and benzoic acid (119 mg) were successively added again. The reaction was then effected overnight at room temperature (for Mitsunobu's reaction). The resulting reaction solution was concentrated to dryness and the residue was dissolved in chloroform. The solution was washed successively with a saturated aqueous solution of sodium bi-carbonate and water and was then dried over anhydrous sodium sulfate. The solution was concentrated under reduced pressure. The residue was purified by chromatography on silica gel ["Merck Silica Gel 60" (trade name), 230-400 mesh, 30g]; in such a way that the column was developed with chloroform, followed by gradient elution with a mixed eluent of chloroform and ethanol while changing their ratio from 20:0 to 20:1, thereby affording the titled Compound (9) (316 mg).

Specific rotation $[\alpha]_D^{23}$ : -8° (c 1.0, chloroform).

$^1$H-NMR spectrum (in deuteropyridine-$D_2O$ (20:1) at 80°C):

δ1.78 (3H, s),
δ2.20 (6H, s),
δ2.28 (3H, s),
δ2.33 (3H, s),
δ2.36 (3H, s),

Each of the above peaks corresponds to $CH_3$ of Ac or Ts.

δ6.24 (1H, bt, H-5).

(E) Preparation of 4″,6″-O-cyclohexylidene-1,3,2′,6′,3″-penta-N-tosyl-5-epi-tobramycin [Compound (10)]

Compound (9) (473 mg) as obtained in the step (D) above was dissolved in a methanolic solution (9.45 ml) of sodium methoxide (0.2 mol/l) to effect the reaction at 58°C for 27 hours (for the removal of acetyl and benzoyl groups). Dilute hydrochloric acid was added to the reaction mixture to neutralize same. The resultant mixture was concentrated to dryness, and the residue was washed with a large volume of water to obtain the titled Compound (10) (418 mg).

(F) Preparation of 4′,2″-di-O-acetyl-4″,6″-O-cyclohexylidene-1,3,2′,6′,3″-penta-N-tosyl-5-epi-tobramycin [Compound (11)]

Compound (10) (3.97 g) as obtained in the step (E) above was dissolved in a mixture of dry dimethylsulfoxide (17.9 ml) and dry pyridine (2.0 ml), followed by addition of N-acetylimidazole (1.376 g). The reaction was effected at 37°C. Six hours later, N-acetylimidazole (1.391 g) was added, followed by reaction overnight at 37°C (for the acetylation). After adding water (2.26 ml) to the reaction mixture, the resulting mixture was poured into a saturated aqueous solution of sodium hydrogen carbonate (400 ml). A precipitate deposited was collected by filtration, washed with water and washed with dry ethyl ether, thereby to afford the titled Compound (11) as a colorless solid (3.853 g). This compound is a novel compound.

Specific rotation $[\alpha]_D^{22}$ : +19° (c 1.0, chloroform).

$^1$H-NMR spectrum (in deuteropyridine):

δ5.05 (1H, bs, H-5),
δ5.46 (1H, dd, H-2″).

Example 2

(A) Preparation of 4',2"-di-O-acetyl-4",6"-O-cyclohexylidene-1,3,2',6',3"-penta-N-tosyl-5-deoxy-5-fluorotobramycin [Compound (12)]

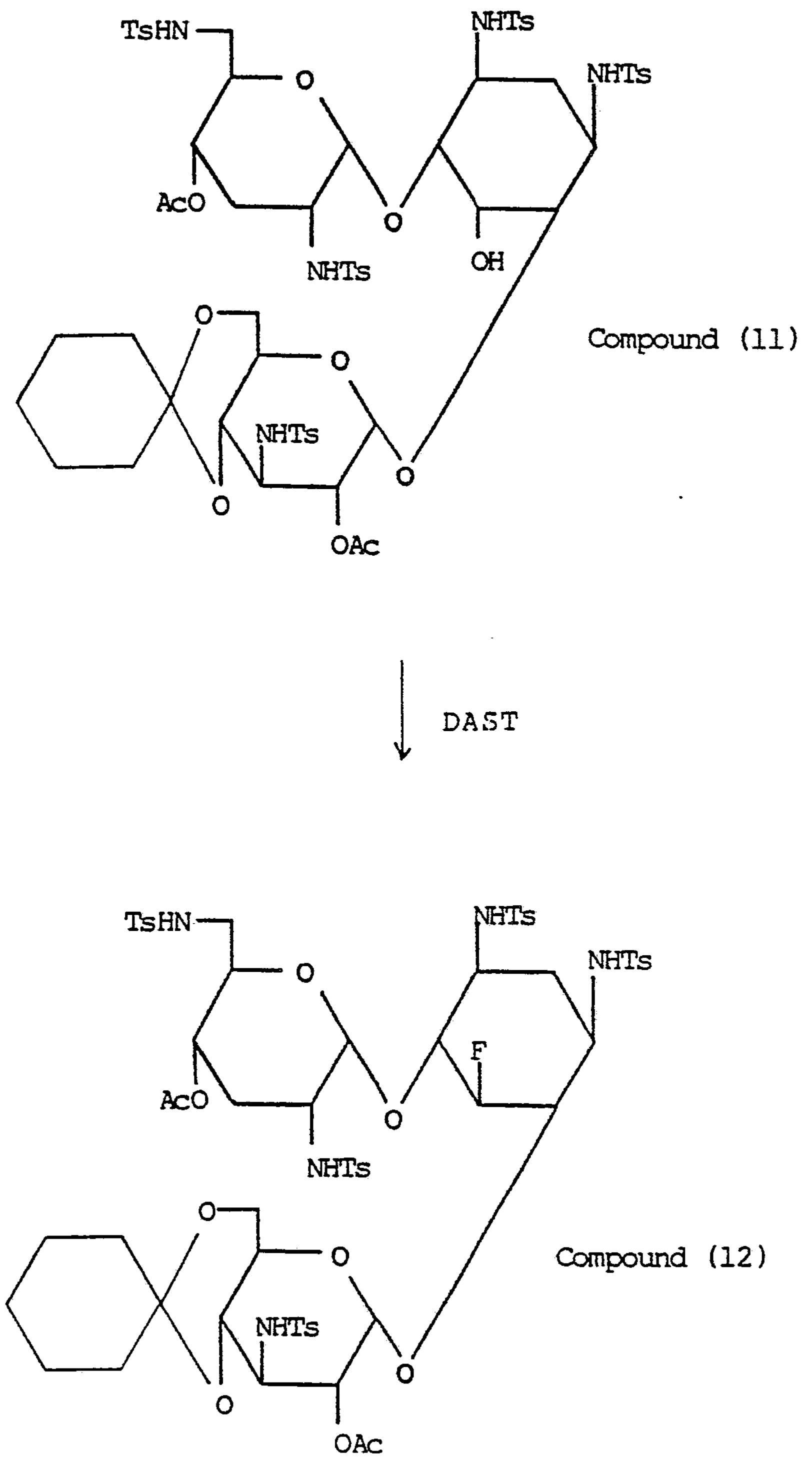


Compound (11)

Compound (12)

Diethylaminosulfur trifluoride (DAST) (0.144 ml) was mixed with dry benzene (5.0 ml) and dry pyridine (0.29 ml), into which was then added at 0°C a solution of Compound (11) (331 mg) in dry benzene (8.23 ml). The reaction was carried out at room temperature for 2 hours (for the fluorination and inversion of the epi-5-OH group). The reaction mixture obtained was poured into an ice-cooled saturated aqueous solution of

sodium bicarbonate (14 ml), followed by extraction with chloroform (30 ml). The resultant chloroform solution was washed successively with a saturated aqueous solution of sodium bicarbonate and water, and was then dried over anhydrous sodium sulfate. The solution was concentrated to dryness under reduced pressure, thereby to afford the titled Compound (12) as a solid (363 mg).

Specific rotation $[\alpha]_D^{22}$ : +21° (c 1.0, chloroform).

$^{19}$F-NMR spectrum (in deutero-pyridine-$D_2O$ (20:1); internal standard: trichlorofluoroethane): -187.40 ppm.

(B) Preparation of 5-deoxy-5-fluorotobramycin, namely, 5,3'-dideoxy-5-fluorokanamycin B [Compound No. 2 of this invention]

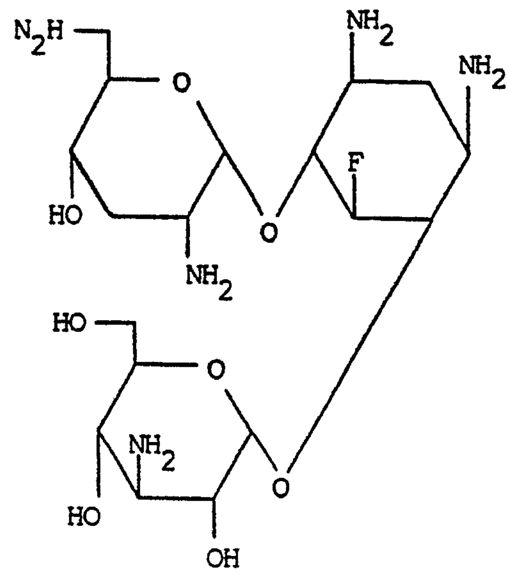

Compound (12) (393 mg) as obtained in the step (A) above was dissolved in liquefied ammonia (80 ml) at -60°C, to which metal sodium (about 400 mg) was added. The reaction was effected at -50°C for 5 minutes (for the removal of tosyl groups). Methanol (10 ml) was added to the reaction mixture, followed by evaporation of ammonia. After concentration of the reaction mixture under reduced pressure, the residue was dissolved in water (40 ml) and then heated at 50°C for 30 minutes (for the removal of acetyl groups). The resulting reaction mixture was cooled to room temperature, to which "Dowex 50W × 2" resin (trade name; 60 ml) was added to adsorb the reaction product thereon (the removal of the cyclohexylidene group took place then). The resultant deprotected reaction product as adsorbed by the resin was placed on the top of a column of 20 ml of the same resin. After washing the column with water, the column was eluted with 1N aqueous ammonia, and the ninhydrin-positive fractions of the eluate were collected. In a column of "CM-Sephadex C-25" (trade name; 26 ml), the reaction product obtained was further developed gradiently with aqueous ammonia while changing the concentration of ammonia from 0 N to 0.15 N. Fractions containing the intended compound were collected and concentrated. The titled Compound (Compound No. 2 of this invention) was obtained as a colorless solid (55.6 mg).

Specific rotation $[\alpha]_D^{23}$ : +124° (c 1.0, water).

$^1$H-NMR spectrum (in 20% aqueous deutero-ammonia; internal standard; sodium 3-(trimethylsilyl)-propionate-$d_4$):

δ1.28 (1H, q, H-2ax),
δ1.60 (6H, q, H-3'ax),
δ3.30 (1H, t, H-4"),
δ4.57 (1H, dt, H-5),
δ5.01 (1H, d, H-1"),
δ5.09 (1H, d, H-1').

Referential Example 3

(A) Preparation of 1,3,2',6',3"-penta-N-tosyl-4'-deoxykanamycin B [Compound (13)]

4'-Deoxykanamycin B (750 mg) was dissolved in water (50 ml), in which sodium carbonate (1.13 g) was then added and dissolved. Dioxane (50 ml) was added, followed by further addition of tosyl chloride (1.84 g) under ice-cooling and stirring. Two hours later, sodium carbonate (560 mg) and tosyl chloride (919 mg) were added to the reaction mixture, followed by reaction overnight at room temperature (for the N-tosylation). The reaction mixture so obtained was concentrated to dryness, and the residue was washed with a large volume of water and then dried. The residue was thereafter dissolved in chloroform-methanol, followed by reprecipitation with ethyl ether. A precipitate as formed was washed with ethyl ether to afford the titled Compound (13) (2.1 g).

(B) Preparation of 3',2",4",6"-tetra-O-acetyl-1,3,2',6',3"-penta-N-tosyl-4'-deoxykanamycin B [Compound (14)]

Compound (13) (492 mg) as obtained in the step (A) above was dissolved in dry pyridine (9.8 ml), followed by addition of acetyl chloride (0.29 ml). The reaction was made at 0°C. Two hours later, acetyl chloride (0.146 ml) was added further, followed by effecting the reaction for 3 hours (for the acetylation). Thereafter, water (0.56 ml) was added to the reaction mixture. The resultant mixture was concentrated and the residue was dissolved in chloroform. The resultant solution was washed with a saturated aqueous solution of sodium bicarbonate, washed with water, and then dried over anhydrous sodium sulfate. The solution thus obtained was concentrated to dryness under reduced pressure, thereby affording the titled Compound (14) (587 mg).

(C) Preparation of 3',2",4",6"-tetra-O-acetyl-5-O-benzoyl-5-epi-1,3,2',6',3"-penta-N-tosyl-4'-deoxykanamycin B [Compound (15)]

Compound (14) (260 mg) as obtained in the step (B) above was dissolved in dry tetrahydrofuran (3.9 ml), followed by successive addition of triphenylphosphine (146 mg), diethyl azodicarboxylate (0.086 ml) and benzoic acid (68 mg). The reaction was effected at room temperature. 1.5 Hour later, triphenylphospine (147 mg), diethyl azodicarboxylate (0.086 mg) and benzoic acid (68 mg) were successively added again, followed by reaction overnight at room temperature (for Mitsunobu's reaction). The reaction mixture was concentrated to dryness and the residue was dissolved in chloroform. The resulting solution was washed successively with a saturated aqueous solution of sodium bicarbonate and water, and was then dried over anhydrous sodium sulfate. The resultant solution was concentrated under reduced pressure, and the residue was purified by column chromatography on silica gel (20 g; the column was developed with chloroform, followed by gradient elution with a mixed eluent of chloroform and ethanol while changing their ratio from 20:0 to 20:1), thereby affording the titled Compound (15) (161 mg).

(D) Preparation of 1,3,2',6',3"-penta-N-tosyl-4'-deoxy-5-epi-kanamycin B [Compound (16)]

Compound (15) (150 mg) as obtained in the step (C) above was dissolved in a methanolic solution (3 ml) of sodium methoxide (0.2 mol/l), followed by effecting the reaction at 58°C for 32 hours (for the removal of acetyl and benzoyl groups). After adding dilute hydrochloric acid to the reaction mixture to neutralize same, the resultant mixture was concentrated to dryness. The residue was washed with a large volume of water and then dried to afford the titled Compound (16) (114 mg).

(E) Preparation of 3′,2″,4″,6″-tetra-O-acetyl-1,3,2′,6′,3″-penta-N-tosyl-4′-deoxy-5-epi-kanamycin B [Compound (17)]

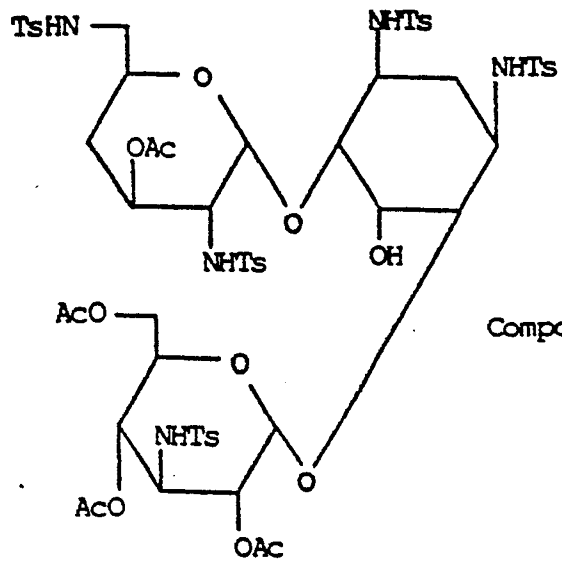

Compound (17)

Compound (16) (350 mg) as obtained in the step (D) above was dissolved in dry pyridine (7 ml), to which acetyl chloride (0.21 ml) was added at 0°C. The reaction was carried out at 0°C for 1 hour (for the acetylation). The reaction mixture was added with water (0.26 ml) and then concentrated to dryness. The residue was taken up in chloroform and the resultant solution was washed successively with a 10% aqueous solution of potassium hydrogen sulfate, a saturated aqueous solution of sodium bicarbonate and water, followed by drying over anhydrous sodium sulfate. The solution obtained was concentrated to dryness, thereby affording the titled Compound (17) as a colorless solid (415 mg).

Example 3

(A) Preparation of 3′,2″,4″,6″-tetra-O-acetyl-1,3,2′,6,3″-penta-N-tosyl-5,4′-dideoxy-5-fluorokanamycin B [Compound (18)]

Diethylaminosulfur trifluoride (DAST) (0.17 ml) was mixed with dry dichloromethane (7.4 ml) and dry pyridine (0.34 ml), to which was added at 0°C a solution in dry dichloromethane (8.4 ml) of Compound (17) (395 mg), which had been obtained in the step (E) of Referential Example 3. The reaction was effected at room temperature for 1.5 hours (for the fluorination and inversion of the epi-5-OH group). The reaction mixture was poured into an ice-cooled saturated aqueous solution of sodium hydrogen carbonate (16 ml). After thoroughly stirring the resultant mixture, the dichloromethane layer was taken out, washed successively with a saturated aqueous solution of sodium bicarbonate and water, and dried over anhydrous sodium sulfate. The resultant solution was concentrated to dryness, thereby affording the titled Compound (18) as a solid (392 mg).

(B) Preparation of 5,4′-dideoxy-fluorokanamycin B [Compound No. 3 of this invention]

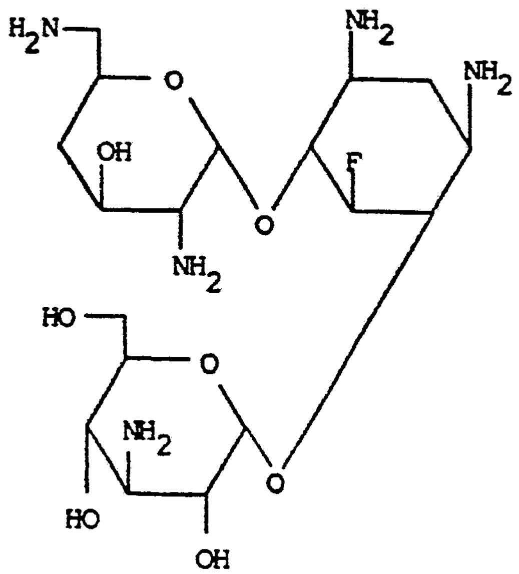

Compound (18) (302 mg) as obtained in the step (A) above was dissolved in liquefied ammonia (90 ml) at -60°C, to which metal sodium (about 300 mg) was added. The reaction was effected at -50°C for 5 minutes (for the removal of tosyl groups). Methanol (10 ml) was added to the reaction mixture, followed by evaporation of ammonia at room temperature. After concentration of the reaction mixture under reduced pressure, the residue was dissolved in water (30 ml) and then heated at 50°C for 30 minutes (for the removal of acetyl groups). The reaction mixture so obtained was cooled to room temperature, to which "Dowex 50W × 2" resin (trade name; 20 ml) was added to adsorb the resultant deprotected reaction product thereon. The deprotected reaction product as adsorbed by the resin was placed together with the resin, on the top of a column of the same resin (10 ml). After washing the column with water, the column was eluted with 1N aqueous ammonia, and the ninhydrin-positive fractions of the eluate were collected and concentrated. The residue was dissolved in water (30 ml) and the resultant solution was charged in a column of "CM-Sephadex C-25" (trade name; 24 ml). The column was gradiently eluted with aqueous ammonia while changing the concentration of ammonia from 0 N to 0.15 N. Fractions of the eluate containing the intended compound were collected and concentrated, thereby affording the titled Compound (Compound No. 3 of this invention) as a colorless solid (41 mg).

Specific rotation $[\alpha]_D^{23}$ : +120° (c 1.0, water).

Referential Example 4

(A) Preparation of 1,3,2′,6′,3″-penta-N-tosyl-3′,4′-dideoxykanamycin B [Compound (19)]

In water (35 ml) was dissolved 3′,4′-dideoxykanamycin B (namely dibekacin) sulfate (3.50 g; potency: 690 μg/mg), followed by addition of sodium carbonate (3.40 g; 6 mole equivalents). Thereafter, 1,4-dioxane (70 ml) was added, and p-toluenesulfonyl chloride (6.12 g; 6 mole equivalents based on the dibekacin) was added further under ice-cooling and stirring. One hour later, the reaction mixture was heated to room temperature, followed by further stirring. 72 Hours later, the reaction mixture was concentrated under reduced pressure, followed by addition of water (300 ml). The resulting precipitate was collected by filtration, washed with water and then dried under reduced pressure. The precipitate was washed further with ethyl ether and dried under reduced pressure, thereby affording the titled Compound (19) as a solid (6.06 g). Yield: 93%.

(B) Preparation of 4",6"-O-cyclohexylidene-1,3,2',6",3"-penta-N-tosyldibekacin [Compound (20)]

Compound (19) (1.63 g) as obtained in the step (A) above was dissolved in dry DMF (8.1 ml), to which were added cyclohexanone dimethylacetal (1 ml) and p-toluenesulfonic acid (51 mg). The mixture obtained was stirred at 50°C under reduced pressure of 30 mmHg. One hour later, the resulting reaction solution was poured into an ice-cooled saturated aqueous solution of sodium bicarbonate (200 ml). A white precipitate thus formed was collected by filtration, washed with water and then dried under reduced pressure, thereby affording the titled Compound (20) as a white solid (1.75 g). The yield was stoichiometric.

Specific rotation $[\alpha]_D^{24}$ : +28° (c 1.0, DMF).

(C) Preparation of 2"-O-benzoyl-4",6"-O-cyclohexylidene-1,3,2',6',3"-penta-N-tosyldibekacin [Compound (21)]

Compound (20) (1.75 g) as obtained in the step (B) above was dissolved in dry pyridine (35 ml), to which benzoyl chloride (0.78 ml) was added at 0°C under stirring so as to conduct a reaction. Water (0.6 ml) was added 3.5 hours later. After allowing the reaction mixture to stand for 1 hour, it was concentrated under reduced pressure. The residue was extracted with chloroform. The resulting chloroform solution was washed successively with a saturated aqueous solution of sodium bicarbonate (100 ml × 3), a 5% aqueous solution of potassium hydrogen sulfate (100 ml × 2) and water (100 ml × 3), dried over anhydrous sodium sulfate and then concentrated to dryness to obtain the titled Compound (21) as a solid (1.885 g). Yield: 99.5%.

Specific rotation $[\alpha]_D^{22}$ : +32° (c 1, $CHCl_3$).

(D) Preparation of 5,2"-di-O-benzoyl-4",6"-O-cyclohexylidene-1,3,2',6',3"-penta-N-toxyl-5-epi-dibekacin [Compound (22)]

Compound (21) (1.62 g) as obtained in the step (C) above was dissolved in dry tetrahydrofuran (24.3 ml), followed by addition of diethyl azodicarboxylate (DEAD) (0.54 ml), triphenylphosphine $[P(C_6H_5)_3]$ (906 mg) and benzoic acid (422 mg). The reaction was effected at 50°C. 1.5 Hour later, the same reagents were added respectively in the same amounts. The resulting reaction mixture was cooled down to room temperature and was allowed to undergo the reaction. Three hours later, the same reagents were again added respectively in the same amounts as above. The reaction mixture was heated to 50°C, and 5 hours later, it was cooled to room temperature, at which the reaction was carried out overnight. 21 Hours later, the same reagents were added further respectively in the same amounts, followed by effecting the reaction at room temperature (for Mitsunobu's reaction). 92 hours later, the reaction mixture was concentrated under reduced pressure, the resulting syrup was dissolved in chloroform (300 ml), washed successively with a saturated aqueous solution of sodium bicarbonate (200 ml × 2) and water (200 ml × 3), dried over anhydrous sodium sulfate, and then concentrated to dryness. A solid obtained (9.68 g) was subjected to column chromatography on silica gel ("Wako-gel C-200", trade name; as developed with a mixed solvent of toluene and acetone) to repeat removal of the remaining reagents and separation and purification of the reaction product, thereby affording the titled Compound (22) as a yellow solid (0.95 g). Yield: 54.5%.

(E) Preparation of 4″,6″-O-cyclohexylidene-1,3,2′,6′,3″-penta-N-tosyl-5-epi-dibekacin [Compound (23)]

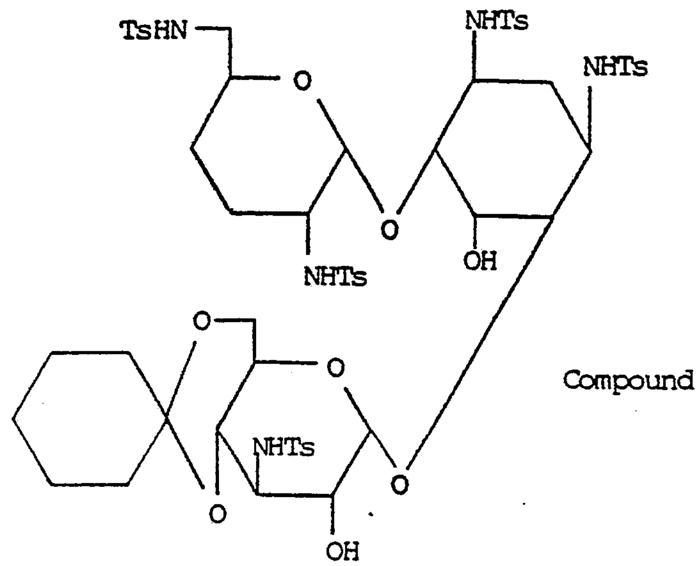

Compound (23)

Compound (22) (0.95 g) as obtained in the step (D) above was mixed with methanol (19 ml) (with a gellation occurring), to which a 28% methanolic solution of sodium methoxide (0.7 ml) was then added to conduct the reaction at 50°C, when a homogeneous solution was formed. After conducting this debenzoylation reaction for 44 hours, the reaction mixture was concentrated under reduced pressure, and a 0.25% aqueous solution of potassium hydrogen sulfate (20 ml) was added to the residue. A white precipitate obtained was collected by filtration, washed with water and dried under reduced pressure, thereby affording the titled Compound (23) as a white solid (0.83 g). The yield was stoichiometric.

Specific rotation $[\alpha]_D^{23}$ : +6° [c 0.5, acetonemethanol (1:1)]

(F) Preparation of 2″-O-benzoyl-4″,6″-O-cyclohexylidene-1,3,2′,6′,3″-penta-N-tosyl-5-epi-dibekacin [Compound (24)]

Compound (23) (0.77 g) as obtained in the step (E) above was dissolved in dry pyridine (15.4 ml). At 0°C and under stirring, benzoyl chloride [0.34 ml, 5 mole equivalents based on Compound (23)] was added to the solution to conduct a reaction (for the 2″-O-benzoylation). Water (0.26 ml) was added 30 minutes later. One hour later, the reaction mixture was concentrated under reduced pressure. The residue was dissolved in chloroform (100 ml), washed successively with a saturated aqueous solution of sodium bicarbonate (50 ml × 2), a 5% aqueous solution of potassium hydrogen sulfate (50 ml × 3) and water (50 ml × 2), dried over anhydrous sodium sulfate and then concentrated to dryness, so that the titled Compound (24) was obtained as a solid (0.84 g). The yield was stoichiometric.

Specific rotation $[\alpha]_D^{23}$ : +29° (c 1, $CHCl_3$).

Example 4

(A) Preparation of 2″-O-benzoyl-4″,6″-O-cyclohexylidene-1,3,2′,6′,3″-penta-N-tosyl-5-deoxy-5-fluorodibekacin [Compound (25)]

Dry pyridine (0.66 ml) was added to dry benzene (15.4 ml), followed by addition of diethylaminosulfur trifluoride (DAST) [0.33 ml, 5 mole equivalents based on Compound (24)] at 0°C under stirring. To the resulting mixture was added Compound (24) (0.77 g) as obtained in the step (F) of Referential Example 4, and then the reaction was effected at room temperature under nitrogen atmosphere. One hour later, the reaction mixture was ice-cooled and then poured into a saturated aqueous solution of sodium bicarbonate (26 ml). The resultant mixture was extracted with chloroform (80 ml). The organic liquid layer obtained was

washed successively with a saturated aqueous solution of sodium bicarbonate (40 ml × 1), a 5% aqueous solution of potassium hydrogen sulfate (25 ml × 3), a saturated aqueous solution of sodium bicarbonate (25 ml × 2) and water (25 ml × 3), dried over anhydrous sodium sulfate, and then concentrated to dryness, to afford the titled Compound (25) as a solid (0.79 g). The yield was stoichiometric.

Specific rotation $[\alpha]_D^{23}$ : +37° (c 1, $CHCl_3$).

(B) Preparation of 5-deoxy-5-fluorodibekacin, namely, 5,3',4'-trideoxy-5-fluorokanamycin B [Compound No. 4 of this invention]

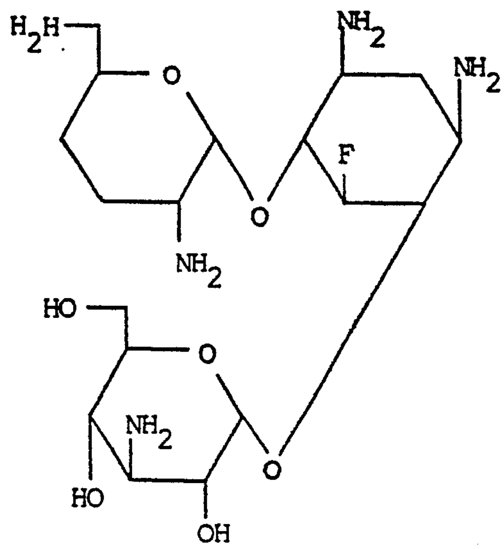

Compound (25) (0.74 g) as obtained in the step (A) above was dissolved in methanol (14.8 ml), to which a 28% methanolic solution of sodium methoxide (0.55 ml) was added, followed by effecting the reaction at room temperature (for the de-benzoylation). Two hours later, the reaction mixture was neutralized by mixing with a resin, "Dowex 50W × 2" (trade name, $H^+$-form, 4 ml) which had been saturated with methanol. The mixture was filtered to remove the resin, which was then washed with methanol. The filtrate and washing were combined together and then concentrated to dryness, thereby affording 0.59 g of a pale yellow solid (yield: 87%).

The solid reaction product so de-benzoylated (0.59 g) was added with 80% acetic acid (12 ml), followed by effecting the reaction at 80°C (for the de-cyclohexylidenization). 30 Minutes later, the reaction mixture was cooled down to room temperature and concentrated to dryness, thereby obtaining 0.52 g of a pale yellow solid (yield: 93%).

The solid, de-cyclohexylidenized product (0.52 g) was dissolved in liquefied ammonia (about 100 ml) at -50°C. Metal sodium (about 500 mg) was added thereto with vigorous stirring to conduct the de-tosylation reaction. 5 Minutes later, methanol was added until the blue color of the mixture disappeared. The reaction mixture was heated to room temperature and ammonia was allowed to evaporate off. The residue was dried under reduced pressure to obtain a solid comprising the de-tosylated product. The solid was then dissolved in water (23 ml), followed by neutralization with "Dowex 50W × 2" (trade name, $H^+$-form, 25 ml). The resin containing the de-tosylated product adsorbed therein was charged on the top of a column of 10 ml of the same resin. After washing the column with water, the column was eluted with 1N aqueous ammonia and the ninhydrin-positive fractions of the eluate were combined together and concentrated to dryness. The resulting solid (0.18 g) was dissolved in water (180 ml) and the resulting solution was charged into a column of "CM-Sephadex C-25" (trade name, $NH_4^+$-form, 90 ml). After washing the Sephadex column with water, the column was eluted gradiently with aqueous ammonia while changing the concentration of ammonia from 0.05 N to 0.2 N. The active fractions of the eluate were combined and concentrated to dryness, to give 0.10 g of the titled Compound No. 4 of this invention as a white solid (yield: 46% as monocarbonate-monohydrate).

Specific rotation $[\alpha]_D^{24}$ : +122° (c 1, water).

$^1$H-NMR (250 MHz, 20%$ND_3$-$D_2O$, standard: TMS):
δ4.55 (dt, H-5, $J_{5,F}$=51.5 Hz, $J_{5,4}$= 8.5 or 9.5 Hz, $J_{5,6}$=9.5 or 8.5 Hz)
δ5.01 (d, H-1", $J_{1'',2''}$=3.8Hz),
δ5.09 (d, H-1', $J_{1',2'}$=3.5Hz).

Example 5

(A) Preparation of 3,2',6'-tris(N-benzyloxycarbonyl)-5-deoxy-5-fluorokanamycin B [Compound (26)]

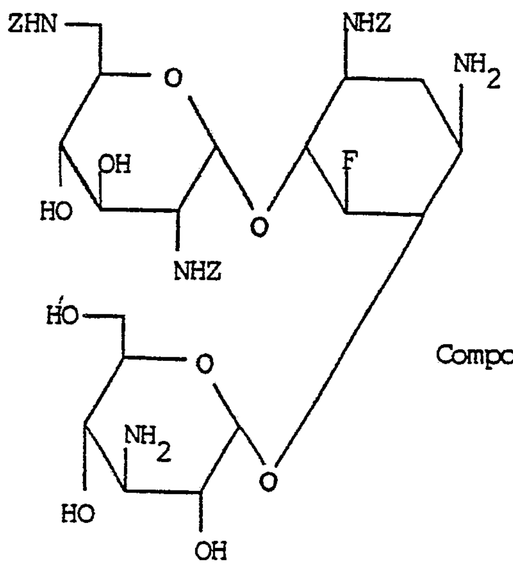

Compound (26)

wherein Z means a benzyloxycarbonyl group; Z will hereinafter have the same meaning as above.

In dry dimethylsulfoxide (0.3 ml) was suspended the carbonate (30 mg) of 5-deoxy-5-fluorokanamycin B (Compound No. 1 of this invention as obtained in Example 1), followed by addition of zinc acetate dihydrate (61.4 mg). The resultant mixture was stirred at 80°C for 1 hour. The resulting homogeneous solution, which contained the complex of 5-deoxy-5-fluorokanamycin B and zinc cation as formed, was cooled to room temperature, followed by gradual addition of N-(benzyloxycarbonyloxy)succinimide (47.4 mg) to said solution. The complex was subjected to the N-benzyloxycarbonylation reaction for 1 hour at room temperature. Ethyl ether was added to the reaction mixture, and a precipitate as deposited was repeatedly washed with ethyl ether. The solid obtained was repeatedly washed with 3N aqueous ammonia to remove the zinc cation from the N-benzyloxycarbonylated complex. The resulting product was washed with water and then dried to give 38 mg of the titled Compound (26).

(B) Preparation of 3,2',6'-tris(N-benzyloxycarbonyl)-5-deoxy-5-fluoro-3"-N-trifluoroacetylkanamycin B [Compound (27)]

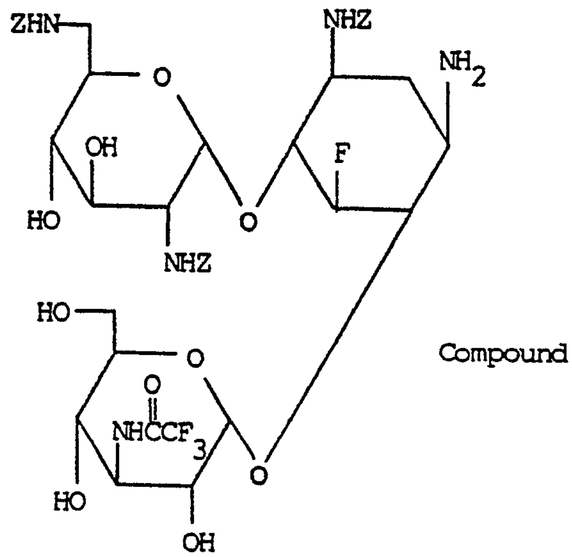

Compound (27)

Compound (26) (37 mg) as obtained in the step (A) above was dissolved in dry dimethylsulfoxide (0.19 ml), followed by addition of ethyl trifluoroacetate (0.0064 ml). The reaction was effected at room temperature for 1 hour (for the 3"-N-trifluoroacetylation). Ethyl ether was added to the reaction mixture. The precipitate as obtained was washed repeatedly with ethyl ether and dried, thereby affording the titled Compound (27) as a solid (42 mg).

(C) Preparation of 3,2',6'-tris(N-benzyloxycarbonyl)-1-N-[3-benzyloxycarbonylamino-2-(S)-hydroxypropionyl]-5-deoxy-5-fluoro-3"-N-trifluoroacetylkanamycin B [Compound (28)]

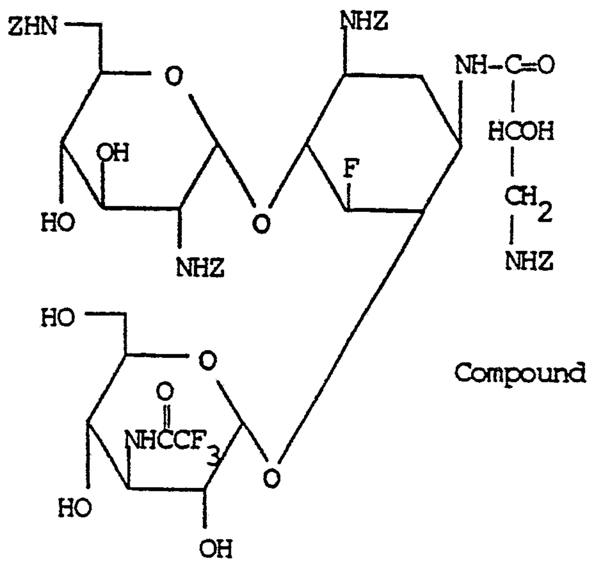

Compound (28)

Compound (27) (46 mg) as obtained in the step (B) above was dissolved in a mixed solvent (1.4 ml) of tetrahydrofuran and water (1:1). The resulting solution was mixed with sodium carbonate (4.0 mg) and then with a solution of the N-hydroxysuccinimide ester (19 mg) of 3-benzyloxycarbonylamino-2-(S)-hydroxypropionic acid in tetrahydrofuran (0.68 ml). The reaction was conducted at room temperature for 1 hour (for the 1-N-acylation). Thereafter, the reaction mixture was concentrated and the residue was washed with water. After drying, the residue was washed with ethyl ether to afford the titled Compound (28) as a solid (50.2 mg).

(D) Preparation of 1-N-[3-amino-2-(S)-hydroxy propionyl]-5-deoxy-5-fluorokanamycin B [Compound No. 5 of this invention]

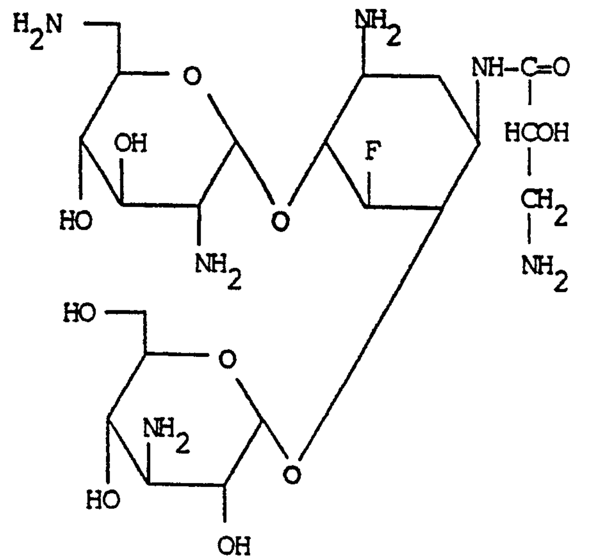

Compound (28) ( 50 mg) as obtained in the step (C) above was dissolved in a (4:3) mixed solvent (3.5 ml) of 2N aqueous ammonia and tetrahydrofuran, followed by effecting the reaction overnight at 28°C to remove the 3"-N-trifluoroacetyl group from Compound (28). Thereafter, the reaction mixture was concentrated and the resulting solid was dissolved in a mixed solvent (2.5 ml) of dioxane, water and acetic acid (4:1:1). The resulting solution was subjected to catalytic reduction with hydrogen gas at room temperature for 1 hour in the presence of palladium black as a catalyst. The reaction mixture was filtered and the filtrate was concentrated to give a solid. The solid was dissolved in water and was developed gradiently in a column of "CM-Sephadex C-25" (trade name) with aqueous ammonia while changing the concentration of ammonia from 0 N to 0.5 N. Fractions of the eluate containing the intended compound were collected and concentrated to afford the titled Compound No. 5 of this invention (17.4 mg).

Specific rotation $[\alpha]_D^{23}$ : +83° (c 1, water).

<u>Example 6</u>

(A) Preparation of 3,2',6'-tris(N-benzyloxycarbonyl)-1-N-[4-benzyloxycarbonylamino-2-(S)-hydroxybutyryl]-5-deoxy-5-fluoro-3"-N-trifluoroacetylkanamycin B [Compound (29)]

Compound (27) (42 mg) as obtained in the step (B) of Example 5 above was dissolved in a mixed solvent (1.25 ml) of tetrahydrofuran and water (1:1). The resulting solution was added with sodium carbonate (3.6 mg) and then with a solution of the N-hydroxysuccinimide ester (16.3 mg) of 4-benzyloxycarbonylamino-2-(S)-hydroxybutyric acid in tetrahydrofuran (0.624 ml). The reaction was effected at room temperature for 2 hours, followed by further addition of a solution of the above active ester (6 mg) in tetrahydrofuran (0.23 ml). The reaction was again effected for 3 hours at room temperature (for the 1-N-acylation). Thereafter, the reaction mixture was concentrated and the residue was washed with water. After drying, the residue was washed with ethyl ether and then dried to afford the titled Compound (29) (46.3 mg).

(B) Preparation of 1-N-[4-amino-2-(S)-hydroxybutyryl]-5-deoxy-5-fluorokanamycin B [Compound No. 6 of this invention]

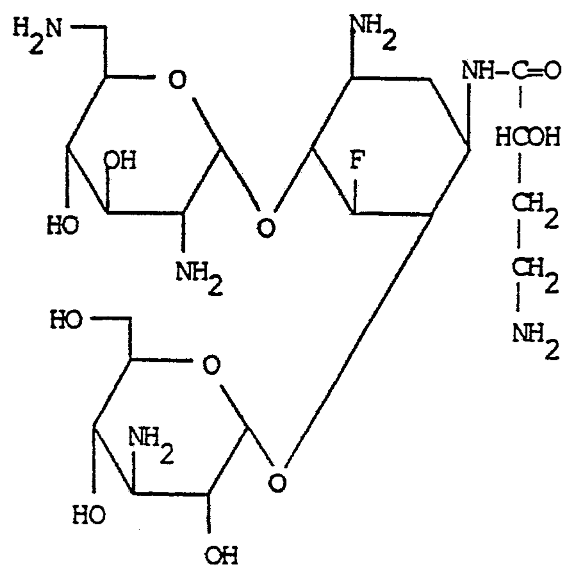

Compound (29) (59.4 mg) as obtained in the step (A) above was dissolved in a mixed solvent (4.15 ml) of 2N aqueous ammonia and tetrahydrofuran (4:3), followed by effecting the reaction overnight at 28°C under stirring to remove the 3"-N-trifluoroacetyl group from Compound (29). The reaction mixture was concentrated and the resulting solid was dissolved in a mixed solvent (3 ml) of dioxane, water and acetic acid (4:1:1). The resulting solution was subjected to catalytic reduction with hydrogen gas at room temperature for 30 minutes in the presence of palladium black as a catalyst, for the removal of the N-benzyloxycarbonyl groups. The reaction mixture was filtered and the filtrate was concentrated to a solid. The solid was dissolved in water and was chromatographed gradiently in a column of "CM-Sephadex C-25" (trade name) as developed with aqueous ammonia, while changing the concentration of ammonia from 0 N to 0.5 N. Fractions of the eluate containing the intended compound were collected and concentrated to afford the titled Compound No. 6 of this invention as a colorless solid (15.7 mg).

Specific rotation $[\alpha]_D^{23}$ : +85° (c 1.0, water).

$^1$H-NMR spectrum (in deutero-hydrochloric acid; internal standard: sodium 3-(trimethylsilyl)-propionate-$d_4$):

δ4.90 (1H, dt, H-5),
δ5.14 (1H, d, H-1" or H-1'),
δ5.76 (1H, d, H-1' or H-1").

$^{19}$F-NMR spectrum (in deutero-hydrochloric acid; external standard: trichlorofluoromethane):
-190.07 ppm (dt).

Example 7

(A) Preparation of 3,2',6'-tris(N-benzyloxy carbonyl)-5-deoxy-5-fluorotobramycin [Compound (30)]

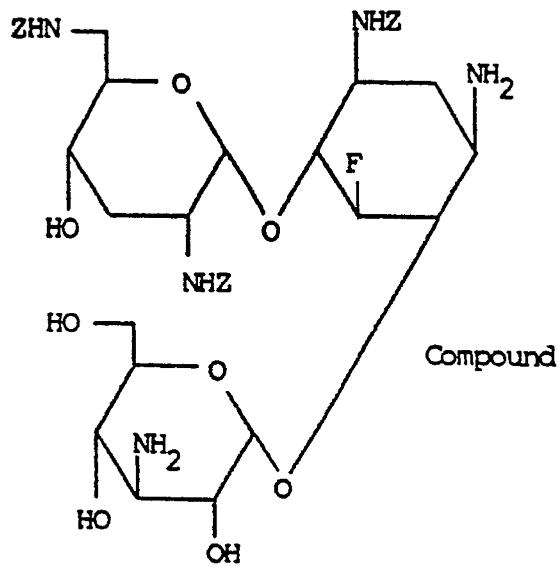

Compound (30)

In dry dimethylsulfoxide (0.31 ml) was suspended the carbonate (31 mg) of 5-deoxy-5-fluorotobramycin (Compound No. 2 of this invention), followed by addition of zinc acetate dihydrate (67 mg). The resulting mixture was stirred at 80°C for 1.5 hours and the resulting homogeneous solution was cooled down to room temperature, followed by gradual addition of N-(benzyloxycarbonyloxy)-succinimide (49 mg). The reaction was effected at room temperature for 1 hour (for the N-benzyloxycarbonylation). Ethyl ether was added to the reaction mixture. The reaction mixture was then washed repeatedly with ethyl ether and the resulting solid was repeatedly washed with 3N aqueous ammonia to remove the zinc cation. The resultant solid product was washed with water to afford the titled Compound (30) (48 mg).

(B) Preparation of 3,2',6'-tris(N-benzyloxycarbonyl)-5-deoxy-5-fluoro-3"-N-trifluoroacetyltobramycin [Compound (31)]

Compound (30) (47 mg) as obtained in the above step (A) above was dissolved in dry dimethylsulfoxide (0.24 ml), followed by addition of ethyl trifluoroacetate (0.0084 ml). The reaction was effected at room temperature for 1 hour (for the 3"-N-trifluoroacetylation). Ethyl ether was added to the reaction mixture. The solid as deposited was washed repeatedly with ethyl ether to afford the titled Compound (31) (53 mg).

(C) Preparation of 3,2',6',-tris(N-benzyloxycarbonyl)-1-N-[3-benzyloxycarbonylamino-2-(RS)-hydroxypropionyl]-3"-N-trifluoroacetyl-5-deoxy-5-fluorotobramycin [Compound (32)]

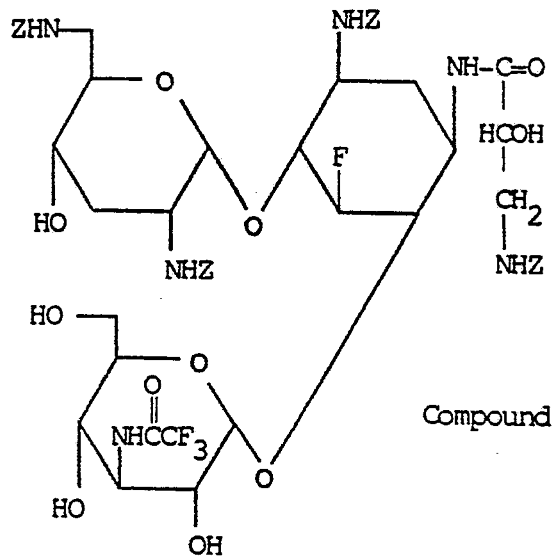

Compound (32)

In a mixed solvent (1.67 ml) of tetrahydrofuran and water (1:1) was dissolved 3,2',6'-tris(N-benzyloxycarbonyl)-3"-N-trifluoroacetyl-5-deoxy-5-fluorotobramycin (56 mg), namely Compound (31) as obtained in the step (B) above. The resulting solution was added with sodium carbonate (4.8 mg) and then with a solution of the N-hydroxysuccinimide ester (24 mg) of 3-benzyloxycarbonylamino-2-(RS)-hydroxypropionic acid in tetrahydrofuran (0.83 ml). The reaction was effected at room temperature for 1 hour (for the 1-N-acylation reaction). Thereafter, the reaction mixture was concentrated. After washing the residue with water and then drying same, the residue was washed with ethyl ether to afford the titled Compound (32) (63 mg).

(D) Preparation of 1-N-[3-amino-2-(RS)-hydroxypropionyl]-5-deoxy-5-fluorotobramycin [Compound No. 7 of this invention]

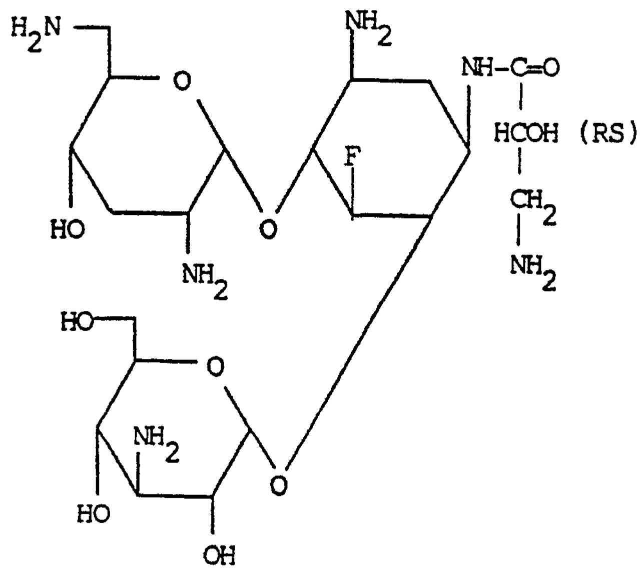

Compound (32) (62 mg) as obtained in the step (C) above was dissolved in a mixed solvent (4.4 ml) of 2N aqueous ammonia and tetrahydrofuran (4:3), followed by effecting the reaction overnight at 28°C to remove the 3″-N-trifluoroacetyl group from Compound (32). A solid reaction product, which had been obtained by concentrating the resulting reaction mixture, was dissolved in a mixed solvent (3.0 ml) of dioxane, acetic acid and water (4:1:1). In the presence of palladium black as a catalyst, the reaction product was subjected to catalytic reduction with hydrogen at room temperature for 1 hour so as to remove the N-benzyloxycarbonyl groups. After filtration of the reaction mixture, the filtrate was concentrated to a solid. The solid was dissolved in water and was chromatographed gradiently in a column of "CM-Sephadex C-25" (trade name) as developed with aqueous ammonia, while changing the concentration of ammonia from 0 N to 0.5 N. Fractions of the eluate containing the intended compound were collected and concentrated to afford the titled Compound No. 7 of this invention (22.1 mg).

Specific rotation $[\alpha]_D^{23}$ : +75° (c 1, water).

Example 8

(A) Preparation of 3,2',6'-tris(N-benzyloxycarbonyl)-1-N-[3-benzyloxycarbonylamino-2-(S)-hydroxypropionyl]-3"-N-trifluoroacetyl-5-deoxy-5-fluorotobramycin [Compound (33)]

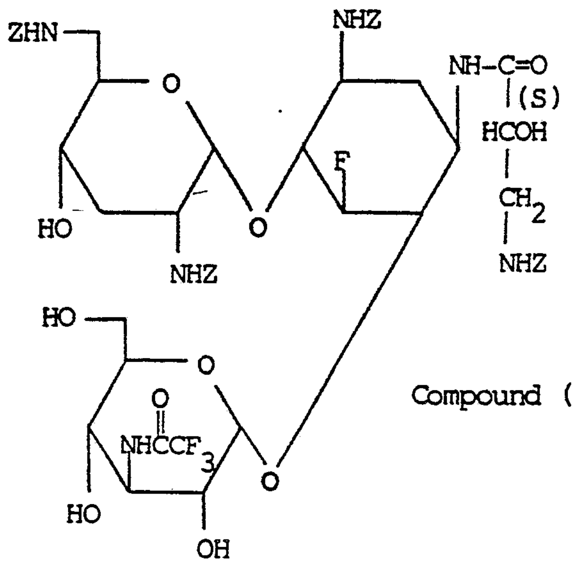

Compound (33)

Compound (31) (47 mg) as obtained in the step (B) of Example 7 was dissolved in a mixed solvent (1.4 ml) of tetrahydrofuran and water (1:1). The resulting solution was added with sodium carbonate (4.0 mg) and then with a solution of the N-hydroxysuccinimide ester (20 mg) of 3-benzyloxycarbonylamino-2-(S)-hydroxypropionic acid in tetrahydrofuran (0.7 ml). After effecting the reaction for 1 hour at room temperature, the reaction mixture was concentrated, and the residue was washed with water, dried and washed with ethyl ether to afford the titled Compound (33) (52 mg).

(B) Preparation of 1-N-[3-amino-2-(S)-hydroxypropionyl]-5-deoxy-5-fluorotobramycin [Compound No. 8 of this invention]

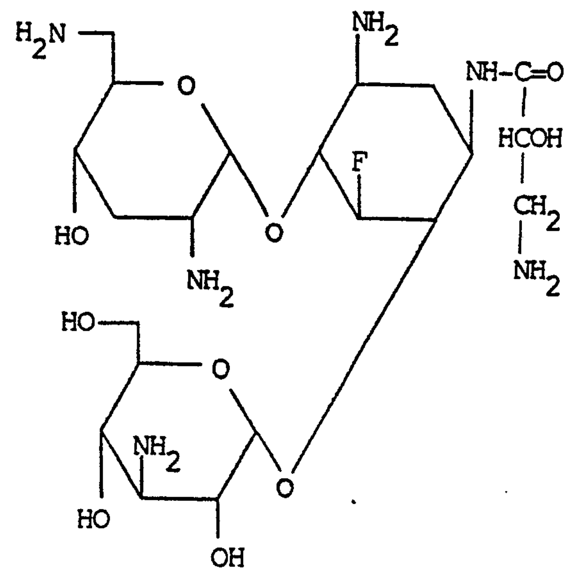

Compound (33) (52 mg) as obtained in the step (A) above was dissolved in a mixed solvent (3.7 ml) of 2N aqueous ammonia and tetrahydrofuran (4:3), followed by effecting the reaction overnight at 28°C to remove the 3″-N-trifluoroacetyl group. Thereafter, a solid which had been obtained by concentrating the resulting reaction mixture was dissolved in a mixed solvent (2.5 ml) of dioxane, acetic acid and water (4:1:1). The reaction product was subjected to catalytic reduction with hydrogen in the presence of palladium black as a catalyst at room temperature for 1 hour so as to remove the N-benzyloxycarbonyl groups. After filtration of the reaction mixture, the filtrate was concentrated to a solid. The solid was dissolved in water and the solution was developed gradiently in a column of "CM-Sephadex C-25" (trade name) with aqueous ammonia, while changing the concentration of ammonia from 0 N to 0.5 N. Fractions of the eluate containing the intended compound were collected and concentrated to afford the titled Compound No. 8 of this invention (18.2 mg).

Specific rotation $[\alpha]_D^{23}$ : +79° (c 1, water).

Example 9

(A) Preparation of 3,2′,6′(N-benzyloxycarbonyl)-1-N-[4-benzyloxycarbonylamino-2-(S)-hydroxybutyryl]-5-deoxy-5-fluoro-3″-N-trifluoroacetyltobramycin [Compound (34)]

Compound (31) (52 mg) as obtained in the step (B) of Example 7 was dissolved in a mixed solvent (1.55 ml) of tetrahydrofuran and water (1:1). The resulting solution was admixed with sodium carbonate (4.5 mg) and then with a solution of the N-hydroxysuccinimide ester (22.5 mg) of (S)-4-benzyloxycarbonylamino-2-(S)-hydroxybutyric acid in tetrahydrofuran (0.78 ml). After effecting the reaction for 2 hours at room temperature, the reaction solution obtained was added with a solution of the above ester (1.9 mg) in tetrahydrofuran (0.065 ml). The reaction was again made for 3 hours (for the 1-N-acylation reaction). Thereafter, the reaction mixture was concentrated, and the residue was washed with water, dried and washed with ethyl ether to afford the titled Compound (34) (59 mg).

(B) Preparation of 1-N-[4-amino-2-(S)-hydroxybutyryl]-5-deoxy-5-fluorotobramycin [Compound No. 9 of this invention]

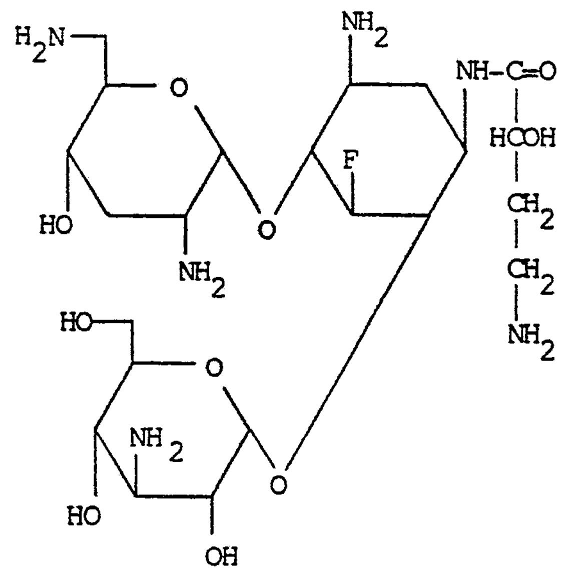

Compound (34) (59 mg) as obtained in the step (A) above was dissolved in a mixed solvent (4.3 ml) of 2N aqueous ammonia and tetrahydrofuran (4:3), followed by effecting the reaction overnight at 28°C to remove the 3"-N-trifluoroacetyl group from Compound (34). Thereafter, the reaction mixture was concentrated and the resulting solid residue was dissolved in a mixed solvent (3 ml) of dioxane, water and acetic acid (4:1:1). The solid reaction product was subjected to catalytic reduction with hydrogen in the presence of palladium black catalyst at room temperature for 1 hour so as to remove the N-benzyloxycarbonyl group. After filtration of the reaction mixture, the filtrate was concentrated to obtain a solid. The solid was dissolved in water and the solution was chromatographed gradiently in a column of "CM-Sephadex C-25" (trade name) as developed with aqueous ammonia, while changing the concentration of ammonia from 0 N to 0.5 N. Fractions of the eluate containing the intended compound were collected and concentrated to afford the above titled Compound No. 9 of this invention as a colorless solid (21.2 mg).

Specific rotation $[\alpha]_D^{23}$ : +77° (c 3.0, water).

$^1$H-NMR spectrum (in deutero-hydrochloric acid; internal standard: sodium 3-(trimethylsilyl)-propionate-$d_4$):

δ4.02 (1H, dt, H-5),
δ5.17 (1H, d, H-1" or H-1'),
δ5.64 (1H, d, H-1' or H-1").

Example 10

(A) Preparation of 3,2',6'-tris(N-benzyloxycarbonyl)-5,4'-dideoxy-5-fluorokanamycin B [Compound (35)]

In dry dimethylsulfoxide (0.47 ml) was suspended the carbonate (45.6 mg) of 5,4'-dideoxy-5-fluorokanamycin B (Compound No. 3 of this invention), followed by addition of zinc acetate dihydrate (100 mg). The mixture obtained was stirred at 80°C for 1.5 hours. The resulting homogeneous solution containing the zinc complex so produced was cooled down to room temperature, followed by gradual addition of N-(benzyloxycarbonyloxy)-succinimide (73.5 mg). The reaction was effected at room temperature for 1 hour (for the amino-protecting reaction by benzyloxycarbonyl group). Ethyl ether was added to the reaction mixture. A solid comprising the N-benzyloxycarbonylated zinc-complex was thus precipitated and this solid was washed repeatedly with ethyl ether, and the solid was again repeatedly washed with 3N aqueous ammonia to remove the zinc cation from the N-benzyloxycarbonylated zinc-complex. The resulting solid reaction product was washed with water and then dried to afford the titled Compound (35) (70 mg).

(B) Preparation of 3,2',6'-tris(N-benzyloxycarbonyl)-3"-N-trifluoroacetyl-5,4'-dideoxy-5-fluorokanamycin B [Compound (36)]

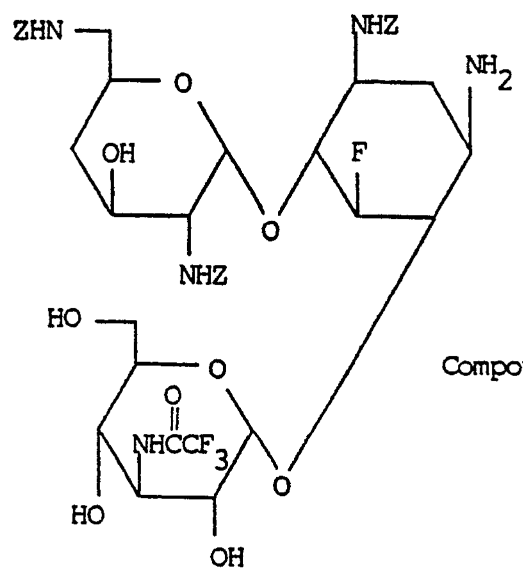

Compound (36)

Compound (35) (65 mg) as obtained in the above step (A) above was dissolved in dry dimethylsulfoxide (0.325 ml), followed by addition of ethyl trifluoroacetate (0.0116 ml). The reaction was effected at room temperature for 1 hour. Ethyl ether was added to the reaction mixture. The solid so precipitated was washed repeatedly with ethyl ether to afford the titled Compound (36) (72 mg).

(C) Preparation of 3,2'6'-tris(N-benzyloxycarbonyl)-1-N-{4-benzyloxycarbonylamino-2-(S)-hydroxybutyryl}-3"-N-trifluoroacetyl-5,4'-dideoxy-5-fluorokanamaycin {Compound (37)}

Compound (36) (70 mg) as obtained in the step (B) above was dissolved in a mixed solvent (2.09 mℓ) of tetrahydrofuran and water (1:1). After addition of sodium carbonate (2.0 mg) to the resulting solution, there was added a solution of the N-hydroxysuccinimide ester (30.3 mg) of (S)-4-benzyloxycarbonylamino-2-hydroxybutyric acid in tetrahydrofuran (1.05 mg). The reaction was effected at room temperature for 2 hours, followed by further addition of a solution of the N-hydroxysuccinimide ester (2.5 mg) of (S)-4-benzyloxycarbonylamino-2-hydroxybutyric acid in tetrahydrofuran (0.0087 mℓ). The reaction was again effected for 3 hours (for the 1-N-acylation). Thereafter, the reaction mixture was concentrated and the residue was washed with water. After drying, the residue was washed with ethyl ether to afford the titled Compound (37) (76 mg).

(D) Preparation of 1-N-{4-amino-2-(S)-hydroxybutyryl)-5,4'-dideoxy-5-fluorokanamycin B (Compound No. 10 of this invention)

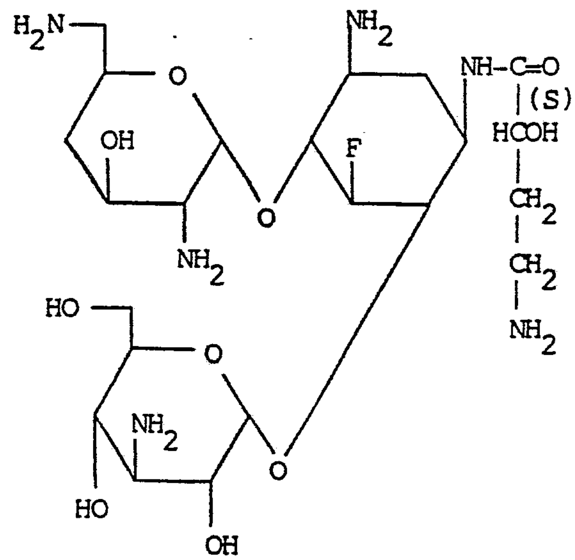

Compound (37) (63 mg) as obtained in the step (C) above was dissolved in a mixed solvent (4.6 ml) of 2N aqueous ammonia and tetrahydrofuran (4:3), followed by effecting the reaction overnight at 28°C to remove the 3"-N-trifluoroacetyl group from Compound (37). The reaction mixture was then concentrated and the resulting solid was dissolved in a mixed solvent (3.2 ml) of dioxane, water and acetic acid (4:1:1). The catalytic reduction was conducted at room temperature for 1 hour in the presence of palladium black as a catalyst, to remove the N-benzyloxycarbonyl groups. The reaction mixture was filtered and the filtrate was concentrated to give a solid. The solid was dissolved in water, and the resulting solution was charged into a column of the "CM-Sephadex C-25" (trade name), and then the column was developed and eluted gradiently with aqueous ammonia while changing the concentration of ammonia from 0 N to 0.5 N. Fractions of the eluate containing the intended compound were collected together and concentrated to afford the titled Compound No. 10 of this invention (22.9 mg).

Specific rotation $[\alpha]_D^{25}$ : +82° (c 1.0, water).

Example 11

(A) Preparation of 3,2'6'-tris(N-benzyloxycarbonyl)-5-deoxy-5-fluorodibekacin {Compound (38)}

Dry DMSO (0.5 ml) was added to 5-deoxy-5-fluorodibekacin as obtained in Example 4, followed by addition of zinc acetate dihydrate (97 mg, 4.5 molar equivalents). Under $N_2$ atmosphere, the resulting mixture was stirred overnight at room temperature. The resulting homogeneous solution was added gradually with N-benzyloxycarbonyloxy-succinimide (73.5 mg). 2.5 Hours later, N-benzyloxycarbonyloxy-succinimide (7.4 mg) was again added. After conducting the reaction for 5 hours, ethyl ether was added to the reaction mixture and the resulting precipitate was repeatedly washed with ethyl ether. The white powder obtained was washed successively with 3N aqueous ammonia and water. The powder was dried under reduced pressure, to give the titled Compound (38) as a solid (84.8 mg). The yield was stoichiometric.

(B) Preparation of 3,2',6'-tris(N-benzyloxycarbonyl)-3"-N-trifluoroacetyl-5-deoxy-5-fluorodibekacin {Compound (39)}

Compound (38) (84.8 mg) of the step (A) above was dissolved in dry DMF (1.7 ml), followed by adding ethyl trifluoroacetate (18 μl) and conducting the reaction at room temperature. 2 Hours Later, ethyl trifluoroacetate (12 μl) was again added. After continuing the reaction for 4 hours, the reaction mixture was concentrated under reduced pressure. The residue was washed with ethyl ether and dried under reduced pressure to afford the titled compound (39) as a solid (93.4 mg). Yield: 99%.

(C) Preparation of 3,2',6'-tris(N-benzyloxycarbonyl)-1-N-{3-benzyloxycarbonylamino-2-(RS)-hydroxypropionyl}-3"-N-trifluoroacetyl-5-deoxy-5-fluorodibekacin {Compound (40)}

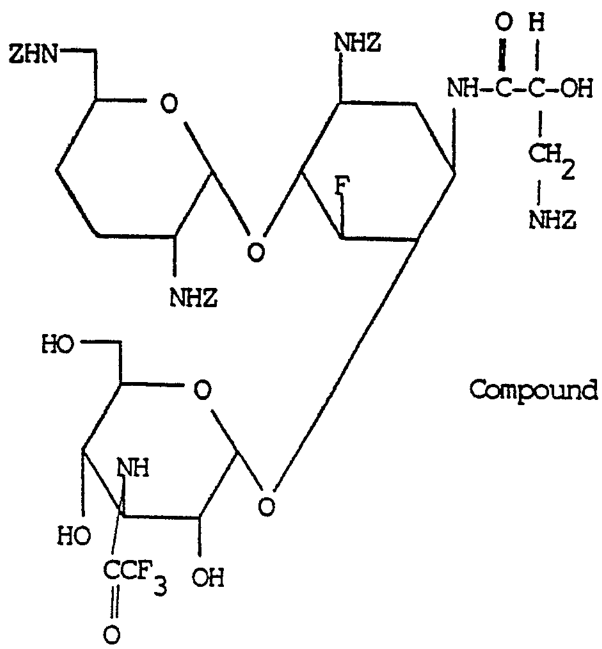

Compound (40)

Compound (39) (100 mg) of the step (B) above was dissolved in tetrahydrofuran (1.5 ml), followed by addition of an aqueous solution (1.5 ml) of sodium carbonate (8.9 mg). Then, a solution of the active N-hydroxysuccinimide ester (46 mg) of (RS)-3-benzyloxycarbonylamino-2-hydroxypropionic acid in tetrahydrofuran (1.5 ml) was added and the reaction was made at room temperature (for the 1-N-acylation). After continuing the reaction for 1 hour, the reaction mixture was concentrated under reduced pressure and the residue was washed with water and then dried under reduced pressure. Furthermore, the residue was washed with ethyl ether and dried under reduced pressure to afford 117 mg of the titled Compound (40) as a solid. Yield: 95%.

(D) Preparation of 1-N-{3-amino-2-(RS)-hydroxypropionyl}-5-deoxy-5-fluorodibekacin {Compound No. 11 of this invention}

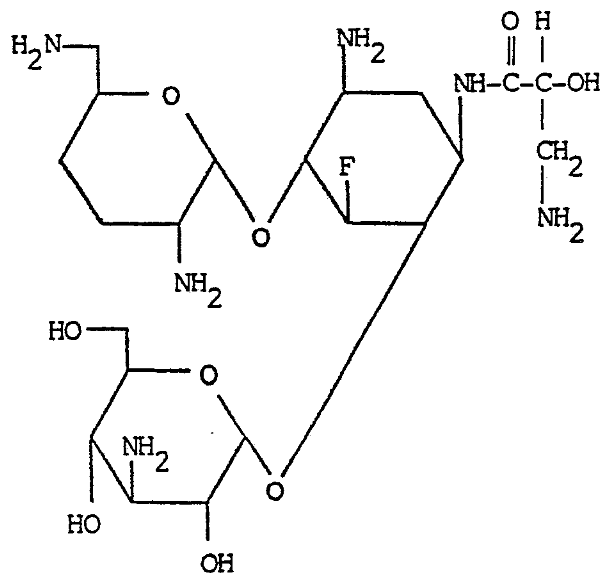

Compound (40) (117 mg) of the step (C) above was added with tetrahydrofuran (4.7 ml) and 2.5N aqueous ammonia (3.5 ml). The reaction was conducted at room temperature. After continuing the reaction for 24 hours, the reaction mixture was concentrated under reduced pressure to yield a de-trifluoroacetylated product (115 mg). A mixed solvent (8 ml) of dioxane, acetic acid and water (4:1:1) was added to the de-trifluoroacetylated product (115 mg), to which palladium black suspended in water was then added. Hydrogen gas was then blown into the resulting mixture at room temperature under atmospheric pressure. After conducting the reduction of said derivative for 2 hours, palladium black was filtered off from the reaction mixture. The catalyst was washed with water. The filtrate and the washings were combined together and then concentrated under reduced pressure. A solid (91 mg) comprising the resulting de-benzyloxycarbonylated product was dissolved in water (45.4 ml). The resultant solution was charged into a column of 20 ml of "CM-Sephadex C-25" (trade name, $NH_4^+$ -form). The column was washed with water and then eluted gradiently with aqueous ammonia, while changing the concentration of ammonia continuously from 0.05 N to 0.15 N. Fractions of the eluate containing the intended substance were combined and concentrated under reduced pressure to afford 37 mg of the titled Compound No. 10 of this invention as a solid (yield: 60%, as the monocarbonate - monohydrate).

Specific roation $[\alpha]_D^{25}$ : +82° (c 1, water).

Example 12

(A) Preparation of 3,2′,6′-tris(N-benzyloxycarbonyl)-1-N-{3-benzyloxycarbonylamino-2-(S)-hydroxypropionyl}-3″-N-trifluoroacetyl-5-deoxy-5-fluorodibekacin {Compound (41)}

Compound (39) (100 mg) as obtained in the step (B) of Example 11 above was dissolved in tetrahydrofurn (1.5 ml), followed by addition of an aqueous solution of sodium carbonate (8.9 mg) in water (1.5 ml). Further, a solution of the active N-hydroxysuccinimide ester (46 mg) of 3-benzyloxycarbonylamino-2-(S)-hydroxypropionic acid in tetrahydrofuran (1.5 ml) was added and the reaction was made at room temperature (for the 1-N-acylation). After the reaction was continued for 2 hours, the reaction mixture was concentrated under reduced pressure, and the residue was washed with water and then dried under reduced pressure. Furthermore, the residue was washed with ethyl ether and dried under reduced pressure to afford 118 mg of the titled Compound (41) as a solid. Yield: 96%.

(B) Preparation of 1-N-{3-amino-2-(S)-hydroxypropionyl}-5-deoxy-5-fluorodibekacin {Compound No. 12 of this invention}

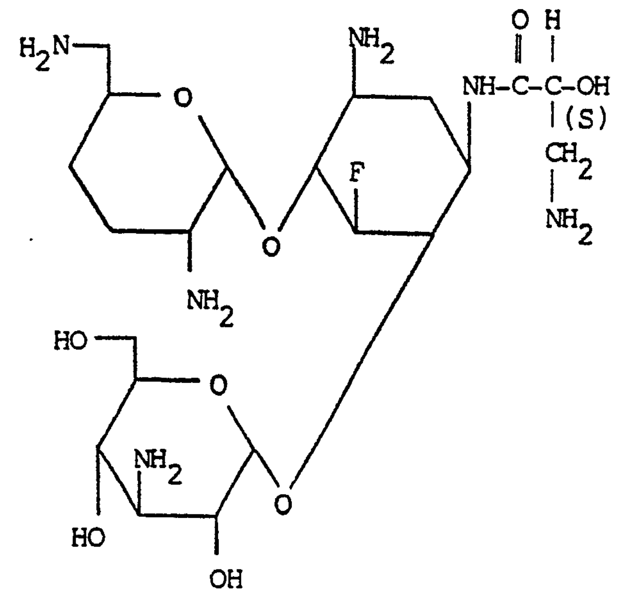

Compound (41) (118 mg) of the step (A) above was added with tetrahydrofuran (4.7 ml) and 2.5N aqueous ammonia (3.5 ml). The reaction was effected at room temperature for 22 hours. Then, the reaction mixture was concentrated under reduced pressure to give a de-trifluoroacetylated product (116 mg). A mixed solvent (8.1 ml) of dioxane, acetic acid and water (4:1:1) and a catalytic quantity of palladium black were added to the de-trifluoroacetylated product (116 mg). Hydrogen gas was then blown into the resulting mixture under normal pressure and at room temperature. After conducting the catalytic reduction for 2 hours, the catalyst was filtered off from the reaction mixture. The filtrate and the washing were combined together and then concentrated to dryness to afford 92 mg of the de-benzyloxycarbonylated product as a solid. This solid was dissolved in water (46 ml) and the resultant solution was charged into a column of 20 ml of "CM-Sephadex C-25" (trade name, $NH_4^+$ -form). The column was eluted gradiently with aqueous ammonia while changing the concentration of ammonia continuously from 0.05 N to 0.15 N. Fractions of the eluate containing the intended substance were combined and concentrated to dryness to afford 43.7 mg of the titled Compound No. 12 of this invention as a solid (yield: 70% as the monocarbonate - monohydrate).

Specific rotation $[\alpha]_D^{25}$ : +82° (c 1, water).

Example 13

(A) Preparation of 3,2',6'-tris(N-benzyloxycarbonyl)-1-N-{4-benzyloxycarbonylamino-(S)-hydroxybutyryl}-3"-N-trifluoroacetyl-5-deoxy-5-fluorodibekacin {Compound (42)}

Compound (39) (93.4 mg) of the step (B) of Example 11 was dissolved in tetrahydrofuran (1.4 ml), followed by addition of an aqueous solution of sodium carbonate (8.3 mg) in water (1.4 ml). Then, a solution of the active N-hydroxysuccinimide ester (44.7 mg) of 4-benzyloxycarbonylamino-2-(S)-hydroxybutyric acid in tetrahydrofuran (1.4 ml) was added, and the reaction was effected at room temperature (for the 1-N-acylation). 1 Hour later, the active ester (6.9 mg) was added further. After the reaction was continued for 1.5 hours, the reaction mixture was concentrated under reduced pressure, and the residue was washed with water and dried under reduced pressure. Furthermore, the residue was washed with ethyl ether and dried under reduced pressure to afford 111.1 mg of the titled Compound (42). Yield: 95%.

(B) Preparation of 1-N-{4-amino-2-(S)-hydroxybutyryl}-5-deoxy-5-fluorodibekacin {Compound No. 13 of this invention}

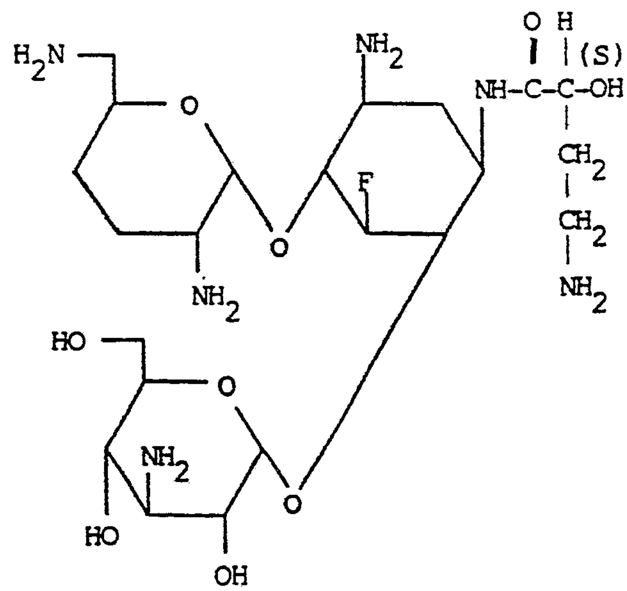

Compound (42) (111.1 mg) of the step (A) above was added with tetrahydrofuran (4.4 ml) and 2.5N aqueous ammonia (3.3 ml). The reaction was conducted at temperature for 22 hours, and then 15N aqueous ammonia (0.6 ml) was added further. After 43 hours reaction, the reaction mixture was concentrated under reduced pressure to give a de-trifluoroacetylated product (110 mg). A mixed solvent (7.7 ml) of dioxane, acetic acid and water (4:1:1) was added to the de-trifluoroacetylated product (110 mg), followed by addition of an aqueous suspension of palladium black (10 drops). Hydrogen gas was then blown into the resulting mixture under atmoshperic pressure. After continuing the catalytic reduction for 2 hours, the catalyst was replaced by a fresh supply of the same catalyst and hydrogen gas was further passed into the reaction mixture to conduct the reduction. 4 Hours later, the catalyst was filtered off from the reaction mixture and the filtrate was concentrated under reduced pressure. The de-benzyloxycarbonylated product (86.9 mg) was obtained as a solid, which was dissolved in water (43.5 ml). The resultant solution was charged into a column of 20 ml of "CM-Sephadex C-25" (trade name, $NH_4^+$ -form). The column was washed with water and then eluted gradiently with aqueous ammonia, while changing the concentration of ammonia continuously from 0.05 N to 0.5 N. The relevant fractions of the eluate were combined together and concentrated to dryness to afford 38 mg of the titled Compound No. 13 of this invention as a solid (yield: 64%, as the monocarbonate - monohydrate).

Specific rotation $[\alpha]_D^{25}$ : +83° (c 1, water).

$^1$H-NMR spectrum (250 MHz; in $D_2O$ containing 20% $ND_3$; standard: TMS):

δ4.71 (H-5, dt, $J_{5,F}$=51 Hz),

δ5.08 (H-1″, d, $J_{1'',2''}$=3.5 Hz)

δ5.17 (H-1′, d, $J_{1',2'}$=3.5 Hz).

Referential Example 5

(A) Preparation of 4",6"-O-cyclohexylidene-5-epidibekacin {Compound (43)}

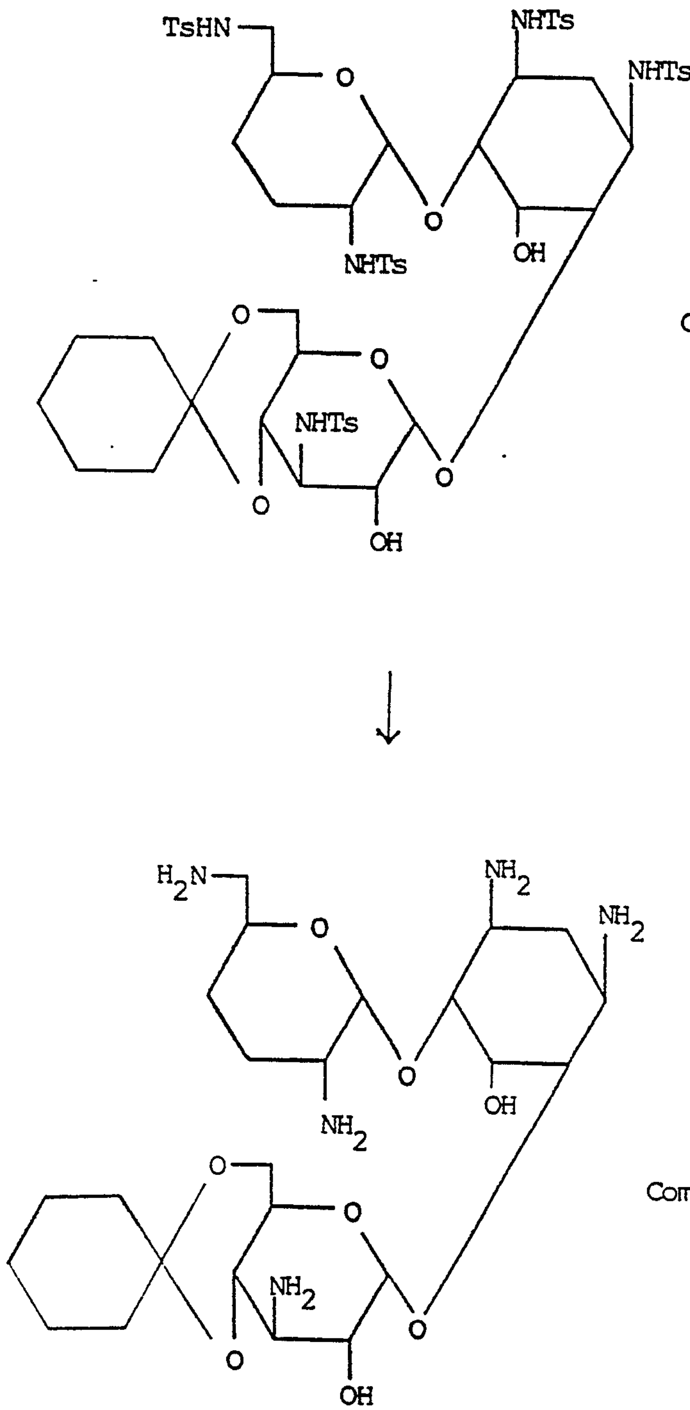

Compound (23)

Compound (43)

Metal sodium (about 50 mg) was added to liquefied ammonia (about 15 ml) at -50°C, to which was then added at once, under vigorous stirring, a solution in tetrahydrofuran (2 ml) of 4",6"-O-cyclohexylidene-1,3,2',6'3"-penta-N-tosyl-5-epidibekacin, namely, Compound (23) (50 mg), which had been obtained in Step (E) of the preceding Example 4. The reaction was conducted for 5 minutes for the de-tosylation of Compound (23). After addition of methanol (0.07 ml), the temperature of the reaction mixture was raised slowly to room temperature to evaporate the ammonia therefrom, and the remaining reaction mixture was concentrated to dryness. Water (2.2 ml) was added to the residue obtained, and an ion-exchange resin,

"Dowex 50w × 2" (trade name, $NH_4^+$ -form, 2 ml) was then added to the resultant mixture to remove the salts from the mixture. The mixture so treated was charged together with the resin into a column of 2 ml of the same resin, and the column was washed with water (12 ml). The column was eluted with 1N aqueous ammonia. The ninhydrin-positive fractions of the eluate were combined and then concentrated to dryness, affording 15.3 mg of the titled Compound (43) as a solid. Yield; 75%.

(B) Preparation of 1,3,2',6',3"-pentakis(N-benzyloxycarbonyl)-4",6"-O-cyclohexylidene-5-epi-dibekacin {Compound (44)}

Compound (43) (20 mg) of the step (A) above was dissolved in water (0.4 ml), followed by addition of sodium carbonate (24 mg) and acetone (0.4 ml). The resulting mixture was added with benzyloxycarbonyl chloride (0.04 ml), followed by conducting the reaction under ice-cooling (for the N-benzyloxycarbonylation). After continuing the reaction for 6 hours, the temperature of the reaction mixture was raised to room temperature, at which the reaction was allowed to proceed further. After 12 hours, the reaction mixture was concentrated under reduced pressure. The residue was washed successively with water and ethyl ether to afford 45 mg of the titled Compound (44) as a white solid. Yield: stoichiometric.

(C) Preparation of 2"-O-benzoyl-1,3,2',6',3"-penta-kis(N-benzyloxycarbonyl)-4",6"-O-cyclohexylidene-5-epi-dibekacin {Compound (45)}

Compound (44) (40 mg) of the preceding step (B) was dissolved in dry pyridine (0.8 ml). Under ice-cooling, the resulting solution was added with benzoyl chloride (0.019 ml), followed by conducting the reaction at 0°C (for the 2"-O-benzoylation). 45 Minutes later, water (0.015 ml) was added and the resultant mixture was left to stand at room temperature for 1 hour. The reaction mixture was then concentrated under reduced pressure. Chloroform (10 ml) was added, and the resultant mixture was washed successively with a saturated aqueous solution of sodium bicarbonate (2 ml × 2), a 5% aqueous solution of potassium hydrogen sulfate (2 ml × 2) and water (2 ml × 3). After drying the mixture over anhydrous sodium sulfate, the mixture was concentrated under reduced pressure to afford 44 mg of the titled Compound (45) as a pale yellow solid. Yield: stoichiometric.

Example 14

Preparation of 5-deoxy-5-fluorodibekacin {Compound No. 4 of this Invention}

(A) Dry pyridine (0.037 ml) was added to dry dichoromethane (0.76 ml), followed by addition of diethylaminosulfur trifluoride (DAST) (0.019 ml) under ice-cooling. To the resultant solution was added 38 mg of Compound (45) as obtained in the step (C) of Referential Example 5. The temperature of the resultant mixture was raised to room temperature, at which the reaction was conducted for 6 hours (for the fluorination and inversion of the epi-5-hydroxyl group). The reaction mixture obtained was then poured into an ice-cooled saturated aqueous solution of sodium bicarbonate (8 ml), followed by addition of chloroform (5 ml) for extraction. The extract in chloroform was washed successively with a saturated aqueous solution of sodium bicarbonate (2 ml × 2), a 5% aqueous solution of potassium hydrogen sulfate (2 ml × 2) and water (2 ml × 3). After drying the extract over anhydrous sodium sulfate, it was concentrated to dryness to give a yellow solid (36.3 mg). The solid was purified by chromatography on silica gel ("Wako-gel C-200", 4g; developed with a mixed solvent of chloroform and ethyl acetate (3:1)), thereby affording a solid (11.5 mg). An NMR analysis of this solid in deutero-pyridine revealed that a double triplet peak (J = 12, 12, 52 Hz) attributable to $19_F$ was occurring at $\delta$ = -189.44 ppm (when measured assuming that the reference peak of $CFCl_3$ was at $\delta$ = 0). The above solid was thus found to be mainly comprised of 2"-O-benzoyl-1,3,2',6',3"-pentakis(N-benzyloxycarbonyl)-4",6"-O-cyclohexylidene-5-deoxy-5-fluorodibekacin {Compound (46)}.

(B) The solid (11.5 mg) obtained as above was added with 80% acetic acid (0.23 ml), followed by effecting the reaction at 60°C (for the de-cyclohexylidenization). 1.5 Hour later, the reaction mixture was concentrated to dryness. The residue was added with toluene and subjected to azeotropic distillation to remove the acetic acid, and to afford a pale yellow solid (10.9 mg).

To the solid were added methanol (0.23 ml) and 15N aqueous ammonia (0.016 ml), followed by conducting the reaction at room temperature for 3.5 hours (for the de-benzoylation). The reaction mixture was concentrated to dryness to yield a pale yellow solid (10.5 mg). A mixed solvent (0.2 ml) of tetrahydrofuran and water (4:1) was added to the solid, followed by addition of Raney nickel. After shaking the resultant mixture thoroughly, the nickel was filtered off. The nickel was washed with a mixed solvent (0.8 ml) of tetrahydrofuran and water. The filtrate and the washing were combined together, and to the resulting solution were added acetic acid (0.2 ml) and palladium black, followed by blowing hydrogen gas into said solution at room temperature and under atmospheric pressure (for the de-benzyloxycarbonylation). 6 Hours later, palladium black was filtered off from the reaction mixture and the filtrate was concentrated to dryness to give a solid (7 mg). This solid was dissolved in water (3.5 ml). The resultant solution was charged into a column of 2 ml of "CM-Sephadex C-25" (trade name). After washing the column with water (10 ml), the column was eluted gradiently with aqueous ammonia, while changing the concentration of ammonia continuously from 0.05 N to 0.15 N. Fractions of the eluate containing the intended substance were combined and then concentrated under reduced pressure, to afford the titled Compound No. 4 of this invention (1.0 mg). The yield (as a monocarbonate) was 6.4%, based on Compound (45).

Referential Example 6

(A) Preparation of 4",6"-O-cyclohexylidene-1,3,2',6',3"-pentakis(N-methoxycarbonyl)-5-epi-dibekacin {Compound (47)}

Water (1 ml) and sodium carbonate (60 mg) were added to 50 mg of Compound (43) as obtained in the step (A) of Referential Example 5. After adding acetone (1 ml) to the resulting aqueous solution, methoxycarbonyl chloride (0.044 ml) was added dropwise to the solution under ice-cooling, and the reaction was made (for the protection by N-methoxycarbonylation). 6 Hours later, the temperature of the reaction mixture was raised to room temperature, at which the reaction was allowed to proceed further. 15 Hours later, the reaction mixture was concentrated to dryness and then washed successivly with water and ethyl ether, to obtain 73 mg of the titled Compound (47) as a white solid. Yield: 95%.

(B) Preparation of 2"-O-acetyl-4",6"-O-cyclohexylidene-1,3,2',6',3"-pentakis(N-methoxycarbonyl)-5-epi-dibekacin {Compound (48)}

In dry pyridine (1.3 ml) was dissolved 65 mg of Compound (47) as obtained in the preceding step (A), followed by addition of acetic anhydride (0.04 ml) under ice-cooling. The reaction was effected at room temperature for 15 hours (for the 2"-O-acetylation). Water (0.035 ml) was then added and the resultant mixture was left to stand at room temperature for 1 hour. The resulting reaction mixture was poured into a saturated aqueous solution of sodium hydrogen carbonate (50 ml). The resulting white precipitate was collected by filtration, washed with water and dried, to afford 66 mg of the titled Compound (48) as a white solid. Yield: 97%.

Example 15

Preparation of 5-deoxy-5-fluorodibekacin {Compound No. 4 of this invention}

(A) Dry pyridine (0.083 ml) was mixed with dry dichloromethane (1.2 ml) and then with DAST (0.043 ml) under ice-cooling, followed by further addition of 10 mg of Compound (48) as obtained in the step (B) of Referential Example 6. The reaction was effected for 6 hours at room temperature (for the fluorination and inversion of the epi-5-hydroxyl group). The reaction mixture was poured into an ice-cooled saturated aqueous solution of sodium hydrogen carbonate (18 ml), to which chloroform (12 ml) was then added. The resultant mixture was allowed to separate into the aqueous layer and the organic layer.

The organic layer was washed successively with a saturated aqueous solution of sodium hydrogen carbonate (5 ml × 2), a 5% aqueous solution of potassium hydrogen sulfate (5 ml × 2) and water (5 ml × 2), dried over anhydrous sodium sulfate, and concentrated to dryness, to afford a crude product of the 5-fluoroderivative (61 mg).

(B) The crude 5-fluoro derivative obtained as above was treated with 80% acetic acid (1.2 ml) at 60°C for 2 hours to perform the de-cyclohexylidenization, yielding the partially deprotected reaction product (55 mg). This reaction product was dissolved in tetrahydrofuran (1.1 ml), to which 6N NaOH (0.55 ml) was added. The resultant mixture was stirred at 80°C for 12 hours (for the de-acetylation and de-methoxycarbonylation). The reaction mixture obtained was concentrated under reduced pressure, and the residue was dissolved in water (3.3 ml) added. An ion-exchange resin, "Dowex 50w × 2" (trade name, $H^+$-form, 4 ml) was added to the resultant solution to de-salt the latter. The de-salted solution together with said resin was charged into a column of 4 ml of the same resin. The column was washed with water and eluted with 1N aqueous ammonia (80 ml). The ninhyrin-positive fractions of the eluate were combined and concentrated to dryness to afford a crude product of 5-deoxy-5-fluorodibekacin (36 mg).

This crude product was dissolved in water (18 ml) and the resultant solution was charged into a column of 10 ml of "CM-Sephadex C-25" (trade name, $NH_4^+$ -form). After washing the column with water (50 ml), the column was eluted gradiently with aqueous ammonia while changing the concentration of ammonia continuously from 0.05 N to 0.15 N. The fractions of the eluate containing the desired compound were combined together and concentrated to dryness. The titled Compound No. 4 of this invention was obtained (10.7 mg). The yield (as a monocarbonate) was 30%, based on Compound (48).

Referential Example 7

Preparation of 2"-O-acetyl-1,3,2',6',3"-penta-N-acetyl-4",6"-O-cyclohexylidene-5-epi-dibekacin {Compound (49)}

In a mixture of dry pyridine (0.5 ml) and dry DMF (0.25 ml) was suspended 25 mg of 4",6"-O-cyclohexylidene-5-epi-dibekacin, namely, Compound (43) as obtained in the step (A) of Referential Example 5, followed by addition of acetic anhydride (0.14 ml). The reaction was effected at room temperature for 18 hours under stirring (for the N,O-acetylation). Water (0.13 ml) was added to the reaction mixture, which was subsequently left over for 1 hour at room temperature. The reaction mixture was then concentrated under reduced pressure and the concentrate was mixed with water, followed by azeotropic distillation (5 times). The mixture thus processed was passed through a column of 3 ml of "Sephadex LH-20" (trade name) and developed with a mixed solvent of tetrahydrofuran and water (1:1). The active fractions of the eluate were combined and then concentrated to dryness. The titled Compound (49) was obtained (31 mg). Yield: 84%.

Example 16

Preparation of 5-deoxy-5-fluorodibekacin {Compound No. 4 of this invention}

To 12.1 mg of Compound (49) as obtained in Referential Example 7 were added dry dichloromethane (0.24 ml), dry pyridine (0.019 ml) and dry acetonitrile (0.24 ml). DAST (0.01 ml) was added to the resulting mixture under ice-cooling and stirring, followed by effecting the reaction at room temperature (for the fluorination and inversion of the epi-5-hydroxy group). 6 Hours later, the reaction mixture was poured into an ice-cooled saturated aqueous solution of sodium hydrogen carbonate (1.4 ml). The resulting whole mixture was concentrated to dryness at room temperature. The solid residue obtained was extracted with DMF (2 ml × 3), and the extracts in DMF were concentrated to dryness to give a crude product (12.1 mg). An NMR analysis of this crude solid product in deutero-water showed that a double triplet peak (J = 12, 12, 49 Hz) attributable to $19_F$ was appearing at $\delta$ = -191.6 ppm (when measured assuming that the reference peak of $CFCl_3$ was at $\delta$ = 0). This crude product mainly comprised 2"-O-acetyl-1,3,2',6',3"-penta-N-acetyl-4",6"-O-cyclohexylidene-5-deoxy-5-fluorodibekacin {Compound (50)}. Subsequently, the cyclohexylidene group and acetyl groups were removed from Compound (50), respectively, by hydrolysis with acetic acid and by hydrolysis with an aqueous solution of sodium hydroxide in the same manner as in the step (B) of Example 15. The resulting crude product of 5-deoxy-5-fluorodibekacin was purified by chromatography. The titled Compound No. 4 of this invention was afforded (2.0 mg). The yield was 25%, based on Compound (49).

## Claims

1. 5-Deoxy-5-fluorokanamycin B or a derivative thereof represented by the general formula

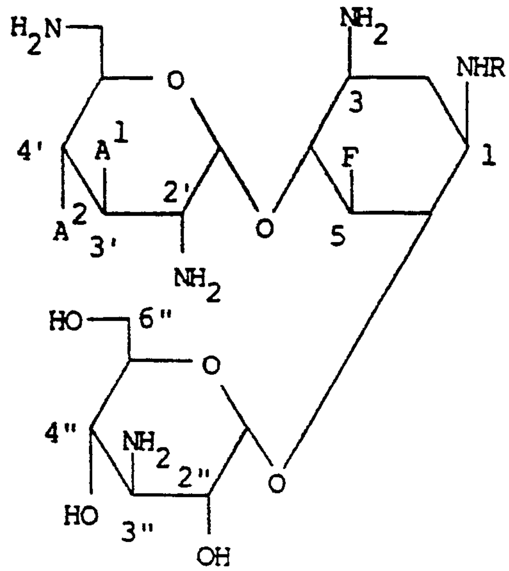

(I)

wherein R is a hydrogen atom or an α-hydroxy-ω-aminoalkanoyl group of the formula

$$-CO-\underset{\displaystyle OH}{\underset{|}{CH}}-(CH_2)_n-NH_2$$

where n is an integer of 1 or 2, and $A^1$ and $A^2$ are independently a hydroxyl group or a hydrogen atom, or a pharmaceutically acceptable acid addition salt thereof.

2. A compound as claimed in Claim 1, which is 5-deoxy-5-fluorokanamycin B, namely the compound of the general formula (I) where R is a hydrogen atom and $A^1$ and $A^2$ are each a hydroxyl group.

3. A compound as claimed in Claim 1, which is 5,3'-dideoxy-5-fluorokanamycin B, namely the compound of the general formula (I) where R and $A^1$ are each a hydrogen atom and $A^2$ is a hydroxyl group.

4. A compound as claimed in Claim 1, which is 5,4'-dideoxy-5-fluorokanamycin B, namely the compound of the general formula (I) where R and $A^2$ are each a hydrogen atom and $A^1$ is a hydroxyl group.

5. A compound as claimed in Claim 1, which 5,3',4'-trideoxy-5-fluorokanamycin B, namely the compound of the geneal formula (I) where R, $A^1$ and $A^2$ are each a hydrogen atom.

6. A compound as claimed in Claim 1, which is 1-N-(3-amino-2-(S)-hydroxypropionyl)-5-deoxy-5-fluorokanamycin B, namely the compound of the general formula (I) where R is a 3-amino-2-(S)-hydroxypropionyl group, and $A^1$ and $A^2$ are each a hydroxyl group.

7. A compound as claimed in Claim 1, which is 1-N-(4-amino-2-(S)-hydroxybutyryl)-5-deoxy-5-fluorokanamycin B, namely the compound of the general formula (I) where R is a 4-amino-2-(S)-hydroxybutyryl group, and $A^1$ and $A^2$ are each a hydroxyl group.

8. A compound as claimed in Claim 1, which is 1-N-(3-amino-2-(RS)-hydroxypropionyl)-5,3'-dideoxy-5-fluorokanamycin B, namely the compound of the general formula (I) where R is a 3-amino-2-(RS)-hydroxypropionyl group, $A^1$ is a hydrogen atom and $A^2$ is a hydroxyl group.

9. A compound as claimed in Claim 1, which is 1-N-(3-amino-2-(S)-hydroxypropionyl)-5,3'-dideoxy-5-fluorokanamycin B, namely the compound of the general formula (I) where R is a 3-amino-2-(S)-hydroxypropionyl group, $A^1$ is a hydrogen atom and $A^2$ is a hydroxyl group.

10. A compound as claimed in Claim 1, which is 1-N-(4-amino-2-(S)-hydroxybutyryl)-5,3'-dideoxy-5-fluorokanamycin B, namely the compound of the general formula (I) where R is a 4-amino-2-(S)-hydroxybutyryl group, $A^1$ is a hydrogen atom and $A^2$ is a hydroxyl group.

11. A compound as claimed in Claim 1, which is 1-N-(4-amino-2-(S)-hydroxybutyryl)-5,4'-dideoxy-5-fluorokanamycin B, namely the compound of the general formula (I) where R is a 4-amino-2-(S)-hydroxybutyryl group, $A^1$ is a hydroxyl group and $A^2$ is a hydrogen atom.

12. A compound as claimed in Claim 1, which is 1-N-(3-amino-2-(S)-hydroxypropionyl)-5,3',4'-trideoxy-5-fluorokanamycin B, namely the compound of the general formula (I) where R is a 3-amino-2-(S)-hydroxypropionyl group, and $A^1$ and $A^2$ are each a hydrogen atom.

13. A compound as claimed in Claim 1, which is 1-N-(4-amino-2-(S)-hydroxybutyryl)-5,3',4'-trideoxy-5-fluorokanamycin B, namely the compound of the general formula (I) where R is a 4-amino-2-(S)-hydroxybutyryl group, and $A^1$ and $A^2$ are each a hydrogen atom.

14. A pharmaceutical composition comprising a compound as claimed in any one of the preceding claims or a pharmaceutically acceptable acid addition salt thereof, as the active ingredient, in association with a pharmaceutically acceptable solid or liquid carrier for the active ingredient.

15. A process for the production of 5-deoxy-5-fluorokanamycin B or a derivative thereof represented by the general formula

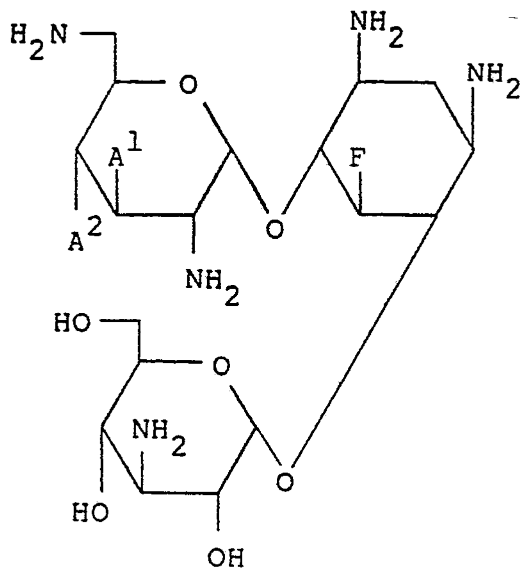

(Ia)

wherein $A^1$ and $A^2$ are independently a hydroxyl group or a hydrogen atom, which comprises (i) reacting an N,O-protected 5-epi-kanamycin B derivative represented by the general formula

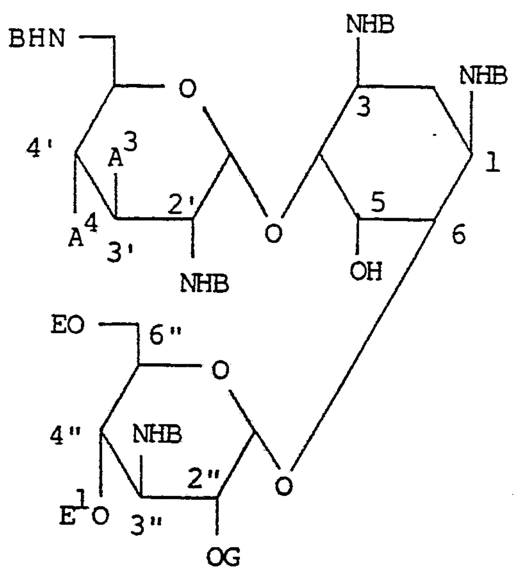

(II)

wherein $A^3$ and $A^4$ are independently a protected hydroxyl group -OG or a hydrogen atom, where G denotes an acyl group as the hydroxyl-protecting group, B is an amino-protecting group, and E and $E^1$ are each an acyl group of the same nature as the aforesaid group G and are the hydroxyl-protecting group, or E and $E^1$ as taken together form a di-valent hydroxyl-protecting group; and the group G present at the 2''-hydroxyl

group is an acyl group of the same nature as the aforesaid group G and is the hydroxyl-protecting group, with a dialkylaminosulfur trifluoride of the formula

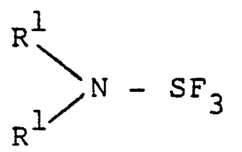

(III)

wherein $R^1$ is an alkyl group of 1 to 4 carbon atoms, or with a bis(dialkylamino)-sulfur difluoride of the formula

$R^1N-SF_2-NR^1$ (III')

wherein $R^1$ is as defined above, or with a fluorination agent equivalent to the compound of formula (III) or (III') in a non-polar organic solvent, to effect the steric inversion of the epi-hydroxyl group at the 5-position of the compound of formula (II) and also the replacement of the 5-hydroxyl group by a fluoro substitutent, thereby producing an N,O-protected 5-deoxy-5-fluorokanamycin B derivative represented by the formula

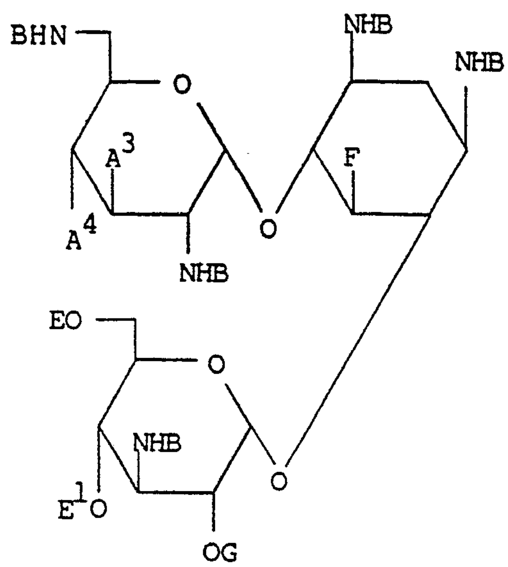

(IV)

wherein $A^3$, $A^4$, B, E, $E^1$ and G are as defined above, and then (ii) removing the remaining amino-protecting group (B) and the remaining hydroxyl-protecting groups (E, $E^1$ and G) from the compound of formula (IV) in a known manner.

16. A process for the production of a 1-N-(α-hydroxy-ω-aminoalkanoyl)-5-deoxy-5-fluorokanamycin B derivative represented by the general formula

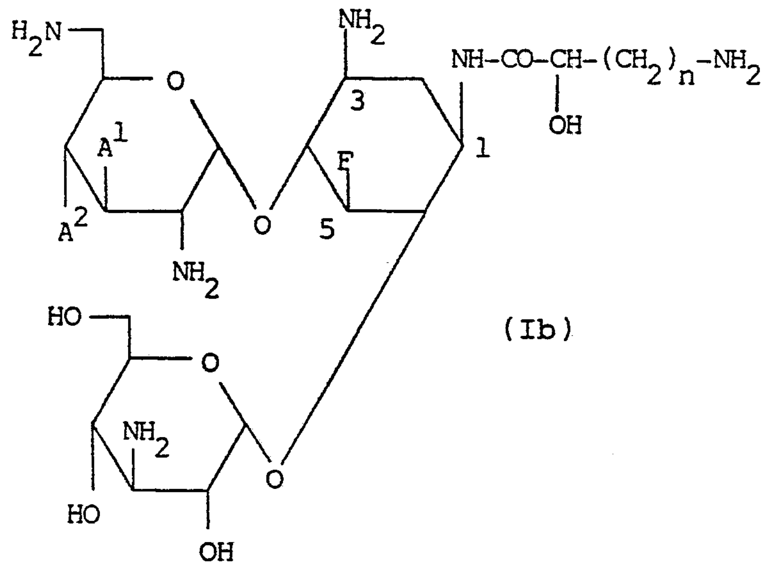

(Ib)

wherein $A^1$ and $A^2$ are independently a hydroxyl group or a hydrogen atom and n is an integer of 1 or 2, which comprises (i) reacting the 1-amino group of 5-deoxy-5-fluorokanamycin B or a derivative thereof represented by the general formula

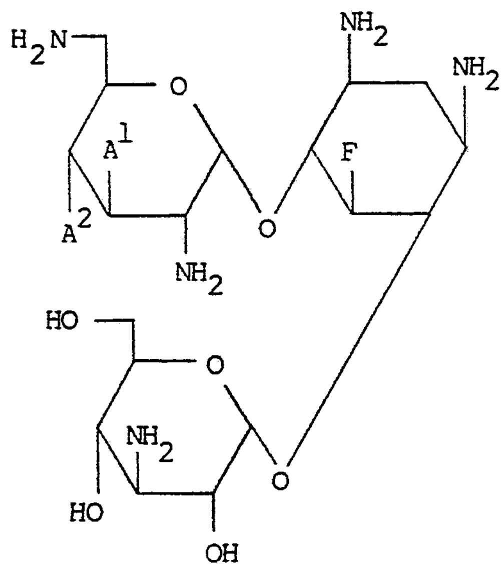

(Ia)

wherein $A^1$ and $A^2$ are each as defined above, or the 1-amino group of such protected derivative of the compound of formula (Ia) whose some or all of the amino groups other than the 1-amino group has or have been protected by amino-protecting groups, with an α-hydroxy-ω-aminoalkanoic acid represented by the general formula

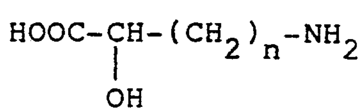

(V)

wherein n is an integer of 1 or 2, or such protected derivative of said aminoalkanoic acid of formula (V) whose the amino group has been protected by an amino-protecting group, or an equivalent reactive derivative thereof, and then (ii) removing from the resulting 1-N-acylated product compound the remaining amino-protecting groups when the latter groups exist, to produce the compound of formula (Ib).